# EUROPEAN PATENT APPLICATION

(11) **EP 4 279 177 A1**
(43) Date of publication of application: **22.11.2023**
(21) Application number: 21919495.8
(22) Date of filing: 22.09.2021
(51) Int. Cl.: B01J 31/28, B01J 31/08, C07B 61/00, C07C 45/65, C07C 49/782, C07C 253/30, C07C 255/50

(54) **CATALYST HAVING PLATINUM-GROUP METAL ION SUPPORTED THEREON, AND CARBON-CARBON BOND FORMATION METHOD**

(30) Priority: 12.01.2021 JP 2021002749
(71) Applicant: Organo Corporation, Tokyo 136-8631 (JP)
(72) Inventor: NAKAMURA, Shinji, Tokyo 136-8631 (JP); TAKADA, Hitoshi, Tokyo 136-8631 (JP); SAJIKI, Hironao, Gifu-city Gifu 501-1196 (JP); YAMADA, Tsuyoshi, Gifu-city Gifu 501-1196 (JP); Park, Kwihwan, Gifu-city Gifu 501-1196 (JP)
(74) Representative: Müller-Boré & Partner Patentanwälte PartG mbB
(86) International application number: PCT/JP2021/034818
(87) International publication number: WO 2022/153604

(57) **Abstract**

A platinum group metal ion-supported catalyst in which platinum group metal ions or platinum group metal complex ions are supported on a non-particulate organic porous ion exchanger, wherein the non-particulate organic porous ion exchanger is formed of a continuous framework phase and a continuous pore phase; has a thickness of a continuous framework of 1 to 100 µm, an average diameter of continuous pores of 1 to 1000 µm, and a total pore volume of 0.5 to 50 ml/g; has an ion exchange capacity per weight in a dry state of 1 to 9 mg equivalent/g; and has ion exchange groups wherein the ion exchange groups are uniformly distributed in the organic porous ion exchanger. According to the present invention, there can be provided a catalyst that enables a method for forming a carbon-carbon bond to obtain the desired compound by forming a carbon-carbon bond to be performed in a fixed-bed continuous circulation manner, the catalyst enabling the reaction to be performed with a high yield in various raw materials.

## Description

### [Technical Field]

The present invention relates to a method for forming a carbon-carbon bond, wherein carbon-carbon bond-forming reaction is performed by passing a raw material liquid of the reaction through a platinum group metal ion-supported catalyst, and a platinum group metal ion-supported catalyst for carrying out the method.

### [Background Art]

Carbon-carbon-forming reaction (coupling reaction) using a platinum group metal such as palladium as a catalyst, which is typified by Suzuki-Miyaura coupling, Sonogashira coupling, and Mizorogi-Heck coupling has become increasingly important in recent years for the synthesis processes of pharmaceutical intermediates or highly functional materials such as liquid crystals.

Heretofore, the platinum group metal catalyst has often been used in a homogeneous system and has exhibited high catalytic activity, but has disadvantages such as difficult recovery of the catalyst and contamination of manufactured products with the catalyst metal. Accordingly, heterogenous catalysts in which the catalyst is supported on a support have been developed so as to facilitate the recovery of the catalyst while reducing the metallic contamination of manufactured products. Methods for continuously forming a carbon-carbon bond by passing a reaction substrate solution through such a heterogenous catalyst have also been developed in recent years. For example, Japanese Patent No. 5638862 reports a method for continuously forming a carbon-carbon bond by using a catalyst in which palladium is supported on a porous silica support, but discloses no actual reaction example. Japanese Patent No. 5255215 and Japanese Patent Laid-Open No. 2008-212765 disclose a method for forming a carbon-carbon bond by using a catalyst in which a platinum group metal is supported on the wall surface of a fine flow channel with a width of 1 mm and a depth of 20 µm. Nonetheless, for unknown reasons, the disclosed reaction examples do not employ water as a solvent and are carried out at a liquid passing speed of a reaction substrate solution of only 1 µm/min, leading to problems with environmental load and production efficiency. A method for continuously forming a carbon-carbon bond by using a column filled with a catalyst in which palladium is supported on an organic support is reported in ChemCatChem, 2019, No. 11, p. 2427, but there exists a complex chemical conversion for obtaining the organic support. Additionally, for unknown reasons, it is necessary to mix a large amount of diatomaceous earth when the palladium-supported catalyst is used.

Meanwhile, we have developed a catalyst in which a platinum group metal is supported on a non-particulate organic ion exchanger having three dimensionally continuous pores, and have reported, in Japanese Patent Laid-Open No. 2014-15420 (Patent Literature 4) or 2016-190853 (Patent Literature 5), that the catalyst exhibits high catalytic activity in an aqueous solvent in carbon-carbon bond-forming reaction. According to the methods disclosed in Patent Literatures 4 and 5, carbon-carbon bond-forming reaction can be performed with a high yield in an aqueous solvent by using, as a catalyst, a platinum group metal ion-supported catalyst in which a platinum group metal is supported on a non-particulate organic ion exchanger.

### [Citation List]

### [Patent Literature]

[Patent Literature 1] Japanese Patent No. 5638862 (Claims)
[Patent Literature 2] Japanese Patent No. 5255215 (Claims)
[Patent Literature 3] Japanese Patent Laid-Open No. 2008-212765 (Claims)
[Patent Literature 4] Japanese Patent Laid-Open No. 2014-15420
[Patent Literature 5] Japanese Patent Laid-Open No. 2016-190853

### [Non Patent Literature]

[Non Patent Literature 1] ChemCatChem, 2019, No. 11, p. 2427

### [Summary of Invention]

### [Technical Problem]

Here, reaction methods with short reaction times are demanded from the viewpoint of production efficiency. An example of a method for enhancing the production efficiency is to perform the reaction in a fixed-bed continuous circulation manner.

However, in Suzuki-Miyaura coupling, Sonogashira coupling, or Mizorogi-Heck coupling, reactivity differs depending on reaction raw materials, and reaction for a short time may be difficult to cause. Accordingly, a fixed-bed continuous circulation manner has a problem in that carbon-carbon bond-forming reactions such as Suzuki-Miyaura coupling, Sonogashira coupling, and Mizorogi-Heck coupling are difficult to cause.

Accordingly, an object of the present invention is to provide a catalyst that enables a method for forming a carbon-carbon bond to obtain the desired compound by forming a carbon-carbon bond to be performed in a fixed-bed continuous circulation manner, the catalyst enabling the reaction to be performed with a high yield in various raw materials.

### [Solution to Problem]

As a result of diligent researches by the present inventors under the present circumstances, it was found that a catalyst in which platinum group metal ions or platinum group metal complex ions are supported on a non-particulate organic porous ion exchanger, that is, a catalyst in which a platinum group metal in an ionic state is supported on a non-particulate organic porous ion exchanger, provides a high yield in so-called carbon-carbon bond reaction in a fixed-bed continuous circulation manner even when an aromatic bromine compound is used as a raw material, and the like, reaching the completion of the present invention.

That is, the present invention (1) provides a platinum group metal ion-supported catalyst in which platinum group metal ions or platinum group metal complex ions are supported on a non-particulate organic porous ion exchanger, wherein the non-particulate organic porous ion exchanger is formed of a continuous framework phase and a continuous pore phase; has a thickness of a continuous framework of 1 to 100 µm, an average diameter of continuous pores of 1 to 1000 µm, and a total pore volume of 0.5 to 50 ml/g; has an ion exchange capacity per weight in a dry state of 1 to 9 mg equivalent/g; and has ion exchange groups wherein the ion exchange groups are uniformly distributed in the organic porous ion exchanger.

In addition, the present invention (2) provides the platinum group metal ion-supported catalyst according to (1), wherein the non-particulate organic porous ion exchanger has a continuous bubble structure with macropores linked to each other and common apertures (mesopores) with an average diameter of 1 to 1000 µm within the walls of the macropores; has a total pore volume of 1 to 50 ml/g; has an ion exchange capacity per weight in a dry state of 1 to 9 mg equivalent/g; and has ion exchange groups wherein the ion exchange groups are uniformly distributed in the organic porous ion exchanger.

In addition, the present invention (3) provides the platinum group metal ion-supported catalyst according to (1), wherein the non-particulate organic porous ion exchanger forms a framework portion with aggregated and thus three dimensionally continuous organic polymer particles with an average particle diameter of 1 to 50 µm; has three dimensionally continuous pores in the framework with an average diameter of 20 to 100 µm; has a total pore volume of 1 to 10 ml/g; has an ion exchange capacity per weight in a dry state of 1 to 9 mg equivalent/g; and has ion exchange groups wherein the ion exchange groups are uniformly distributed in the organic porous ion exchanger.

In addition, the present invention (4) provides the platinum group metal ion-supported catalyst according to (1), wherein the non-particulate organic porous ion exchanger is a continuous macropore structural material in which bubble-like macropores overlap each other and these overlapping areas become apertures with an average diameter of 30 to 300 µm; has a total pore volume of 0.5 to 10 ml/g and an ion exchange capacity per weight in a dry state of 1 to 9 mg equivalent/g; has ion exchange groups wherein the ion exchange groups are uniformly distributed in the organic porous ion exchanger; and, in a SEM image of the cut section of the continuous macropore structural material (dried material), has an area of the framework part appearing in the cross section of 25 to 50% in the image region.

In addition, the present invention (5) provides the platinum group metal ion-supported catalyst according to (1), wherein the non-particulate organic porous ion exchanger is a co-continuous structural material formed of a three dimensionally continuous framework comprising an aromatic vinyl polymer containing 0.1 to 5.0 mol% of crosslinked structural units among the entire constituent units into which ion exchange groups have been introduced, with an average thickness of 1 to 60 µm, and three dimensionally continuous pores in the framework with an average diameter of 10 to 200 µm; has a total pore volume of 0.5 to 10 ml/g; has an ion exchange capacity per weight in a dry state of 1 to 9 mg equivalent/g; and has ion exchange groups wherein the ion exchange groups are uniformly distributed in the organic porous ion exchanger.

In addition, the present invention (6) provides the platinum group metal ion-supported catalyst according to (1), wherein the non-particulate organic porous ion exchanger is formed of a continuous framework phase and a continuous pore phase; in the framework, has a number of particle materials with a diameter of 4 to 40 µm adhering to the surface or a number of protruding materials with a size of 4 to 40 µm formed on the framework surface of the organic porous material; has an average diameter of continuous pores of 10 to 200 µm and a total pore volume of 0.5 to 10 ml/g; has an ion exchange capacity per weight in a dry state of 1 to 9 mg equivalent/g; and has ion exchange groups wherein the ion exchange groups are uniformly distributed in the organic porous ion exchanger.

In addition, the present invention (7) provides the platinum group metal ion-supported catalyst according to any of (1) to (6), wherein the amount of the platinum group metal ions or the platinum group metal complex ions supported is 0.05 to 10.0% by mass based on the platinum group metal atom.

In addition, the present invention (8) provides a method for forming a carbon-carbon bond to form a carbon-carbon bond by performing (1) reaction of an aromatic halide with an organoboron compound, (2) reaction of an aromatic halide with a compound having a terminal alkynyl group, or (3) reaction of an aromatic halide with a compound having an alkenyl group,
wherein the carbon-carbon bond-forming reaction is performed by introducing a raw material liquid (i) containing the aromatic halide and the organoboron compound, a raw material liquid (ii) containing the aromatic halide and the compound having a terminal alkynyl group, or a raw material liquid (iii) containing the aromatic halide and the compound having an alkenyl group, through an introduction path of a filling container filled with a platinum group metal ion-supported catalyst, into the filling container, passing the raw material liquid through the platinum group metal ion-supported catalyst, and discharging the reaction liquid from a discharge path of the filling container, and
the platinum group metal ion-supported catalyst is the platinum group metal ion-supported catalyst according to any of (1) to (7).

In addition, the present invention (9) provides the method for forming a carbon-carbon bond according to (8), wherein the carbon-carbon bond-forming reaction is performed in the presence of an inorganic base.

In addition, the present invention (10) provides the method for forming a carbon-carbon bond according to (8) or (9), wherein the raw material liquid (i) containing the aromatic halide and the organoboron compound, the raw material liquid (ii) containing the aromatic halide and the compound having a terminal alkynyl group, or the raw material liquid (iii) containing the aromatic halide and the compound having an alkenyl group is an inorganic base-dissolved raw material liquid in which the raw material and an inorganic base are dissolved in water or a hydrophilic solvent, and
the carbon-carbon bond-forming reaction is performed by introducing the inorganic base-dissolved raw material liquid through an introduction path of a filling container filled with the platinum group metal ion-supported catalyst into the filling container, passing the inorganic base-dissolved raw material liquid through the platinum group metal ion-supported catalyst, and discharging the reaction liquid from a discharge path of the filling container.

In addition, the present invention (11) provides the method for forming a carbon-carbon bond according to (8) or (9), wherein the raw material liquid (i) containing the aromatic halide and the organoboron compound, the raw material liquid (ii) containing the aromatic halide and the compound having a terminal alkynyl group, or the raw material liquid (iii) containing the aromatic halide and the compound having an alkenyl group is a hydrophobic solvent raw material liquid in which the raw material is dissolved in a hydrophobic organic solvent, and
the carbon-carbon bond-forming reaction is performed by introducing a mixture of the hydrophobic solvent raw material liquid and an aqueous inorganic base solution through an introduction path of a filling container filled with the platinum group metal ion-supported catalyst into the filling container, passing the hydrophobic solvent-dissolved raw material liquid and the aqueous inorganic base solution through the platinum group metal ion-supported catalyst, and discharging the reaction liquid from a discharge path of the filling container.

### [Advantageous Effects of Invention]

According to the present invention, there can be provided a catalyst that enables a method for forming a carbon-carbon bond to obtain the desired compound by forming a carbon-carbon bond to be performed in a fixed-bed continuous circulation manner, the catalyst enabling the reaction to be performed with a high yield in various raw materials.

### [Brief Description of Drawings]

[Figure 1] Figure 1 is a SEM photograph of an exemplary embodiment of a first monolith.
[Figure 2] Figure 2 is a SEM photograph of an exemplary embodiment of a second monolith.
[Figure 3] Figure 3 is a SEM photograph of an exemplary embodiment of a third monolith.
[Figure 4] Figure 4 is a diagram in which the framework part appearing in the cross section of the SEM photograph of Figure 3 was transferred.
[Figure 5] Figure 5 is a SEM photograph of an exemplary embodiment of a fourth monolith.
[Figure 6] Figure 6 is a schematic diagram of the co-continuous structure of the fourth monolith and weakly basic monolithic ion exchanger.
[Figure 7] Figure 7 is a SEM photograph of an exemplary embodiment of a monolithic intermediate (4).
[Figure 8] Figure 8 is a schematic cross-sectional view of a protruding material.
[Figure 9] Figure 9 is a SEM photograph of an exemplary embodiment of a fifth-1 monolith.
[Figure 10] Figure 10 is a SEM photograph of a monolithic intermediate of Reference Example 1.
[Figure 11] Figure 11 is a SEM photograph of a monolith of Reference Example 1.
[Figure 12] Figure 12 shows results of EPMA analysis for a platinum group metal ion-supported catalyst of Example 1.
[Figure 13] Figure 13 shows a Pd3d_{5/2} spectrum in ESCA analysis of the platinum group metal ion-supported catalyst of Example 1.
[Figure 14] Figure 14 is a flow diagram of an exemplary embodiment of a reaction apparatus illustrating an exemplary first embodiment of the method for forming a carbon-carbon bond of the present invention.
[Figure 15] Figure 15 is a flow diagram of an exemplary embodiment of a reaction apparatus illustrating an exemplary second embodiment of the method for forming a carbon-carbon bond of the present invention.

### [Description of Embodiments]

The platinum group metal ion-supported catalyst of the present invention is a platinum group metal ion-supported catalyst in which platinum group metal ions or platinum group metal complex ions are supported on a non-particulate organic porous ion exchanger, wherein the non-particulate organic porous ion exchanger is formed of a continuous framework phase and a continuous pore phase; has a thickness of a continuous framework of 1 to 100 µm, an average diameter of continuous pores of 1 to 1000 µm, and a total pore volume of 0.5 to 50 ml/g; has an ion exchange capacity per weight in a dry state of 1 to 9 mg equivalent/g; and has ion exchange groups wherein the ion exchange groups are uniformly distributed in the organic porous ion exchanger.

In the platinum group metal ion-supported catalyst of the present invention, the support on which platinum group metal ions or platinum group metal complex ions are supported is a non-particulate organic porous ion exchanger, and this non-particulate organic porous ion exchanger is an acidic monolithic organic porous cation exchanger or a basic monolithic organic porous anion exchanger. The monolithic organic porous material is a porous material that has a framework formed of an organic polymer, and has a number of communication holes in the framework that serve as flow channels for the reaction liquid. And, the monolithic organic porous ion exchanger is a porous material formed by introducing cation exchange groups or anion exchange groups into the framework of this monolithic organic porous material such that the cation exchange groups or anion exchange groups are uniformly distributed therein. Note that, in the present specification, the "monolithic organic porous material" may also be simply referred to as a "monolith", the "monolithic organic porous ion exchanger" may also be simply referred to as a "monolithic ion exchanger", and a "monolithic organic porous intermediate", which is an intermediate in the production of a monolith (a precursor), may also be simply referred to as a "monolithic intermediate".

The non-particulate organic porous ion exchanger according to the platinum group metal ion-supported catalyst of the present invention is obtained by introducing ion exchange groups into a monolith, the structure of which is an organic porous material formed of a continuous framework phase and a continuous pore phase, wherein the thickness of the continuous framework is 1 to 100 µm, the average diameter of the continuous pores is 1 to 1000 µm, and the total pore volume is 0.5 to 50 ml/g.

The thickness of the continuous framework of the non-particulate organic porous ion exchanger in a dry state according to the platinum group metal ion-supported catalyst of the present invention is 1 to 100 µm. When the thickness of the continuous framework of the non-particulate organic porous ion exchanger is less than 1 µm, it is not preferable because there is not only a disadvantage such as a decrease in the ion exchange capacity per volume, but also a decrease in mechanical strength leading to a large deformation of the non-particulate organic porous ion exchanger, especially when the liquid is passed through at a high flow rate. Furthermore, the contact efficiency between the reaction liquid and the non-particulate organic porous ion exchanger is reduced, thereby reducing the catalytic activity, which is not preferable. On the other hand, when the thickness of the continuous framework of the non-particulate organic porous ion exchanger is greater than 100 µm, it is not preferable because the framework becomes too thick and it takes more time for the substrate to be diffused, which reduces the catalytic activity. Note that the thickness of the continuous framework is determined by SEM observation.

The average diameter of the continuous pores of the non-particulate organic porous ion exchanger in a dry state according to the platinum group metal ion-supported catalyst of the present invention is 1 to 1000 µm. When the average diameter of the continuous pores of the non-particulate organic porous ion exchanger is less than 1 µm, it is not preferable because the pressure loss upon passing the liquid is large. On the other hand, when the average diameter of the continuous pores of the non-particulate organic porous ion exchanger is greater than 1000 µm, it is not preferable because the contact between the reaction liquid and the non-particulate organic porous ion exchanger is insufficient, which reduces the catalytic activity. Note that the average diameter of the continuous pores of the non-particulate organic porous ion exchanger in a dry state is measured by the mercury injection method and refers to the maximum value of the pore distribution curve obtained by the mercury injection method.

The total pore volume of the non-particulate organic porous ion exchanger according to the platinum group metal ion-supported catalyst of present invention in a dry state is 0.5 to 50 ml/g. When the total pore volume of the non-particulate organic porous ion exchanger is less than 0.5 ml/g, it is not preferable because the pressure loss upon passing the liquid is large, and furthermore, it is not preferable because the amount of permeate per unit cross sectional area is small, which reduces the throughput. On the other hand, when the total pore volume of the non-particulate organic porous ion exchanger is greater than 50 ml/g, it is not preferable because the ion exchange capacity per volume is reduced and also the amount of platinum group metal ions to be supported is reduced, which reduces the catalytic activity. In addition, it is not preferable because the mechanical strength is decreased and the non-particulate organic porous ion exchanger is largely deformed, especially when the liquid is passed through at a high speed, causing the pressure loss upon passing the liquid to rise rapidly. Furthermore, the contact efficiency between the reaction liquid and the non-particulate organic porous ion exchanger is reduced, thereby markedly reducing the catalytic activity. Note that the total pore volume is measured by the mercury injection method.

Exemplary structures of such a non-particulate organic porous ion exchanger include the continuous bubble structures disclosed in Japanese Patent Laid-Open No. 2002-306976 and Japanese Patent Laid-Open No. 2009-62512, the co-continuous structure disclosed in Japanese Patent Laid-Open No. 2009-67982, the particle aggregated structure disclosed in Japanese Patent Laid-Open No. 2009-7550, and the particle composite structure disclosed in Japanese Patent Laid-Open No. 2009-108294.

The ion exchange capacity per weight of the non-particulate organic porous ion exchanger in a dry state according to the platinum group metal ion-supported catalyst of the present invention is 1 to 9 mg equivalent/g. When the ion exchange capacity of the non-particulate organic porous ion exchanger in a dry state is less than 1 mg equivalent/g, it is not preferable because the amount of the platinum group metal ion that can be supported is small. On the other hand, when it is greater than 9 mg equivalent/g, it is not preferable because the introduction reaction of the ion exchange groups becomes severe conditions which drastically accelerate the oxidative degradation of the monolith. The ion exchange capacity per weight of the non-particulate organic porous ion exchanger in a dry state is 1 to 9 mg equivalent/g, preferably 1 to 8 mg equivalent/g, and particularly preferably 1 to 7 mg equivalent/g. Note that the ion exchange capacity of a porous material in which ion exchange groups are introduced only on the framework surface is at most 500 µg equivalent/g, although it is not possible to determine it in general, depending on the types of the porous material and ion exchange groups.

In the non-particulate organic porous ion exchanger according to the platinum group metal ion-supported catalyst of the present invention, the introduced ion exchange groups are uniformly distributed not only on the surface of the monolith, but also inside the framework of the monolith. The term "ion exchange groups are uniformly distributed" herein refers to the fact that the distribution of the ion exchange groups is such that they are uniformly distributed on the surface and inside the framework at least on the order of µm. The distribution of ion exchange groups can be easily confirmed by using EPMA. Also, when the ion exchange groups are uniformly distributed not only on the surface of the monolith but also inside the framework of the monolith, the physical properties and chemical properties of the surface and the inside can be made uniform, thus improving the resistance against swelling and shrinkage.

The cation exchange groups introduced into the non-particulate organic porous ion exchanger according to the platinum group metal ion-supported catalyst of the present invention are strongly acidic cation exchange groups or weakly acidic cation exchange groups. Examples of the strongly acidic cation exchange groups include a sulfonic acid group. Examples of the weakly acidic cation exchange groups include a carboxyl group. The anion exchange groups introduced into the non-particulate organic porous ion exchanger according to the platinum group metal ion-supported catalyst of the present invention are strongly basic anion exchange groups or weakly basic anion exchange groups. Examples of the strongly basic anion exchange groups include a quaternary ammonium group such as a trimethylammonium group, a triethylammonium group, a tributylammonium group, dimethylhydroxyethylammonium group, a dimethylhydroxypropylammonium group, and a methyldihydroxyethylammonium group. Examples of the weakly basic anion exchange groups include a tertiary amino group such as a dimethylamino group, a diethylamino group, a dipropylamino group, a dibutylamino group, a methylhydroxyethylamino group, a methylhydroxypropylamino group, a dicyclohexylamino group, a pyrrolidyl group, a piperidyl group, a 2,2,6,6-tetramethylpiperidyl group, and a morpholyl group, a secondary amino group such as a methylamino group, an ethylamino group, a propylamino group, a butylamino group, a hydroxyethylamino group, and a hydroxybutylamino group, and a primary amino group.

In the non-particulate organic porous ion exchanger according to the platinum group metal ion-supported catalyst of the present invention, the material constituting the continuous framework is an organic polymer material having a crosslinked structure. Although the crosslinking density of the polymer material is not particularly limited, it is preferable to include 0.1 to 30 mol%, suitably 0.1 to 20 mol% of crosslinked structural units with respect to the entire constituent units that constitute the polymer material. When the crosslinked structural units are less than 0.1 mol%, it is not preferable because the mechanical strength is insufficient. On the other hand, when they are greater than 30 mol%, it is not preferable because the introduction of ion exchange groups may be difficult. There is no particular limitation on the type of the polymer material, and examples thereof include a crosslinked polymer, including, for example, an aromatic vinyl polymer such as polystyrene, poly(α-methylstyrene), polyvinyl toluene, polyvinylbenzyl chloride, polyvinyl biphenyl, and polyvinyl naphthalene; a polyolefin such as polyethylene and polypropylene; a poly(halogenated polyolefin) such as polyvinyl chloride and polytetrafluoroethylene; a nitrile-based polymer such as polyacrylonitrile; and a (meth)acrylic polymer such as polymethyl methacrylate, polyglycidyl methacrylate, and polyethyl acrylate. The polymers described above may be polymers obtained by copolymerizing a single vinyl monomer and a crosslinking agent, polymers obtained by polymerizing a plurality of vinyl monomers and a crosslinking agent, or a blend of two or more polymers. Among these organic polymer materials, crosslinked polymers of aromatic vinyl polymers are preferable because of the ease of forming a continuous structure, the ease of introducing ion exchange groups, the high mechanical strength, and the high stability against acids or alkalis, and in particular, styrene-divinylbenzene copolymers and vinylbenzyl chloride-divinylbenzene copolymers are preferable materials.

### <Exemplary Embodiments of Monolithic Organic Porous Material and Monolithic Organic Porous Ion Exchanger>

Exemplary embodiments of the monolith include the first monolith to the fifth monolith, which will be shown below. In addition, the monolithic ion exchanger includes the first monolithic ion exchanger to the fifth monolithic ion exchanger. Note that the ion exchange groups introduced into the monolith in the first monolithic ion exchanger to the fifth monolithic ion exchanger are strongly acidic cation exchange groups, weakly acidic cation exchange groups, strongly basic anion exchange groups, or weakly basic anion exchange groups.

### <Description of First Monolith and First Monolithic Ion Exchanger>

In the platinum group metal ion-supported catalyst of the present invention, the first monolithic ion exchanger serving as a support for platinum group metal ions or platinum group metal complex ions is a monolithic ion exchanger having a continuous bubble structure with macropores linked to each other and common apertures (mesopores) with an average diameter of 1 to 1000 µm in a dry state within the walls of the macropores, having a total pore volume of 1 to 50 ml/g in a dry state, having ion exchange groups wherein the ion exchange groups are uniformly distributed, and having an ion exchange capacity per weight in a dry state of 1 to 9 mg equivalent/g. In addition, the first monolith is a monolith before introducing the ion exchange groups, and is an organic porous material having a continuous bubble structure with macropores linked to each other and common apertures (mesopores) with an average diameter of 1 to 1000 µm in a dry state within the walls of the macropores, and having a total pore volume of 1 to 50 mL/g in a dry state.

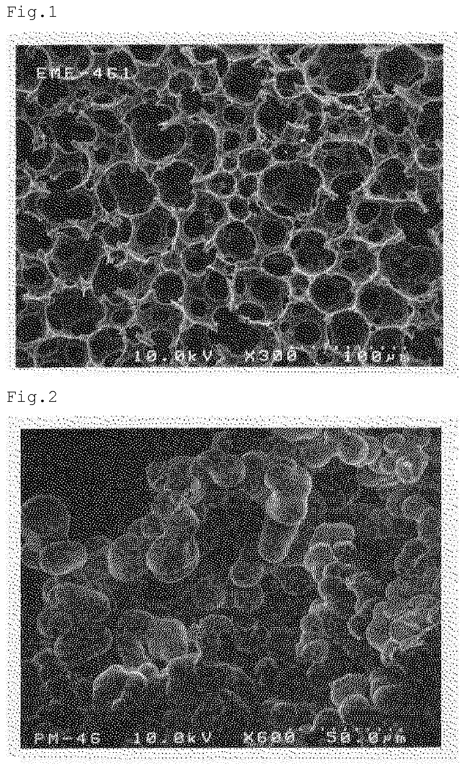

The first monolithic ion exchanger is a continuous macropore structural material in which bubble-like macropores overlap each other and these overlapping areas become common apertures (mesopores) with an average diameter of 1 to 1000 µm, preferably 10 to 200 µm, and particularly preferably 20 to 100 µm, in a dry state, the majority of which has an open pore structure. In the open pore structure, when water flows, the flow channels are in the bubbles formed by the macropores and the mesopores. The number of overlaps between macropores is 1 to 12 for a single macropore and 3 to 10 for most. Figure 1 shows a scanning electron microscope (SEM) photograph of an exemplary embodiment of the first weakly basic monolithic anion exchanger. The first monolithic ion exchanger shown in Figure 1 has a large number of bubble-like macropores, and is a continuous macropore structural material in which the bubble-like macropores overlap each other and these overlapping areas become common apertures (mesopores), the majority of which has an open pore structure. When the average diameter of the mesopores in a dry state is less than 1 µm, it is not preferable because the pressure loss upon passing the liquid is drastically large. When the average diameter of the mesopores in a dry state is greater than 1000 µm, it is not preferable because the contact between the reaction liquid and the monolithic ion exchanger is insufficient, which reduces the catalytic activity. When the structure of the first monolithic ion exchanger is a continuous bubble structure as described above, groups of macropores and mesopores can be formed uniformly, and the pore volume and specific surface area can also be made significantly larger than those of particle aggregated porous materials as described in Japanese Patent Laid-Open No. 8-252579 and the like.

Note that, in the present invention, the average diameter of the apertures of the first monolith in a dry state and the average diameter of the apertures of the first monolithic ion exchanger in a dry state are measured by the mercury injection method and refer to the maximum value of the pore distribution curve obtained by the mercury injection method.

The total pore volume per weight of the first monolithic ion exchanger in a dry state is 1 to 50 ml/g, and suitably 2 to 30 ml/g. When the total pore volume is less than 1 ml/g, it is not preferable because the pressure loss upon passing the liquid is large, and furthermore, it is not preferable because the amount of permeate per unit cross sectional area is small, which reduces the throughput capacity. On the other hand, when the total pore volume is greater than 50 mL/g, it is not preferable because the mechanical strength is decreased and the monolithic ion exchanger is largely deformed, especially when the liquid is passed through at a high flow rate. Furthermore, the contact efficiency of the reaction liquid with the monolithic ion exchanger and the platinum group metal particles supported thereon is reduced, thereby also reducing the catalytic effect, which is not preferable. Since the total pore volume of conventional particulate porous ion exchange resins is 0.1 to 0.9 ml/g at most, those with a high pore volume of 1 to 50 ml/g and a high specific surface area, which have not been available in the past, can be used.

In the first monolithic ion exchanger, the material constituting the framework is an organic polymer material having a crosslinked structure. Although the crosslinking density of that polymer material is not particularly limited, it is preferable to include 0.3 to 10 mol%, suitably 0.3 to 5 mol% of crosslinked structural units with respect to the entire constituent units that constitute the polymer material. When the crosslinked structural units are less than 0.3 mol%, it is not preferable because the mechanical strength is insufficient. On the other hand, when they are greater than 10 mol%, it is not preferable because the introduction of ion exchange groups may be difficult.

There is no particular limitation on the type of the organic polymer material constituting the framework of the first monolithic ion exchanger, and examples thereof include a crosslinked polymer, including, for example, an aromatic vinyl polymer such as polystyrene, poly(α-methylstyrene), polyvinyl toluene, polyvinylbenzyl chloride, polyvinyl biphenyl, and polyvinyl naphthalene; a polyolefin such as polyethylene and polypropylene; a poly(halogenated polyolefin) such as polyvinyl chloride and polytetrafluoroethylene; a nitrile-based polymer such as polyacrylonitrile; and a (meth)acrylic polymer such as polymethyl methacrylate, polyglycidyl methacrylate, and polyethyl acrylate. The organic polymers described above may be polymers obtained by copolymerizing a single vinyl monomer and a crosslinking agent, polymers obtained by polymerizing a plurality of vinyl monomers and a crosslinking agent, or a blend of two or more polymers. Among these organic polymer materials, crosslinked polymers of aromatic vinyl polymers are preferable because of the ease of forming a continuous macropore structure, the ease of introducing ion exchange groups, the high mechanical strength, and the high stability against acids or alkalis, and in particular, styrene-divinylbenzene copolymers and vinylbenzyl chloride-divinylbenzene copolymers are preferable materials.

The ion exchange groups introduced into the first monolithic ion exchanger are the same in the second monolithic ion exchanger to the fifth monolithic ion exchanger. The cation exchange groups introduced into the monolithic ion exchanger are strongly acidic cation exchange groups or weakly acidic cation exchange groups. Examples of the strongly acidic cation exchange groups include a sulfonic acid group. Examples of the weakly acidic cation exchange groups include a carboxyl group. The anion exchange groups introduced into the monolithic ion exchanger are strongly basic anion exchange groups or weakly basic anion exchange groups. Examples of the strongly basic anion exchange groups include a quaternary ammonium group such as a trimethylammonium group, a triethylammonium group, a tributylammonium group, dimethylhydroxyethylammonium group, a dimethylhydroxypropylammonium group, and a methyldihydroxyethylammonium group. Examples of the weakly basic anion exchange groups include a tertiary amino group such as a dimethylamino group, a diethylamino group, a dipropylamino group, a dibutylamino group, a methylhydroxyethylamino group, a methylhydroxypropylamino group, a dicyclohexylamino group, a pyrrolidyl group, a piperidyl group, a 2,2,6,6-tetramethylpiperidyl group, and a morpholyl group, a secondary amino group such as a methylamino group, an ethylamino group, a propylamino group, a butylamino group, a hydroxyethylamino group, and a hydroxybutylamino group, and a primary amino group.

In the first monolithic ion exchanger (the same applies to the second monolithic ion exchanger to the fifth monolithic ion exchanger), the introduced ion exchange groups are uniformly distributed not only on the surface of the porous material, but also inside the framework of the porous material. The term "ion exchange groups are uniformly distributed" herein refers to the fact that the distribution of the ion exchange groups is such that they are uniformly distributed on the surface and inside the framework at least on the order of µm. The distribution of ion exchange groups can be confirmed by using EPMA. Also, when the ion exchange groups are uniformly distributed not only on the surface of the monolith but also inside the framework of the porous material, the physical properties and chemical properties of the surface and the inside can be made uniform, thus improving the resistance against swelling and shrinkage.

The ion exchange capacity per weight of the first monolithic ion exchanger in a dry state is 1 to 9 mg equivalent/g. When the ion exchange capacity per weight in a dry state is in the range described above, the ambient environment of a catalytic active point, such as pH inside the catalyst, can be changed, thereby increasing the catalytic activity. The ion exchange capacity per weight of the first monolithic ion exchanger in a dry state is 1 to 9 mg equivalent/g, preferably 1 to 8 mg equivalent/g, and particularly preferably 1 to 7 mg equivalent/g. Note that the ion exchange capacity of a porous material in which ion exchange groups are introduced only on the surface is at most 500 µg equivalent/g, although it is not possible to determine it in general, depending on the types of the porous material and ion exchange groups.

### <Method for Producing First Monolith and First Monolithic Ion Exchanger>

Although there is no limitation on the method for producing the first monolith, an example of the production method, according to the method described in Japanese Patent Laid-Open No. 2002-306976, is shown below. That is, the first monolith is obtained by mixing an oil soluble monomer without ion exchange groups, a surfactant, water and, if required, a polymerization initiator, to obtain a water in oil type emulsion, which is then polymerized to form the monolith. Such a method for producing the first monolith is preferable because of the ease of controlling the porous structure of the monolith.

The oil soluble monomer without ion exchange groups used in the production of the first monolith refers to a monomer that does not contain either ion exchange groups such as carboxylic acid groups or sulfonic acid groups or anion exchange groups such as quaternary ammonium groups, and that has low solubility in water and is lipophilic. Specific examples of such a monomer include styrene, α-methylstyrene, vinyl toluene, vinylbenzyl chloride, divinylbenzene, ethylene, propylene, isobutene, butadiene, isoprene, chloroprene, vinyl chloride, vinyl bromide, vinylidene chloride, tetrafluoroethylene, acrylonitrile, methacrylonitrile, vinyl acetate, methyl acrylate, ethyl acrylate, butyl acrylate, 2-ethylhexyl acrylate, trimethylolpropane triacrylate, butanediol diacrylate, methyl methacrylate, ethyl methacrylate, propyl methacrylate, butyl methacrylate, 2-ethylhexyl methacrylate, cyclohexyl methacrylate, benzyl methacrylate, glycidyl methacrylate, and ethylene glycol dimethacrylate. These monomers may be used alone as one kind, or may be used in combination of two or more kinds. However, in the present invention, it is preferable to select a crosslinkable monomer such as divinylbenzene or ethylene glycol dimethacrylate as at least one component of the oil soluble monomer and set the content thereof to 0.3 to 10 mol%, or suitably 0.3 to 5 mol%, of the entire oil soluble monomers in that ion exchange groups can be introduced quantitatively in the subsequent step and a practically sufficient mechanical strength can be ensured.

The surfactant used in the production of the first monolith is not particularly limited as long as it is capable of forming a water in oil type (W/O) emulsion when mixed with an oil soluble monomer without ion exchange groups and water. Examples of the surfactant that can be used include a non-ionic surfactant such as sorbitan monooleate, sorbitan monolaurate, sorbitan monopalmitate, sorbitan monostearate, sorbitan trioleate, polyoxyethylene nonylphenyl ether, polyoxyethylene stearyl ether, and polyoxyethylene sorbitan monooleate; a negative ionic surfactant such as potassium oleate, sodium dodecylbenzene sulfonate, sodium dioctyl sulfosuccinate; a positive ionic surfactant such as distearyl dimethyl ammonium chloride; and an amphoteric surfactant such as lauryl dimethyl betaine. These surfactants may be used alone as one kind or may be used in combination of two or more kinds. Note that a water in oil type emulsion refers to an emulsion in which the oil phase becomes a continuous phase and water droplets are dispersed therein. As for the amount of the surfactant to be added, it is difficult to say in general because it varies significantly depending on the type of oil soluble monomer and the size of the target emulsion particles (macropores), but it can be selected in the range of about 2 to 70% with respect to the total amount of the oil soluble monomer and the surfactant. Also, in order to control the bubble shape and size of the monolith, although it is not necessarily required, an alcohol such as methanol or stearyl alcohol; a carboxylic acid such as stearic acid; a hydrocarbon such as octane, dodecane, or toluene; or a cyclic ether such as tetrahydrofuran or dioxane may coexist in the system.

In addition, in the production of the first monolith, upon forming the monolith by polymerization, a compound that generates radicals by heat and light irradiation is suitably used as the polymerization initiator that is used if required. The polymerization initiator may be water soluble or oil soluble, and examples thereof include, for example, azobisisobutyronitrile, azobisdimethylvaleronitrile, azobiscyclohexanenitrile, azobiscyclohexanecarbonitrile, benzoyl peroxide, potassium persulfate, ammonium persulfate, hydrogen peroxide-ferrous chloride, sodium persulfate-sodium hydrogen sulfite, tetramethylthiuram disulfide. However, in some systems, polymerization proceeds only by heating or light irradiation without the addition of a polymerization initiator, and therefore the addition of a polymerization initiator is not necessary in such systems.

In the production of the first monolith, there is no limitation on the mixing method upon mixing an oil soluble monomer without ion exchange groups, a surfactant, water, and a polymerization initiator to form a water in oil type emulsion. For example, a method in which all components are mixed at once, or a method in which oil soluble components, including an oil soluble monomer, a surfactant, and an oil soluble polymerization initiator, and water soluble components, including water and a water soluble polymerization initiator, are separately dissolved to be uniform, and then these components are mixed together can be used. There is no particular limitation on the mixing apparatus for forming an emulsion, either. For example, an ordinary mixer, homogenizer, high pressure homogenizer, or so-called planetary stirring apparatus, which mixes the objects to be treated by placing them in a mixing vessel and allowing the vessel to rotate on its axis while making the vessel inclined and revolving around the revolution axis can be used, and an appropriate apparatus may be selected to obtain the target emulsion particle diameter. Also, there is no particular limitation on the mixing conditions, and the stirring speed and stirring time at which the target emulsion particle diameter can be obtained can be arbitrarily set. Among these mixing apparatuses, the planetary stirring apparatus is preferably used because it can uniformly produce water droplets in the W/O emulsion and its average diameter can be arbitrarily set over a wide range.

In the production of the first monolith, as for the polymerization conditions under which the water in oil type emulsion thus obtained is polymerized, a variety of conditions can be selected depending on the type of monomer and the initiator system. For example, when azobisisobutyronitrile, benzoyl peroxide, potassium persulfate, or the like is used as the polymerization initiator, heat polymerization may be performed at 30 to 100°C for 1 to 48 hours in a sealed container under an inert atmosphere, and when hydrogen peroxide-ferrous chloride, sodium persulfate-sodium hydrogen sulfite, or the like is used as the initiator, polymerization may be performed at 0 to 30°C for 1 to 48 hours in a sealed container under an inert atmosphere. After the completion of polymerization, the contents are taken out and soxhlet extracted with a solvent such as isopropanol to remove the unreacted monomer and residual surfactant, thereby obtaining the first monolith.

There is no particular limitation on the method for producing the first monolithic ion exchanger, and examples thereof include a method in which, instead of the monomer without ion exchange groups in the method for producing the first monolith described above, a monomer with ion exchange groups, such as a monomer formed by introducing ion exchange groups into the oil soluble monomer without ion exchange groups described above, is polymerized to form a monolithic ion exchanger in one step, and a method in which a monomer without ion exchange groups is used and polymerized to form the first monolith and ion exchange groups are then introduced thereinto. Among these methods, the method in which a monomer without ion exchange groups is used and polymerized to form the first monolith and ion exchange groups are then introduced thereinto is preferable because the porous structure of the monolithic ion exchanger can be easily controlled and ion exchange groups can also be introduced quantitatively.

There is no particular limitation on the method for introducing ion exchange groups into the first monolith, and a known method such as polymer reaction or graft polymerization can be used. Examples of a method for introducing strongly acidic cation exchange groups, for example, sulfonic acid groups include a method in which a styrene-divinylbenzene copolymer is heated in concentrated sulfuric acid and a method in which a styrene-divinylbenzene copolymer is allowed to react with chlorosulfonic acid. Examples of a method for introducing weakly acidic cation exchange groups, for example, carboxyl groups include a method in which an acrylate polymer is hydrolyzed. Examples of a method for introducing strongly basic anion exchange groups, for example, quaternary ammonium groups include a method in which a vinylbenzyl chloride-divinylbenzene copolymer is allowed to react with a tertiary amine. Examples of a method for introducing weakly basic anion exchange groups, for example, amine groups include: a method in which, when the monolith is a styrene-divinylbenzene copolymer or the like, chloromethyl groups are introduced using chloromethyl methyl ether or the like, and then the monolith is allowed to react with a secondary amine for introduction; a method in which chloromethylstyrene and divinylbenzene are copolymerized to produce a monolith, which is then allowed to react with a secondary amine for introduction; and a method in which radical initiation groups or chain transfer groups are introduced into the monolith, glycidyl methacrylate is graft polymerized, and then the monolith is allowed to react with a secondary amine for introduction. The amine groups are weakly basic amine groups, and examples thereof include a tertiary amino group such as a dimethylamino group, a diethylamino group, a dipropylamino group, a dibutylamino group, a methylhydroxyethylamino group, and a methylhydroxypropylamino group, a secondary amino group such as a methylamino group, an ethylamino group, a propylamino group, a butylamino group, a hydroxyethylamino group, and a hydroxybutylamino group, and a primary amino group.

### <Description of Second Monolith and Second Monolithic Ion Exchanger>

In the platinum group metal ion-supported catalyst of the present invention, the second monolithic ion exchanger serving as a support for platinum group metal ions or platinum group metal complex ions is a particle aggregated monolith which is an organic porous material forming a framework portion with aggregated and thus three dimensionally continuous organic polymer particles with an average particle diameter of 1 to 50 µm in a dry state, having three dimensionally continuous pores in the framework with an average diameter of 20 to 100 µm in a dry state, and having a total pore volume in a dry state of 1 to 10 ml/g, and is a monolithic ion exchanger having ion exchange groups, and having an ion exchange capacity per weight in a dry state of 1 to 9 mg equivalent/g, wherein the ion exchange groups are uniformly distributed in the organic porous ion exchanger. In addition, the second monolith is a monolith before introducing the ion exchange groups, and is a particle aggregated monolith which is an organic porous material forming a framework portion with aggregated and thus three dimensionally continuous organic polymer particles with an average particle diameter of 1 to 50 µm in a dry state, having three dimensionally continuous pores in the framework with an average diameter of 20 to 100 µm in a dry state, and having a total pore volume in a dry state of 1 to 10 ml/g,

The basic structure of the second monolithic ion exchanger is a particle aggregated structure that forms a framework portion with aggregated and thus three dimensionally continuous organic polymer particles having crosslinked structural units with an average particle diameter of 1 to 50 µm, and preferably 1 to 30 µm in a dry state, and has three dimensionally continuous pores in the framework with an average diameter of 20 to 100 µm, and preferably 20 to 90 µm in a dry state, and the three dimensionally continuous pores serve as flow channels for liquids or gases. Figure 2 shows a SEM photograph of an exemplary embodiment of the second monolithic ion exchanger. The second monolithic ion exchanger shown in Figure 2 has a particle aggregated structure that forms a framework portion with aggregated and thus three dimensionally continuous organic polymer particles, and has three dimensionally continuous pores in the framework. When the average particle diameter of the organic polymer particles is less than 1 µm in a dry state, it is not preferable because the average diameter of the continuous pores in the framework is as small as less than 20 µm in a dry state. When it is greater than 50 µm, it is not preferable because the contact between the reaction liquid and the monolithic ion exchanger is insufficient, resulting in a reduction in the catalytic activity. When the average diameter of the three dimensionally continuous pores present in the framework is less than 20 µm in a dry state, it is not preferable because the pressure loss when the reaction liquid is allowed to permeate through is large. On the other hand, when it is greater than 100 µm, it is not preferable because the contact between the reaction liquid and the monolithic ion exchanger is insufficient, which reduces the catalytic activity.

Note that the average particle diameter in a dry state of the organic polymer particles constituting the framework portion in the second monolith and the second monolithic ion exchanger is conveniently measured by using SEM. Specifically, SEM photographs of an arbitrarily extracted portion of the cross section of the second monolithic ion exchanger in a dry state are taken, and the diameters of all the organic polymer particles in the SEM photographs are measured, and the average value thereof is defined as the average particle diameter.

The average diameter of the three dimensionally continuous pores present in the framework in the second monolith in a dry state or the average diameter of the three dimensionally continuous pores present in the framework in the second monolithic ion exchanger in a dry state is determined by the mercury injection method and refers to the maximum value of the pore distribution curve obtained by the mercury injection method.

The total pore volume per weight of the second monolithic ion exchanger in a dry state is 1 to 10 ml/g. When the total pore volume is less than 1 ml/g, it is not preferable because the pressure loss upon passing the liquid is large, and furthermore, it is not preferable because the amount of permeate per unit cross sectional area is small, which reduces the throughput capacity. On the other hand, when the total pore volume is greater than 10 ml/g, it is not preferable because the mechanical strength is decreased and the monolithic ion exchanger is largely deformed, especially when the liquid is passed through at a high flow rate.

In the second monolithic ion exchanger, the material of the framework portion is an organic polymer material having crosslinked structural units. That is, the organic polymer material has constituent units comprising a vinyl monomer and a crosslinking agent structural units having two or more vinyl groups in the molecule, and it is preferable for the polymer material to include 1 to 5 mol%, suitably 1 to 4 mol% of crosslinked structural units with respect to the entire constituent units that constitute the polymer material. When the crosslinked structural units are less than 1 mol%, it is not preferable because the mechanical strength is insufficient. On the other hand, when they are greater than 5 mol%, it is not preferable because the diameter of the three dimensionally continuous pores present in the framework is small and the pressure loss is large.

There is no particular limitation on the type of the polymer material constituting the framework of the second monolithic ion exchanger, and examples thereof include a crosslinked polymer, including, for example, a styrene-based polymer such as polystyrene, poly(α-methylstyrene), and polyvinylbenzyl chloride; a polyolefin such as polyethylene and polypropylene; a poly(halogenated polyolefin) such as polyvinyl chloride and polytetrafluoroethylene; a nitrile-based polymer such as polyacrylonitrile; a (meth)acrylic polymer such as polymethyl methacrylate, polyglycidyl methacrylate, and polyethyl acrylate; and a styrene-divinylbenzene copolymer and a vinylbenzyl chloride-divinylbenzene copolymer. The polymers described above may be polymers obtained by copolymerizing a single monomer and a crosslinking agent, polymers obtained by polymerizing a plurality of monomers and a crosslinking agent, or a blend of two or more polymers. Among these organic polymer materials, styrene-divinylbenzene copolymers and vinylbenzyl chloride-divinylbenzene copolymers are preferable materials because of the ease of forming a particle aggregated structure, the ease of introducing ion exchange groups, the high mechanical strength, and the high stability against acids or alkalis.

The anion exchange groups introduced into the second monolithic ion exchanger are the same as the anion exchange groups introduced into the first monolithic ion exchanger.

In the second monolithic ion exchanger, the introduced ion exchange groups are uniformly distributed not only on the surface of the porous material, but also inside the framework of the porous material, as in the first monolithic ion exchanger.

The ion exchange capacity per weight of the second monolithic ion exchanger is 1 to 9 mg equivalent/g in a dry state. The second monolithic ion exchanger can have a significantly large ion exchange capacity per weight while the pressure loss is kept low. When the ion exchange capacity per weight in a dry state is in the range described above, the ambient environment of a catalytic active point, such as pH inside the catalyst, can be changed, thereby increasing the catalytic activity. The ion exchange capacity per weight of the second monolithic ion exchanger in a dry state is 1 to 9 mg equivalent/g, preferably 1 to 8 mg equivalent/g, and particularly preferably 1 to 7 mg equivalent/g.

### <Method for Producing Second Monolith and Second Monolithic Ion Exchanger>

Examples of the method for producing the second monolith include a method in which a vinyl monomer, a particular amount of a crosslinking agent, an organic solvent and a polymerization initiator are mixed and polymerized while left to stand still to obtain the second monolith.

As for the vinyl monomer used in the production of the second monolith, there is no particular limitation as long as it contains a polymerizable vinyl group in the molecule and is lipophilic monomer with high solubility in an organic solvent. Specific examples of such a vinyl monomer include a styrene-based monomer such as styrene, α-methylstyrene, vinyl toluene, and vinylbenzyl chloride; an α-olefin such as ethylene, propylene, 1-butene, and isobutene; a diene-based monomer such as butadiene, isoprene, and chloroprene; a halogenated olefin such as vinyl chloride, vinyl bromide, vinylidene chloride, and tetrafluoroethylene; a nitrile-based monomer such as acrylonitrile and methacrylonitrile; a vinyl ester such as vinyl acetate and vinyl propionate; and a (meth)acrylic monomer such as methyl acrylate, ethyl acrylate, butyl acrylate, 2-ethylhexyl acrylate, methyl methacrylate, ethyl methacrylate, propyl methacrylate, butyl methacrylate, 2-ethylhexyl methacrylate, cyclohexyl methacrylate, benzyl methacrylate, and glycidyl methacrylate. These monomers are used alone as one kind, or are used in combination of two or more kinds. A vinyl monomer that is suitably used is a styrene-based monomer such as styrene or vinylbenzyl chloride.

As the crosslinking agent used in the production of the second monolith, those containing at least two polymerizable vinyl groups in the molecule and having a high solubility in an organic solvent are preferable. Specific examples of the crosslinking agent include divinylbenzene, divinyl naphthalene, divinyl biphenyl, ethylene glycol dimethacrylate, trimethylolpropane triacrylate, and butanediol diacrylate. These crosslinking agents are used alone as one kind, or are used in combination of two or more kinds. A preferable crosslinking agent is an aromatic polyvinyl compound such as divinylbenzene, divinyl naphthalene, and divinyl biphenyl because of its high mechanical strength and stability against hydrolysis. The amount of the crosslinking agent to be used with respect to the total amount of the vinyl monomer and crosslinking agent ({crosslinking agent / (vinyl monomer + crosslinking agent)} × 100) is 1 to 5 mol%, and preferably 1 to 4 mol%. The amount of the crosslinking agent to be used largely influences the porous structure of the resulting monolith. When the amount of the crosslinking agent to be used is greater than 5 mol%, it is not preferable because the size of the continuous pores formed in the framework is small. On the other hand, when the amount of the crosslinking agent to be used is less than 1 mol%, it is not preferable because the mechanical strength of the monolith is insufficient, causing a large deformation upon passing the liquid or the destruction of the monolith.

The organic solvent used in the production of the second monolith is an organic solvent that dissolves the vinyl monomer and the crosslinking agent, but does not dissolve a polymer produced by polymerization of the vinyl monomer. In other words, it is a poor solvent for a polymer produced by polymerization of the vinyl monomer. Since the organic solvent greatly varies depending on the type of the vinyl monomer, it is difficult to specifically recite general examples, but for example, when the vinyl monomer is styrene, examples of the organic solvent include an alcohol such as methanol, ethanol, propanol, butanol, hexanol, cyclohexanol, octanol, 2-ethylhexanol, decanol, dodecanol, ethylene glycol, tetramethylene glycol, and glycerin; a chain ether such as diethyl ether and ethylene glycol dimethyl ether; a chain saturated hydrocarbon such as hexane, octane, decane, and dodecane. Among these, alcohols are preferable because the particle aggregated structure is easily formed by static polymerization while the three dimensionally continuous pores are large. Also, even a good solvent for polystyrene, such as benzene or toluene, is used as the organic solvent when it is used together with the poor solvents described above and the amount thereof to be used is small.

As the polymerization initiator used in the production of the second monolith, a compound that generates radicals by heat or light irradiation is preferable. It is preferable that the polymerization initiator should be oil soluble. Specific examples of the polymerization initiator include 2,2'-azobis(isobutyronitrile), 2,2'-azobis(2,4-dimethylvaleronitrile), 2,2'-azobis(2-methylbutyronitrile), 2,2'-azobis(4-methoxy-2,4-dimethylvaleronitrile), dimethyl 2,2'-azobis(isobutyrate), 4,4'-azobis(4-cyanovaleric acid), 1,1'-azobis(cyclohexane-1-carbonitrile), benzoyl peroxide, lauroyl peroxide, potassium persulfate, ammonium persulfate, and tetramethylthiuram disulfide. Although the amount of the polymerization initiator to be used varies greatly depending on the type of monomer, polymerization temperature, and the like, the amount of the polymerization initiator to be used with respect to the total amount of the vinyl monomer and the crosslinking agent ({polymerization initiator / (vinyl monomer + crosslinking agent)} × 100) is about 0.01 to 5 mol%.

As for the polymerization conditions in the production of the second monolith, a variety of conditions can be selected depending on the type of monomer and the type of initiator. For example, when 2,2'-azobis(isobutyronitrile), 2,2'-azobis(2,4-dimethylvaleronitrile), benzoyl peroxide, lauroyl peroxide, potassium persulfate, or the like is used as the initiator, heat polymerization may be performed at 30 to 100°C for 1 to 48 hours in a sealed container under an inert atmosphere. After the completion of polymerization, the contents are taken out and extracted with a solvent such as acetone for the purpose of removing the unreacted vinyl monomer and the organic solvent, thereby obtaining the second monolith.

If the production of the second monolith is carried out under conditions where the polymerization of a vinyl monomer dissolved in an organic solvent proceeds fast, organic polymer particles with an average particle diameter close to 1 µm can be precipitated and aggregated and thus three dimensionally continuous to form a framework portion. Although the conditions where the polymerization of a vinyl monomer proceeds fast differ depending on the vinyl monomer, crosslinking agent, polymerization initiator, polymerization temperature, and the like and cannot be generalized, they are an increase in the amount of the crosslinking agent, an increase in the monomer concentration, the elevation of the temperature, and the like. In light of such polymerization conditions, the polymerization conditions for aggregating the organic polymer particles with an average particle diameter of 1 to 50 µm may be appropriately determined. In order to form three dimensionally continuous pores with an average diameter of 20 to 100 µm in the framework, as mentioned above, the amount of the crosslinking agent to be used with respect to the total amount of the vinyl monomer and the crosslinking agent may be set to a particular amount. In order to adjust the total pore volume of the monolith to 1 to 5 ml/g, although it differs depending on the vinyl monomer, crosslinking agent, polymerization initiator, polymerization temperature, and the like and cannot be generalized, the polymerization may generally be performed under conditions in which the amount of the organic solvent to be used with respect to the total amount of the organic solvent, monomer and crosslinking agent to be used ({organic solvent / (organic solvent + monomer + crosslinking agent)} × 100) is 30 to 80% by weight, and suitably 40 to 70% by weight.

Examples of the method for producing the second monolithic ion exchanger include a method in which, in the method for producing the second monolith described above, a monomer with ion exchange groups, such as a monomer formed by introducing ion exchange groups, such as carboxylic acid groups or sulfonic acid groups into a vinyl monomer without ion exchange groups, is used and polymerized to form a monolithic ion exchanger in one step, and a method in which a vinyl monomer without ion exchange groups is used and polymerized to form the second monolith and ion exchange groups are then introduced thereinto.

The method for introducing ion exchange groups into the second monolith is the same as the method for introducing ion exchange groups into the first monolith.

### <Description of Third Monolith and Third Monolithic Ion Exchanger>

In the platinum group metal ion-supported catalyst of the present invention, the third monolithic ion exchanger serving as a support for platinum group metal ions or platinum group metal complex ions is a continuous macropore structural material in which bubble-like macropores overlap each other and these overlapping areas become apertures with an average diameter of 30 to 300 µm in a dry state, and is a monolithic ion exchanger having a total pore volume in a dry state of 0.5 to 10 ml/g, in a SEM image of the cut section of the continuous macropore structural material (dried material), having an area of the framework part appearing in the cross section of 25 to 50% in the image region, having ion exchange groups, and having an ion exchange capacity per weight in a dry state of 1 to 9 mg equivalent/g, wherein the ion exchange groups are uniformly distributed in the organic porous ion exchanger. In addition, the third monolith is a monolith before introducing the ion exchange groups, and is a continuous macropore structural material in which bubble-like macropores overlap each other and these overlapping areas become apertures with an average diameter of 30 to 300 µm in a dry state, and is an organic porous material having a total pore volume in a dry state of 0.5 to 10 ml/g, and in a SEM image of the cut section of the continuous macropore structural material (dried material), having an area of the framework part appearing in the cross section of 25 to 50% in the image region.

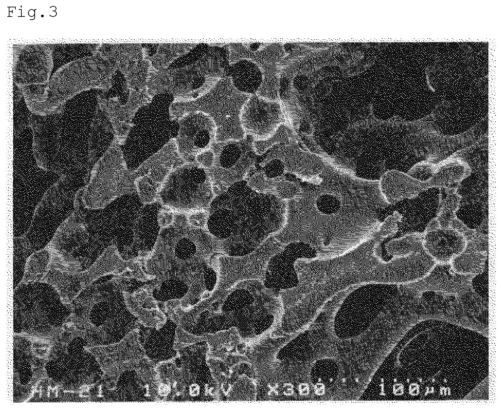

The third monolithic ion exchanger is a continuous macropore structural material in which bubble-like macropores overlap each other and these overlapping areas become apertures (mesopores) with an average diameter of 30 to 300 µm, preferably 30 to 200 µm, and particularly preferably 40 to 100 µm, in a dry state. Figure 3 shows a SEM photograph of an exemplary embodiment of the third monolithic ion exchanger. The third monolithic ion exchanger shown in Figure 3 has a large number of bubble-like macropores, and is a continuous macropore structural material in which the bubble-like macropores overlap each other and these overlapping areas become common apertures (mesopores), the majority of which has an open pore structure. When the average diameter of the apertures in a dry state is less than 30 µm, it is not preferable because the pressure loss upon passing the liquid is large. When the average diameter of the apertures in a dry state is too large, it is not preferable because the contact of the reaction liquid with the monolithic ion exchanger and the platinum group metal particles supported thereon is insufficient, resulting in a reduction in the catalytic activity.

Note that the average diameter of the apertures of the third monolith in a dry state, the average diameter of the apertures of the third monolithic ion exchanger in a dry state, and the average diameter of the apertures of a third monolithic intermediate (3) in a dry state, which is obtained by a step I of the production of the third monolith, which will be mentioned later, are measured by the mercury injection method and refer to the maximum value of the pore distribution curve obtained by the mercury injection method.

In the third monolithic ion exchanger, in a SEM image of the cut section of the continuous macropore structural material (dried material), the area of the framework part appearing in the cross section is 25 to 50%, and preferably 25 to 45% in the image region. When the area of the framework part appearing in the cross section is less than 25% in the image region, it is not preferable because a thin framework is formed and there is a decrease in mechanical strength leading to a large deformation of the monolithic ion exchanger, especially when the liquid is passed through at a high flow rate. Furthermore, the contact efficiency of the reaction liquid with the monolithic ion exchanger and the platinum group metal particles supported thereon is reduced, thereby reducing the catalytic activity, which is not preferable. When it is greater than 50%, it is not preferable because the framework becomes too thick and the pressure loss upon passing the liquid is increased.

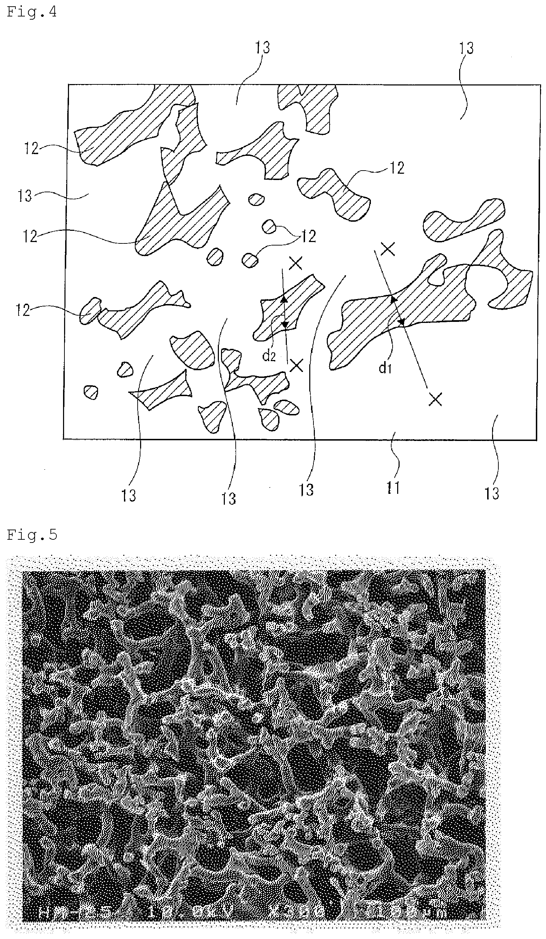

The conditions for obtaining SEM images may be conditions where the framework part appearing in the cross section of the cut section appears clearly, and are, for example, a magnification of 100 to 600 and a photograph region of about 150 mm × 100 mm. It is preferable to perform SEM observation, by eliminating the subjectivity and using three or more, and preferably five or more images differing in the cut site photographed an arbitrary site in an arbitrary cut section of the third monolithic ion exchanger or differing in the photographing site. The third monolithic ion exchanger to be cut is applied to an electron microscope and is therefore in a dry state. The framework part in the cut section in the SEM image will be described with reference to Figures 3 and 4. In Figure 4, the framework part appearing as the cross section of the SEM photograph of Figure 3 was transferred. In Figures 3 and 4, the objects having a generally indefinite shape and appearing as the cross section correspond to the "framework part appearing in the cross section (reference number 12)" of the present invention, and the round pores shown in Figure 3 correspond to the apertures (mesopores), and the objects with a relatively large curvature or curve correspond to the macropores (reference number 13 in Figure 4). The area of the framework part appearing in the cross section of Figure 4 is 28% in a rectangular image region 11. In this way, the framework part can be clearly judged.

There is no particular limitation on the method for measuring the area of the framework part appearing in the cross section of the cut section in a SEM image, and examples thereof include a calculation method by automatic calculation with a computer or manual calculation after identifying the framework part by known computer processing. Examples of the manual calculation include a method in which objects with an indefinite shape are replaced with a set of quadrangles, triangles, circles or trapezoids, which are then stacked, and the area is determined.

The total pore volume per weight of the third monolithic ion exchanger in a dry state is 0.5 to 10 ml/g, and preferably 0.8 to 8 ml/g. When the total pore volume is less than 0.5 ml/g, it is not preferable because the pressure loss upon passing the liquid is large, and furthermore, it is not preferable because the amount of fluid permeate per unit cross sectional area is small, which reduces the throughput. On the other hand, when the total pore volume is greater than 10 ml/g, it is not preferable because the mechanical strength is decreased and the monolithic ion exchanger is largely deformed, especially when the liquid is passed through at a high flow rate. Furthermore, the contact efficiency of the reaction liquid with the monolithic ion exchanger and the platinum group metal ions supported thereon is reduced, thereby also reducing the catalytic effect, which is not preferable.

In the third monolithic ion exchanger, the material constituting the framework is an organic polymer material having a crosslinked structure. Although the crosslinking density of that polymer material is not particularly limited, it is preferable to include 0.3 to 10 mol%, suitably 0.3 to 5 mol% of crosslinked structural units with respect to the entire constituent units that constitute the polymer material. When the crosslinked structural units are less than 0.3 mol%, it is not preferable because the mechanical strength is insufficient. On the other hand, when they are greater than 10 mol%, it is not preferable because the introduction of ion exchange groups may be difficult.

There is no particular limitation on the type of the polymer material constituting the framework of the third monolithic ion exchanger, and examples thereof include a crosslinked polymer, including, for example, an aromatic vinyl polymer such as polystyrene, poly(α-methylstyrene), polyvinyl toluene, polyvinylbenzyl chloride, polyvinyl biphenyl, and polyvinyl naphthalene; a polyolefin such as polyethylene and polypropylene; a poly(halogenated polyolefin) such as polyvinyl chloride and polytetrafluoroethylene; a nitrile-based polymer such as polyacrylonitrile; and a (meth)acrylic polymer such as polymethyl methacrylate, polyglycidyl methacrylate, and polyethyl acrylate. The polymers described above may be polymers obtained by copolymerizing a single vinyl monomer and a crosslinking agent, polymers obtained by polymerizing a plurality of vinyl monomers and a crosslinking agent, or a blend of two or more polymers. Among these organic polymer materials, crosslinked polymers of aromatic vinyl polymers are preferable because of the ease of forming a continuous macropore structure, the ease of introducing ion exchange groups when the ion exchange groups are introduced, the high mechanical strength, and the high stability against acids or alkalis, and in particular, styrene-divinylbenzene copolymers and vinylbenzyl chloride-divinylbenzene copolymers are preferable materials.

The ion exchange groups introduced into the third monolithic ion exchanger are the same as the ion exchange groups introduced into the first monolithic ion exchanger.

In the third monolithic ion exchanger, the introduced ion exchange groups are uniformly distributed not only on the surface of the porous material, but also inside the framework of the porous material.

The third monolithic ion exchanger has an ion exchange capacity per weight in a dry state of 1 to 9 mg equivalent/g. The third monolithic ion exchanger can have a dramatically large ion exchange capacity per volume while the pressure loss is kept low, because the aperture diameter can be larger and the framework of the continuous macropore structural material can be thick

(the wall part of the framework can be thick). When the ion exchange capacity per weight in a dry state is in the range described above, the ambient environment of a catalytic active point, such as pH inside the catalyst, can be changed, thereby increasing the catalytic activity. The ion exchange capacity per weight of the third monolithic ion exchanger in a dry state is 1 to 9 mg equivalent/g, preferably 1 to 8 mg equivalent/g, and particularly preferably 1 to 7 mg equivalent/g.

### <Method for Producing Third Monolith and Third Monolithic Ion Exchanger>

The third monolith is obtained by carrying out the following steps: stirring a mixture of an oil soluble monomer without ion exchange groups, a surfactant, and water, thereby preparing a water in oil type emulsion, and then polymerizing the water in oil type emulsion to obtain a monolithic organic porous intermediate (hereinafter, also referred to as a monolithic intermediate (3)) having a continuous macropore structure with a total pore volume of 5 to 16 ml/g (a step I); preparing a mixture formed of a vinyl monomer, a crosslinking agent having at least two or more vinyl groups in one molecule, an organic solvent that dissolves the vinyl monomer and the crosslinking agent, but does not dissolve a polymer produced by polymerization of the vinyl monomer, and a polymerization initiator (a step II); polymerizing the mixture obtained in the step II while leaving it to stand still and in the presence of the monolithic intermediate (3) obtained in the step I, thereby obtaining a third monolith having a framework thicker than the framework of the monolithic intermediate (3) (a step III).

In the method for producing the third monolith, the step I may be carried out in accordance with the method described in Japanese Patent Laid-Open No. 2002-306976.

In the production of the monolithic intermediate (3) in the step I according to the method for producing the third monolith, examples of the oil soluble monomer without ion exchange groups include, for example, a monomer that does not contain ion exchange groups such as carboxylic acid groups, sulfonic acid groups, and quaternary ammonium groups, and that has low solubility in water and is lipophilic. Among these monomers, examples of the suitable one include styrene, α-methylstyrene, vinyl toluene, vinylbenzyl chloride, divinylbenzene, ethylene, propylene, isobutene, butadiene, and ethylene glycol dimethacrylate. These monomers may be used alone as one kind, or may be used in combination of two or more kinds. However, it is preferable to select a crosslinkable monomer such as divinylbenzene or ethylene glycol dimethacrylate as at least one component of the oil soluble monomer and set the content thereof to 0.3 to 10 mol%, or suitably 0.3 to 5 mol%, of the entire oil soluble monomers because ion exchange groups can be introduced in a quantitative amount when the ion exchange groups are introduced.

The surfactant used in the step I according to the method for producing the third monolith is not particularly limited as long as it is capable of forming a water in oil type (W/O) emulsion when mixed with an oil soluble monomer without ion exchange groups and water. Examples of the surfactant that can be used include a non-ionic surfactant such as sorbitan monooleate, sorbitan monolaurate, sorbitan monopalmitate, sorbitan monostearate, sorbitan trioleate, polyoxyethylene nonylphenyl ether, polyoxyethylene stearyl ether, and polyoxyethylene sorbitan monooleate; an anionic surfactant such as potassium oleate, sodium dodecylbenzene sulfonate, sodium dioctyl sulfosuccinate; a cationic surfactant such as distearyl dimethyl ammonium chloride; and an amphoteric surfactant such as lauryl dimethyl betaine. These surfactants may be used alone as one kind or may be used in combination of two or more kinds. Note that a water in oil type emulsion refers to an emulsion in which the oil phase becomes a continuous phase and water droplets are dispersed therein. As for the amount of the surfactant to be added, it is difficult to say in general because it varies significantly depending on the type of oil soluble monomer and the size of the target emulsion particles (macropores), but it can be selected in the range of about 2 to 70% with respect to the total amount of the oil soluble monomer and the surfactant.

In addition, in the step I according to the method for producing the third monolith, a polymerization initiator may be used, if required, upon forming the water in oil type emulsion. As the polymerization initiator, a compound that generates radicals by heat or light irradiation is suitably used. The polymerization initiator may be water soluble or oil soluble, and examples thereof include, for example, azobisisobutyronitrile, azobisdimethylvaleronitrile, azobiscyclohexanenitrile, azobiscyclohexanecarbonitrile, benzoyl peroxide, potassium persulfate, ammonium persulfate, hydrogen peroxide-ferrous chloride, sodium persulfate-sodium hydrogen sulfite, and tetramethylthiuram disulfide.

In the step I according to the method for producing the third monolith, there is no limitation on the mixing method upon mixing an oil soluble monomer without ion exchange groups, a surfactant, water, and a polymerization initiator to form a water in oil type emulsion. For example, a method in which all components are mixed at once, or a method in which oil soluble components, including an oil soluble monomer, a surfactant, and an oil soluble polymerization initiator, and water soluble components, including water and a water soluble polymerization initiator, are separately dissolved to be uniform, and then these components are mixed together can be used. There is no particular limitation on the mixing apparatus for forming an emulsion, either. For example, an ordinary mixer, homogenizer, or high pressure homogenizer can be used, and an appropriate apparatus may be selected to obtain the target emulsion particle diameter. Also, there is no particular limitation on the mixing conditions, and the stirring speed and stirring time at which the target emulsion particle diameter can be obtained can be arbitrarily set.

The monolithic intermediate (3) obtained in the step I according to the method for producing the third monolith has a continuous macropore structure. When this is allowed to coexist in the polymerization system, a porous structure with a thick framework is formed using the structure of the monolithic intermediate (3) as a mold. In addition, the monolithic intermediate (3) is an organic polymer material having a crosslinked structure. Although the crosslinking density of that polymer material is not particularly limited, it is preferable to include 0.3 to 10 mol%, preferably 0.3 to 5 mol% of crosslinked structural units with respect to the entire constituent units that constitute the polymer material. When the crosslinked structural units are less than 0.3 mol%, it is not preferable because the mechanical strength is insufficient. In particular, when the total pore volume is as large as 10 to 16 ml/g, it is preferable to contain 2 mol% or more of crosslinked structural units in order to maintain the continuous macropore structure. On the other hand, when they are greater than 10 mol%, it is not preferable because the introduction of ion exchange groups may be difficult.

In the step I according to the method for producing the third monolith, there is no particular limitation on the type of polymer material of the monolithic intermediate (3), and examples thereof include those that are the same as the polymer material of the first monolith mentioned above. This can form a similar polymer in the framework of the monolithic intermediate (3) and thicken the framework, thereby obtaining the third monolith with a uniform framework structure.

The total pore volume per weight of the monolithic intermediate (3) in a dry state, obtained in the step I according to the method for producing the third monolith, is 5 to 16 ml/g, and suitably 6 to 16 ml/g. When the total pore volume is too small, it is not preferable because the total pore volume of the monolith obtained after the polymerization of the vinyl monomer is too small and the pressure loss upon fluid permeation is large. On the other hand, when the total pore volume is too large, it is not preferable because the structure of the monolith obtained after the polymerization of the vinyl monomer deviates from the continuous macropore structure. To make the total pore volume of the monolithic intermediate (3) within the numerical range described above, the ratio of monomer to water should be generally 1:5 to 1:20.

In addition, for the monolithic intermediate (3) obtained in the step I according to the method for producing the third monolith, the average diameter of apertures (mesopores), which are the overlapping portions of macropores with each other, in a dry state is 20 to 200 µm. When the average diameter of apertures in a dry state is less than 20 µm, it is not preferable because the aperture diameter of the monolith obtained after the polymerization of the vinyl monomer is small and the pressure loss upon passing the liquid is large. On the other hand, when it is greater than 200 µm, it is not preferable because the aperture diameter of the monolith obtained after the polymerization of the vinyl monomer is too large and the contact between the reaction liquid and the monolithic anion exchanger is insufficient, resulting in a reduction in the catalytic activity. It is suitable for the monolithic intermediate (3) to have a uniform structure in which the sizes of the macropores and the diameters of the apertures are uniform, but it is not limited to this, and may be dotted with nonuniform macropores that are larger than the size of the uniform macropores in the uniform structure.

The step II according to the method for producing the third monolith is a step of preparing a mixture formed of a vinyl monomer, a crosslinking agent having at least two or more vinyl groups in one molecule, an organic solvent that dissolves the vinyl monomer and the crosslinking agent, but does not dissolve a polymer produced by polymerization of the vinyl monomer, and a polymerization initiator. Note that there is no order for the step I and the step II, and the step II may be performed after the step I or the step I may be performed after the step II.

As for the vinyl monomer used in the step II according to the method for producing the third monolith, there is no particular limitation as long as it contains a polymerizable vinyl group in the molecule and is lipophilic vinyl monomer with high solubility in an organic solvent. However, it is preferable to select a vinyl monomer that produces a polymer material of the same type as or similar to the monolithic intermediate (3) coexisting in the polymerization system described above. Specific examples of such a vinyl monomer include an aromatic vinyl monomer such as styrene, α-methylstyrene, vinyl toluene, vinylbenzyl chloride, vinyl biphenyl, and vinyl naphthalene; an α-olefin such as ethylene, propylene, 1-butene, and isobutene; a diene-based monomer such as butadiene, isoprene, and chloroprene; a halogenated olefin such as vinyl chloride, vinyl bromide, vinylidene chloride, and tetrafluoroethylene; a nitrile-based monomer such as acrylonitrile and methacrylonitrile; a vinyl ester such as vinyl acetate and vinyl propionate; and a (meth)acrylic monomer such as methyl acrylate, ethyl acrylate, butyl acrylate, 2-ethylhexyl acrylate, methyl methacrylate, ethyl methacrylate, propyl methacrylate, butyl methacrylate, 2-ethylhexyl methacrylate, cyclohexyl methacrylate, benzyl methacrylate, and glycidyl methacrylate. These monomers may be used alone as one kind, or may be used in combination of two or more kinds. A vinyl monomer that is suitably used is an aromatic vinyl monomer such as styrene or vinylbenzyl chloride.

The amount to be added of the vinyl monomer used in the step II according to the method for producing the third monolith is 3 to 50 times, preferably 4 to 40 times by weight, with respect to the monolithic intermediate (3) coexisting at the time of polymerization. When the amount of the vinyl monomer to be added is less than 3 times that of the monolithic intermediate, it is not preferable because the framework of the formed monolith (the thickness of the wall part of the monolith framework) cannot be made thicker and the ion exchange capacity per volume after introduction is small when the ion exchange groups are introduced. On the other hand, when the amount of the vinyl monomer to be added is greater than 50 times, it is not preferable because the aperture diameter is small and the pressure loss upon passing the liquid is large.

As the crosslinking agent used in the step II according to the method for producing the third monolith, those containing at least two polymerizable vinyl groups in the molecule and having a high solubility in an organic solvent are suitably used. Specific examples of the crosslinking agent include divinylbenzene, divinyl naphthalene, divinyl biphenyl, ethylene glycol dimethacrylate, trimethylolpropane triacrylate, and butanediol diacrylate. These crosslinking agents may be used alone as one kind, or may be used in combination of two or more kinds. A preferable crosslinking agent is an aromatic polyvinyl compound such as divinylbenzene, divinyl naphthalene, and divinyl biphenyl because of its high mechanical strength and stability against hydrolysis. The amount of the crosslinking agent to be used is 0.3 to 10 mol%, and particularly preferably 0.3 to 5 mol%, of the total amount of the vinyl monomer and crosslinking agent. When the amount of the crosslinking agent to be used is less than 0.3 mol%, it is not preferable because the mechanical strength of the monolith is insufficient. On the other hand, when it is greater than 10 mol%, it is not preferable because the amount of the ion exchange groups to be introduced may be decreased. Note that it is preferable to use the crosslinking agent described above in an amount to be used such that the crosslinking density of the vinyl monomer and the crosslinking agent is approximately equal to that of the monolithic intermediate (3) coexisting upon the polymerization of the vinyl monomer/crosslinking agent. When both are used in amounts that are too far apart, a deviation in the crosslinking density distribution occurs in the produced monolith, and cracks are likely to be generated upon the introduction reaction of the ion exchange groups.

The organic solvent used in the step II according to the method for producing the third monolith is an organic solvent that dissolves the vinyl monomer and the crosslinking agent, but does not dissolve a polymer produced by polymerization of the vinyl monomer. In other words, it is a poor solvent for a polymer produced by polymerization of the vinyl monomer. Since the organic solvent greatly varies depending on the type of the vinyl monomer, it is difficult to specifically recite general examples, but for example, when the vinyl monomer is styrene, examples of the organic solvent include an alcohol such as methanol, ethanol, propanol, butanol, hexanol, cyclohexanol, octanol, 2-ethylhexanol, decanol, dodecanol, ethylene glycol, propylene glycol, tetramethylene glycol, and glycerin; a chain (poly)ether such as diethyl ether, ethylene glycol dimethyl ether, cellosolve, methyl cellosolve, butyl cellosolve, polyethylene glycol, polypropylene glycol, and polytetramethylene glycol; a chain saturated hydrocarbon such as hexane, heptane, octane, isooctane, decane, and dodecane; an ester such as ethyl acetate, isopropyl acetate, cellosolve acetate, and ethyl propionate. Also, even a good solvent for polystyrene, such as dioxane, THF, or toluene, can be used as the organic solvent when it is used together with the poor solvents described above and the amount thereof to be used is small. It is preferable to use these organic solvents in an amount to be used such that the concentration of the above vinyl monomer is 30 to 80% by weight. When the amount of the organic solvent to be used departs from the range described above and the concentration of the vinyl monomer is less than 30% by weight, it is not preferable because the polymerization rate is reduced or the monolithic structure after polymerization departs from the range of the third monolith. On the other hand, when the concentration of the vinyl monomer is greater than 80% by weight, it is not preferable because the polymerization may run out of control.

As the polymerization initiator used in the step II according to the method for producing the third monolith, a compound that generates radicals by heat or light irradiation is suitably used. It is preferable that the polymerization initiator should be oil soluble. Specific examples of the polymerization initiator include 2,2'-azobis(isobutyronitrile), 2,2'-azobis(2,4-dimethylvaleronitrile), 2,2'-azobis(2-methylbutyronitrile), 2,2'-azobis(4-methoxy-2,4-dimethylvaleronitrile), dimethyl 2,2'-azobis(isobutyrate), 4,4'-azobis(4-cyanovaleric acid), 1,1'-azobis(cyclohexane-1-carbonitrile), benzoyl peroxide, lauroyl peroxide, potassium persulfate, ammonium persulfate, and tetramethylthiuram disulfide. Although the amount of the polymerization initiator to be used varies greatly depending on the type of monomer, polymerization temperature, and the like, it can be used in a range of about 0.01 to 5% with respect to the total amount of the vinyl monomer and the crosslinking agent.

The step III according to the method for producing the third monolith is a step of polymerizing the mixture obtained in the step II while leaving it to stand still and in the presence of the monolithic intermediate (3) obtained in the step I, thereby obtaining a third monolith having a framework thicker than the framework of the monolithic intermediate (3). The monolithic intermediate (3) used in the step III plays an extremely important role in creating the third monolith, and when the monolithic intermediate (3) with a continuous macropore structure is present in the above polymerization system, the third monolith is obtained.

In the method for producing the third monolith, there is no particular limitation on the inner volume of the reaction vessel as long as it is large enough to allow the monolithic intermediate (3) to exist in the reaction vessel, and when the monolithic intermediate (3) is placed in the reaction vessel, a gap may be created around the monolith in a plane view or the monolithic intermediate (3) may be placed in the reaction vessel with no gap, either of which is fine. Among the above, a case is efficient in which a monolith with a thick framework after polymerization is placed in the reaction vessel with no gap without receiving pressure from the inner wall of the vessel, which does not cause distortion of the monolith and does not waste reaction raw materials and the like. Note that, even when the inner volume of the reaction vessel is large and there are gaps around the monolith after polymerization, the vinyl monomer and the crosslinking agent are adsorbed and distributed to the monolithic intermediate (3), and therefore, no particle aggregated structure is produced in the part of gaps in the reaction vessel.

In the step III according to the method for producing the third monolith, the monolithic intermediate (3) is placed in the reaction vessel in a state of being impregnated with the mixture (solution). As for the compounding ratio between the mixture obtained in the step II and the monolithic intermediate (3), it is suitable that they should be compounded such that the amount of the vinyl monomer to be added is 3 to 50 times, preferably 4 to 40 times by weight with respect to the monolithic intermediate (3), as mentioned above. By doing so, a third monolith with a thick framework while having a moderate aperture diameter can be obtained. In the reaction vessel, the vinyl monomer and crosslinking agent in the mixture are adsorbed and distributed to the framework of the monolithic intermediate that is left to stand still, and polymerization proceeds in the framework of the monolithic intermediate (3).

As for the polymerization conditions in the step III according to the method for producing the third monolith, a variety of conditions are selected depending on the type of monomer and the type of initiator. For example, when 2,2'-azobis(isobutyronitrile), 2,2'-azobis(2,4-dimethylvaleronitrile), benzoyl peroxide, lauroyl peroxide, potassium persulfate, or the like is used as the initiator, heat polymerization may be performed at 30 to 100°C for 1 to 48 hours in a sealed container under an inert atmosphere. By the heat polymerization, the vinyl monomer and crosslinking agent that have been adsorbed and distributed to the framework of the monolithic intermediate (3) are polymerized in the framework, making the framework thicker. After the completion of polymerization, the contents are taken out and extracted with a solvent such as acetone for the purpose of removing the unreacted vinyl monomer and the organic solvent, thereby obtaining the third monolith.

The third monolithic ion exchanger is obtained by performing a step IV to introduce ion exchange groups into the third monolith, which is an organic porous material with a thick framework obtained in the step III.

The method for introducing ion exchange groups into the third monolith is the same as the method for introducing ion exchange groups into the first monolith.

The third monolith and the third monolithic ion exchanger have high mechanical strength due to their thick framework even though the aperture diameter is significantly large.

### <Description of Fourth Monolith and Fourth Monolithic Ion Exchanger>

In the platinum group metal ion-supported catalyst of the present invention, the fourth weakly basic monolithic anion exchanger serving as a support for platinum group metal ions or platinum group metal complex ions is a co-continuous structural material formed of a three dimensionally continuous framework comprising an aromatic vinyl polymer containing 0.1 to 5.0 mol% of crosslinked structural units among the entire constituent units with an average thickness of 1 to 60 µm in a dry state, and three dimensionally continuous pores in the framework with an average diameter of 10 to 200 µm in a dry state, and is a monolithic ion exchanger having a total pore volume in a dry state of 0.5 to 10 ml/g, having ion exchange groups, and having an ion exchange capacity per weight in a dry state of 1 to 9 mg equivalent/g, wherein the ion exchange groups are uniformly distributed in the organic porous ion exchanger. In addition, the fourth monolith is a monolith before introducing the ion exchange groups, and is a co-continuous structural material formed of a three dimensionally continuous framework comprising an aromatic vinyl polymer containing 0.1 to 5.0 mol% of crosslinked structural units among the entire constituent units, with an average thickness of 1 to 60 µm in a dry state, and three dimensionally continuous pores in the framework with an average diameter of 10 to 200 µm in a dry state; and is an organic porous material with a total pore volume of 0.5 to 10 mL/g in a dry state.

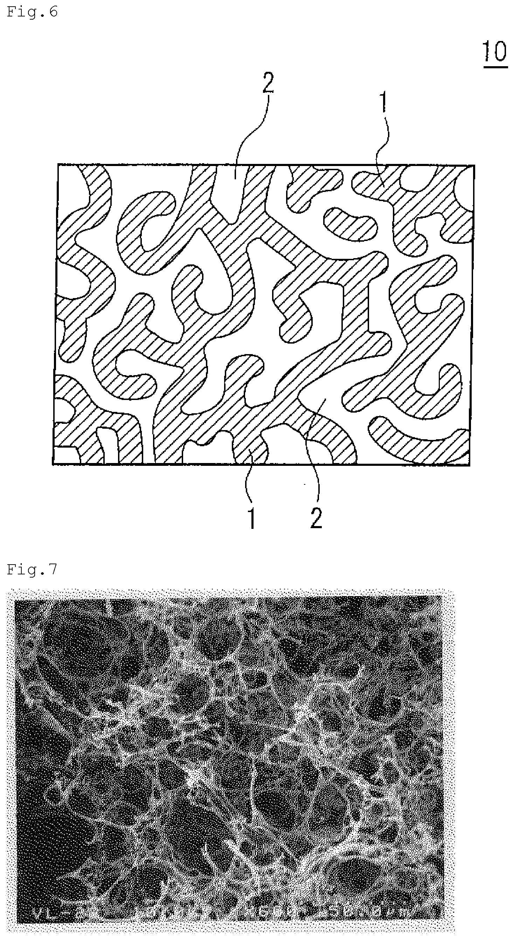

The fourth monolithic ion exchanger is a co-continuous structural material formed of a three dimensionally continuous framework with an average thickness of 1 to 60 µm, preferably 3 to 58 µm, in a dry state, and three dimensionally continuous pores in the framework with an average diameter of 10 to 200 µm, preferably 15 to 180 µm, and particularly preferably 20 to 150 µm, in a dry state. Figure 5 shows a SEM photograph of an exemplary embodiment of the fourth monolithic ion exchanger, and Figure 6 illustrates a schematic diagram of the co-continuous structure of the fourth monolithic ion exchanger. The co-continuous structure is a structure 10 in which a continuous framework phase 1 and a continuous pore phase 2 are intertwined and are both three dimensionally continuous, as illustrated in the schematic diagram of Figure 6. These continuous pores 2 have a higher degree of continuity of pores and have less deviation in their size than conventional continuous bubble monoliths and particle aggregated monoliths. In addition, the mechanical strength is high due to the thick framework.

When the average diameter of the three dimensionally continuous pores in a dry state is less than 10 µm, it is not preferable because the pressure loss upon passing the liquid is large. When it is greater than 200 µm, it is not preferable because the contact between the reaction liquid and the monolithic ion exchanger is insufficient, which results in insufficient catalytic activity. Also, when the average thickness of the framework is less than 1 µm in a dry state, it is not preferable because the monolithic ion exchanger is largely deformed when the liquid is passed through at a high speed. Furthermore, the contact efficiency between the reaction liquid and the monolithic ion exchanger is reduced, thereby reducing the catalytic effect, which is not preferable. On the other hand, when the thickness of the framework is greater than 60 µm, it is not preferable because the framework becomes too thick and the pressure loss upon passing the liquid is increased.

The average diameter of the apertures of the fourth monolith in a dry state, the average diameter of the apertures of the fourth monolithic ion exchanger in a dry state, and the average diameter of the apertures of a fourth monolithic intermediate (4) in a dry state, which is obtained in a step I of the production of the fourth monolith, which will be mentioned later, are measured by the mercury injection method and refer to the maximum value of the pore distribution curve obtained by the mercury injection method. Also, the average thickness of the framework of the fourth monolithic ion exchanger in a dry state is determined by SEM observation of the fourth monolithic ion exchanger in a dry state. Specifically, SEM observations of the fourth monolithic ion exchanger in a dry state are performed at least three times, and the thickness of the framework in the obtained images is measured, and the average value thereof is defined as the average thickness. Note that the framework is rod-shaped and has a circular cross sectional shape, but it may also include one with a different diameter cross section, such as an oval cross sectional shape. In this case, the thickness is the average of the short and long diameters.

In addition, the total pore volume per weight of the fourth monolithic ion exchanger in a dry state is 0.5 to 10 ml/g. When the total pore volume is less than 0.5 ml/g, it is not preferable because the pressure loss upon passing the liquid is large, and furthermore, it is not preferable because the amount of permeate per unit cross sectional area is small, which reduces the throughput. On the other hand, when the total pore volume is greater than 10 ml/g, it is not preferable because the mechanical strength is decreased and the monolithic ion exchanger is largely deformed, especially when the liquid is passed through at a high flow rate. Furthermore, the contact efficiency between the reaction liquid and the monolithic ion exchanger is reduced, thereby also reducing the catalytic efficiency, which is not preferable. When the size and total pore volume of the three dimensionally continuous pores are within the ranges described above, the contact with the reaction liquid is extremely uniform and the contact area is large. Besides, it is possible to pass the liquid with low pressure loss.

In the fourth monolithic ion exchanger, the material constituting the framework is an aromatic vinyl polymer including 0.1 to 5 mol%, preferably 0.5 to 3.0 mol% of crosslinked structural units among the entire constituent units, and is hydrophobic. When the crosslinked structural units are less than 0.1 mol%, it is not preferable because the mechanical strength is insufficient. On the other hand, when they are greater than 5 mol%, the structure of the porous material easily deviates from the co-continuous structure. There is no particular limitation on the type of the aromatic vinyl polymer, and examples thereof include, for example, polystyrene, poly(α-methylstyrene), polyvinyl toluene, polyvinylbenzyl chloride, polyvinyl biphenyl, and polyvinyl naphthalene. The polymers described above may be polymers obtained by copolymerizing a single vinyl monomer and a crosslinking agent, polymers obtained by polymerizing a plurality of vinyl monomers and a crosslinking agent, or a blend of two or more polymers. Among these organic polymer materials, styrene-divinylbenzene copolymers and vinylbenzyl chloride-divinylbenzene copolymers are preferable because of the ease of forming a co-continuous structure, the ease of introducing ion exchange groups, the high mechanical strength, and the high stability against acids or alkalis.

The ion exchange groups introduced into the fourth monolithic ion exchanger are the same as the ion exchange groups introduced into the first monolithic ion exchanger.

In the fourth monolithic ion exchanger, the introduced ion exchange groups are uniformly distributed not only on the surface of the porous material, but also inside the framework of the porous material.

The fourth monolithic ion exchanger has an ion exchange capacity per weight in a dry state of 1 to 9 mg equivalent/g. The fourth monolithic ion exchanger has a high degree of continuity and uniformity of three dimensionally continuous pores, which allows for few increases in the pressure loss even if the total pore volume is decreased. Hence, the ion exchange capacity per volume can be dramatically large while the pressure loss is kept low. When the ion exchange capacity per weight is in the range described above, the ambient environment of a catalytic active point, such as pH inside the catalyst, can be changed, thereby increasing the catalytic activity. The ion exchange capacity per weight of the fourth monolithic ion exchanger in a dry state is 1 to 9 mg equivalent/g, preferably 1 to 8 mg equivalent/g, and particularly preferably 1 to 7 mg equivalent/g.

### <Method for Producing Fourth Monolith and Fourth Monolithic Ion Exchanger>

The fourth monolith is obtained by carrying out the following steps: stirring a mixture of an oil soluble monomer without ion exchange groups, a surfactant, and water, thereby preparing a water in oil type emulsion, and then polymerizing the water in oil type emulsion to obtain a monolithic organic porous intermediate (hereinafter, also referred to as a monolithic intermediate (4)) having a continuous macropore structure with a total pore volume of greater than 16 mL/g and not more than 30 mL/g (a step I); preparing a mixture formed of an aromatic vinyl monomer, a crosslinking agent at 0.3 to 5 mol% among the entire oil soluble monomers having at least two or more vinyl groups in one molecule, an organic solvent that dissolves the aromatic vinyl monomer and the crosslinking agent, but does not dissolve a polymer produced by polymerization of the aromatic vinyl monomer, and a polymerization initiator (a step II); polymerizing the mixture obtained in the step II while leaving it to stand still and in the presence of the monolithic intermediate (4) obtained in the step I, thereby obtaining a fourth monolith, which is an organic porous material with a co-continuous structure (a step III) .

In the step I according to the method for producing the fourth monolith, the step I of obtaining the monolithic intermediate (4) may be carried out in accordance with the method described in Japanese Patent Laid-Open No. 2002-306976.

That is, in the step I according to the method for producing the fourth monolith, examples of the oil soluble monomer without ion exchange groups include, for example, a monomer that does not contain ion exchange groups such as carboxylic acid groups, sulfonic acid groups, tertiary amino groups, and quaternary ammonium groups, and that has low solubility in water and is lipophilic. Specific examples of such a monomer include an aromatic vinyl monomer such as styrene, α-methylstyrene, vinyl toluene, vinylbenzyl chloride, vinyl biphenyl, and vinyl naphthalene; an α-olefin such as ethylene, propylene, 1-butene, and isobutene; a diene-based monomer such as butadiene, isoprene, and chloroprene; a halogenated olefin such as vinyl chloride, vinyl bromide, vinylidene chloride, and tetrafluoroethylene; a nitrile-based monomer such as acrylonitrile and methacrylonitrile; a vinyl ester such as vinyl acetate and vinyl propionate; and a (meth)acrylic monomer such as methyl acrylate, ethyl acrylate, butyl acrylate, 2-ethylhexyl acrylate, methyl methacrylate, ethyl methacrylate, propyl methacrylate, butyl methacrylate, 2-ethylhexyl methacrylate, cyclohexyl methacrylate, benzyl methacrylate, and glycidyl methacrylate. Among these monomers, the aromatic vinyl monomer is suitable, and examples thereof include styrene, α-methylstyrene, vinyl toluene, vinylbenzyl chloride, and divinylbenzene. These monomers may be used alone as one kind, or may be used in combination of two or more kinds. However, it is preferable to select a crosslinkable monomer such as divinylbenzene or ethylene glycol dimethacrylate as at least one component of the oil soluble monomer and set the content thereof to 0.3 to 5 mol%, or suitably 0.3 to 3 mol%, of the entire oil soluble monomers because it is advantageous for the formation of a co-continuous structure.

The surfactant used in the step I according to the method for producing the fourth monolith is the same as the surfactant used in the step I according to the method for producing the third monolith, so that the description thereof is omitted.

In addition, in the step I according to the method for producing the fourth monolith, a polymerization initiator may be used, if required, upon forming the water in oil type emulsion. As the polymerization initiator, a compound that generates radicals by heat or light irradiation is suitably used. The polymerization initiator may be water soluble or oil soluble, and examples thereof include, for example, 2,2'-azobis(isobutyronitrile), 2,2'-azobis(2,4-dimethylvaleronitrile), 2,2'-azobis(2-methylbutyronitrile), 2,2'-azobis(4-methoxy-2,4-dimethylvaleronitrile), dimethyl 2,2'-azobis(isobutyrate), 4,4'-azobis(4-cyanovaleric acid), 1,1'-azobis(cyclohexane-1-carbonitrile), benzoyl peroxide, lauroyl peroxide, potassium persulfate, ammonium persulfate, tetramethylthiuram disulfide, hydrogen peroxide-ferrous chloride, and sodium persulfate-sodium hydrogen sulfite.

In the step I according to the method for producing the fourth monolith, the mixing method upon mixing an oil soluble monomer without ion exchange groups, a surfactant, water, and a polymerization initiator to form a water in oil type emulsion is the same as the mixing method in the step I according to the method for producing the third monolith, so that the description thereof is omitted.

The monolithic intermediate (4) obtained in the step I according to the method for producing the fourth monolith is an organic polymer material having a crosslinked structure, and is suitably an aromatic vinyl polymer. Although the crosslinking density of that polymer material is not particularly limited, it is preferable to include 0.1 to 5 mol%, preferably 0.3 to 3 mol% of crosslinked structural units with respect to the entire constituent units that constitute the polymer material. When the crosslinked structural units are less than 0.3 mol%, it is not preferable because the mechanical strength is insufficient. On the other hand, when they are greater than 5 mol%, it is not preferable because the structure of the monolith easily deviates from the co-continuous structure. In particular, when the total pore volume is 16 to 20 ml/g, it is preferable that the crosslinked structural units should be less than 3 mol% in order to form a co-continuous structure.

In the step I according to the method for producing the fourth monolith, the type of polymer material of the monolithic intermediate (4) is the same as the type of polymer material of the monolithic intermediate (4) according to the method for producing the third monolith, so that the description thereof is omitted.

The total pore volume per weight of the monolithic intermediate (4) in a dry state, obtained in the step I according to the method for producing the fourth monolith, is greater than 16 mL/g and not more than 30 mL/g, and suitably greater than 16 mL/g and not more than 25 mL/g. That is, although this monolithic intermediate (4) basically has a continuous macropore structure, its apertures (mesopores), which are the overlapping portions of macropores with each other, are significantly large, and therefore the framework constituting the monolithic structure has a structure that is very close to a two dimensional wall surface to a one dimensional rod-like framework. Figure 7 shows a SEM photograph of an exemplary embodiment of the monolithic intermediate (4), which has a near rod-like framework. When this is allowed to coexist in the polymerization system, a porous material with a co-continuous structure is formed using the structure of the monolithic intermediate (4) as a mold. When the total pore volume is too small, it is not preferable because the structure of the monolith obtained after the polymerization of the vinyl monomer changes from a co-continuous structure to a continuous macropore structure. On the other hand, when the total pore volume is too large, it is not preferable because the mechanical strength of the monolith obtained after the polymerization of the vinyl monomer is reduced, or when ion exchange groups are introduced, the ion exchange capacity per volume is reduced. To make the total pore volume of the monolithic intermediate (4) within the range described above, the ratio of monomer to water should be generally 1:20 to 1:40.

In addition, for the monolithic intermediate (4) obtained in the step I according to the method for producing the fourth monolith, the average diameter of apertures (mesopores), which are the overlapping portions of macropores with each other, in a dry state is 5 to 100 µm. When the average diameter of apertures in a dry state is less than 5 µm, it is not preferable because the aperture diameter of the monolith obtained after the polymerization of the vinyl monomer is small and the pressure loss upon fluid permeation is large. On the other hand, when it is greater than 100 µm, it is not preferable because the aperture diameter of the monolith obtained after the polymerization of the vinyl monomer is too large and the contact between the reaction liquid and the weakly basic monolithic anion exchanger is insufficient, resulting in a reduction in the catalytic activity. It is suitable for the monolithic intermediate (4) to have a uniform structure in which the sizes of the macropores and the diameters of the apertures are uniform, but it is not limited to this, and may be dotted with nonuniform macropores that are larger than the size of the uniform macropores in the uniform structure.

The step II according to the method for producing the fourth monolith is a step of preparing a mixture formed of an aromatic vinyl monomer, a crosslinking agent at 0.3 to 5 mol% among the entire oil soluble monomers having at least two or more vinyl groups in one molecule, an organic solvent that dissolves the aromatic vinyl monomer and the crosslinking agent, but does not dissolve a polymer produced by polymerization of the aromatic vinyl monomer, and a polymerization initiator. Note that there is no order for the step I and the step II, and the step II may be performed after the step I or the step I may be performed after the step II.

As for the aromatic vinyl monomer used in the step II according to the method for producing the fourth monolith, there is no particular limitation as long as it contains a polymerizable vinyl group in the molecule and is lipophilic aromatic vinyl monomer with high solubility in an organic solvent. However, it is preferable to select a vinyl monomer that produces a polymer material of the same type as or similar to the monolithic intermediate (4) coexisting in the polymerization system described above. Specific examples of such a vinyl monomer include styrene, α-methylstyrene, vinyl toluene, vinylbenzyl chloride, vinyl biphenyl, and vinyl naphthalene. These monomers may be used alone as one kind, or may be used in combination of two or more kinds. An aromatic vinyl monomer that is suitably used is styrene, vinylbenzyl chloride, or the like.

The amount to be added of the aromatic vinyl monomer used in the step II according to the method for producing the fourth monolith is 5 to 50 times, preferably 5 to 40 times by weight, with respect to the monolithic intermediate (4) coexisting at the time of polymerization. When the amount of the aromatic vinyl monomer to be added is less than 5 times that of the monolithic intermediate (4), it is not preferable because the rod-like framework cannot be made thicker and the ion exchange capacity per volume after the introduction of ion exchange groups is small when the ion exchange groups are introduced. On the other hand, when the amount of the aromatic vinyl monomer to be added is greater than 50 times, it is not preferable because the diameter of the continuous pores is small and the pressure loss upon passing the liquid is large.

As the crosslinking agent used in the step II according to the method for producing the fourth monolith, those containing at least two polymerizable vinyl groups in the molecule and having a high solubility in an organic solvent are suitably used. Specific examples of the crosslinking agent include divinylbenzene, divinyl naphthalene, divinyl biphenyl, ethylene glycol dimethacrylate, trimethylolpropane triacrylate, and butanediol diacrylate. These crosslinking agents may be used alone as one kind, or may be used in combination of two or more kinds. A preferable crosslinking agent is an aromatic polyvinyl compound such as divinylbenzene, divinyl naphthalene, and divinyl biphenyl because of its high mechanical strength and stability against hydrolysis. The amount of the crosslinking agent to be used is 0.3 to 5 mol%, in particular 0.3 to 3 mol%, of the total amount of the vinyl monomer and crosslinking agent (the entire oil soluble monomers). When the amount of the crosslinking agent to be used is less than 0.3 mol%, it is not preferable because the mechanical strength of the monolith is insufficient. On the other hand, when it is too large, it is not preferable because quantitative introduction of ion exchange groups may be difficult when the ion exchange groups are introduced. Note that it is preferable to use the crosslinking agent described above in an amount to be used such that the crosslinking density of the vinyl monomer and the crosslinking agent is approximately equal to that of the monolithic intermediate (4) coexisting upon the polymerization of the vinyl monomer/crosslinking agent. When both are used in amounts that are too far apart, a deviation in the crosslinking density distribution occurs in the produced monolith, and when ion exchange groups are introduced, cracks are likely to be generated upon the introduction reaction of the ion exchange groups.

The organic solvent used in the step II according to the method for producing the fourth monolith is an organic solvent that dissolves the aromatic vinyl monomer and the crosslinking agent, but does not dissolve a polymer produced by polymerization of the aromatic vinyl monomer. In other words, it is a poor solvent for a polymer produced by polymerization of the aromatic vinyl monomer. Since the organic solvent greatly varies depending on the type of the aromatic vinyl monomer, it is difficult to specifically recite general examples, but for example, when the aromatic vinyl monomer is styrene, examples of the organic solvent include an alcohol such as methanol, ethanol, propanol, butanol, hexanol, cyclohexanol, octanol, 2-ethylhexanol, decanol, dodecanol, propylene glycol, and tetramethylene glycol; a chain (poly)ether such as diethyl ether, butyl cellosolve, polyethylene glycol, polypropylene glycol, and polytetramethylene glycol; a chain saturated hydrocarbon such as hexane, heptane, octane, isooctane, decane, and dodecane; an ester such as ethyl acetate, isopropyl acetate, cellosolve acetate, and ethyl propionate. Also, even a good solvent for polystyrene, such as dioxane, THF, or toluene, can be used as the organic solvent when it is used together with the poor solvents described above and the amount thereof to be used is small. It is preferable to use these organic solvents in an amount to be used such that the concentration of the above aromatic vinyl monomer is 30 to 80% by weight. When the amount of the organic solvent to be used departs from the range described above and the concentration of the aromatic vinyl monomer is less than 30% by weight, it is not preferable because the polymerization rate is reduced or the monolithic structure after polymerization departs from the range of the fourth monolith. On the other hand, when the concentration of the aromatic vinyl monomer is greater than 80% by weight, it is not preferable because the polymerization may run out of control.

The polymerization initiator used in the step II according to the method for producing the fourth monolith is the same as the polymerization initiator used in the step II according to the method for producing the third monolith, so that the description thereof is omitted.

The step III according to the method for producing the fourth monolith is a step of polymerizing the mixture obtained in the step II while leaving it to stand still and in the presence of the monolithic intermediate (4) obtained in the step I, thereby changing the continuous macropore structure of the monolithic intermediate (4) to a co-continuous structure, obtaining a fourth monolith, which is a monolith with a co-continuous structure. The monolithic intermediate (4) used in the step III plays an extremely important role in creating the monolith with a novel structure of the present invention. As disclosed in Japanese Translation of PCT International Application Publication No. 1995-501140 and the like, static polymerization of the vinyl monomer and the crosslinking agent in a certain organic solvent in the absence of the monolithic intermediate (4) yields a particle aggregated type monolithic organic porous material. In contrast, when a monolithic intermediate (4) with a particular continuous macropore structure is present in the above polymerization system, as in the case of producing the fourth monolith, the structure of the monolith after polymerization is changed dramatically, the particle aggregated structure disappears, and the fourth monolith with the co-continuous structure mentioned above is obtained. Although the reason for this has not been elucidated in detail, it is thought that, when the monolithic intermediate (4) is not present, the particle aggregated structure is formed by the precipitation and sedimentation of the crosslinked polymer produced by the polymerization in a particle form, whereas when a porous material (intermediate) with a large total pore volume is present in the polymerization system, the vinyl monomer and the crosslinking agent are adsorbed or distributed from the liquid phase to the framework part of the porous material, polymerization proceeds in the porous material, and the framework constituting the monolithic structure is changed from a two dimensional wall surface to a one dimensional rod-like framework, thereby forming a fourth monolith having a co-continuous structure.

In the method for producing the fourth monolith, the inner volume of the reaction vessel is the same as in the description of the inner volume of the reaction vessel according to the method for producing the third monolith, so that the description thereof is omitted.

In the step III according to the method for producing the fourth monolith, the monolithic intermediate (4) is placed in the reaction vessel in a state of being impregnated with the mixture (solution). As for the compounding ratio between the mixture obtained in the step II and the monolithic intermediate (4), it is suitable that they should be compounded such that the amount of the aromatic vinyl monomer to be added is 5 to 50 times, preferably 5 to 40 times by weight with respect to the monolithic intermediate (4), as mentioned above. By doing so, a fourth monolith with a co-continuous structure in which moderately sized pores are three dimensionally continuous and the thick framework is also three dimensionally continuous can be obtained. In the reaction vessel, the aromatic vinyl monomer and crosslinking agent in the mixture are adsorbed and distributed to the framework of the monolithic intermediate (4) that is left to stand still, and polymerization proceeds in the framework of the monolithic intermediate (4).

The polymerization conditions in the step III according to the method for producing the fourth monolith are the same as in the description of the polymerization conditions in the step III according to the method for producing the third monolith, so that the description thereof is omitted. By carrying out the step III, the fourth monolith is obtained.

The fourth monolithic ion exchanger is obtained by performing a step IV to introduce ion exchange groups into the fourth monolith obtained in the step III.

The method for introducing ion exchange groups into the fourth monolith is the same as the method for introducing ion exchange groups into the first monolith.

The fourth monolith and the fourth monolithic ion exchanger have high mechanical strength due to their thick framework even though the size of the three dimensionally continuous pores is significantly large. In addition, since the fourth monolithic ion exchanger has a thick framework, the ion exchange capacity per volume in a dry state can be increased, and furthermore, the reaction liquid can be kept flowing at a low pressure and high flow rate for a long period of time.

### <Description of Fifth Monolith and Fifth Monolithic Ion Exchanger>

In the platinum group metal ion-supported catalyst of the present invention, the fifth monolithic ion exchanger serving as a support for platinum group metal ions or platinum group metal complex ions is a composite structural material of an organic porous material formed of a continuous framework phase and a continuous pore phase, and a number of particle materials with a diameter of 4 to 40 µm in a dry state adhering to the framework surface of the organic porous material or a number of protruding materials with a size of 4 to 40 µm in a dry state formed on the framework surface of the organic porous material, and is a monolithic ion exchanger having an average diameter of pores in a dry state of 10 to 200 µm and a total pore volume in a dry state of 0.5 to 10 ml/g, having ion exchange groups, and having an ion exchange capacity per weight in a dry state of 1 to 9 mg equivalent/g, wherein the ion exchange groups are uniformly distributed in the organic porous ion exchanger. In addition, the fifth monolith is a monolith before introducing the ion exchange groups, and is a composite structural material of an organic porous material formed of a continuous framework phase and a continuous pore phase, and a number of particle materials with a diameter of 4 to 40 µm in a dry state adhering to the framework surface of the organic porous material or a number of protruding materials with a size of 4 to 40 µm in a dry state formed on the framework surface of the organic porous material, and is an organic porous material having an average diameter of pores in a dry state of 10 to 200 µm and a total pore volume in a dry state of 0.5 to 10 ml/g.

The fifth monolithic ion exchanger is a composite structural material of an organic porous material formed of a continuous framework phase and a continuous pore phase, and a number of particle materials with a diameter of 4 to 40 µm in a dry state adhering to the framework surface of the organic porous material or a number of protruding materials with a size of 4 to 40 µm in a dry state formed on the framework surface of the organic porous material. Note that, in the present specification, the "particle materials" and the "protruding materials" may also be collectively referred to as "particle materials, etc."

The continuous framework phase and the continuous pore phase of the fifth monolithic ion exchanger are observed from SEM images. The basic structure of the fifth monolithic ion exchanger includes a continuous macropore structure and a co-continuous structure. The framework phase of the fifth monolithic ion exchanger appears as a columnar continuous material, a continuous material of a concave wall surface, or a composite material thereof and has a shape evidently different from the particle shape or the protruding shape.

A preferable structure of the fifth monolithic ion exchanger includes a continuous macropore structural material in which bubble-like macropores overlap each other and these overlapping areas become apertures with an average diameter of 10 to 120 µm in a dry state (hereinafter, also referred to as a "fifth-1 monolithic ion exchanger"), and a co-continuous structural material formed of a three dimensionally continuous framework with an average thickness of 0.8 to 40 µm in a dry state, and three dimensionally continuous pores in the framework with an average diameter of 8 to 80 µm in a dry state (hereinafter, also referred to as a "fifth-2 monolithic ion exchanger"). In addition, the fifth monolith is preferably a monolith before introducing the ion exchange groups in the fifth-1 monolithic ion exchanger (hereinafter, also referred to as a "fifth-1 monolith"), and a monolith before introducing the ion exchange groups in the fifth-2 monolithic ion exchanger (hereinafter, also referred to as a "fifth-2 monolith").

In the case of the fifth-1 monolithic ion exchanger, the fifth-1 monolithic ion exchanger is a continuous macropore structural material in which bubble-like macropores overlap each other and these overlapping areas become apertures (mesopores) with an average diameter of 20 to 150 µm, preferably 30 to 150 µm, and particularly preferably 35 to 150 µm, in a dry state, and the flow channels are in the bubbles formed by the macropores and the apertures (mesopores). It is suitable for the continuous macropore structure to have a uniform structure in which the sizes of the macropores and the diameters of the apertures are uniform, but it is not limited to this, and may be dotted with nonuniform macropores that are larger than the size of the uniform macropores in the uniform structure. When the average diameter of the apertures of the fifth-1 monolithic ion exchanger in a dry state is less than 20 µm, it is not preferable because the pressure loss upon passing the liquid is large. When the average diameter of the apertures in a dry state is greater than 150 µm, it is not preferable because the contact of the reaction liquid with the monolithic ion exchanger and the platinum group metal ions supported thereon is insufficient, resulting in a reduction in the catalytic activity.

Note that the average diameter of the apertures of the fifth monolith in a dry state, the average diameter of the apertures of the fifth monolithic ion exchanger in a dry state, and the average diameter of the apertures of a monolithic intermediate (5) in a dry state, which is obtained in a step I of the production of the fifth monolith, which will be mentioned later, refer to the maximum value of the pore distribution curve obtained by the mercury injection method.

In the case of the fifth-2 monolithic ion exchanger, the fifth-2 monolithic ion exchanger has a co-continuous structure having a three dimensionally continuous framework with an average thickness of 1 to 50 µm, and preferably 5 to 50 µm in a dry state, and three dimensionally continuous pores in the framework with an average diameter of 10 to 100 µm, and preferably 10 to 90 µm in a dry state. When the average diameter of the three dimensionally continuous pores of the fifth-2 monolithic ion exchanger in a dry state is less than 10 µm, it is not preferable because the pressure loss upon passing the liquid is large. When it is greater than 100 µm, it is not preferable because the contact of the reaction liquid with the monolithic ion exchanger and the platinum group metal ions supported thereon is insufficient, resulting in a reduction in the catalytic activity. Also, when the average thickness of the framework of the fifth-2 monolithic ion exchanger is less than 1 µm in a dry state, it is not preferable because the mechanical strength is decreased and the monolithic ion exchanger is largely deformed, especially when the liquid is passed through at a high flow rate. On the other hand, when the average thickness of the framework of the fifth-2 monolithic ion exchanger is greater than 50 µm in a dry state, it is not preferable because the framework becomes too thick and the pressure loss upon passing the liquid is increased.

The average thickness of the framework of the fifth-2 monolithic ion exchanger in a dry state is determined by SEM observation of the fifth-2 monolithic ion exchanger in a dry state. Specifically, SEM observations of the fifth-2 monolithic ion exchanger in a dry state are performed at least three times, and the thickness of the framework in the obtained images is measured, and the average value thereof is defined as the average thickness. Note that the framework is rod-shaped and has a circular cross sectional shape, but it may also include one with a different diameter cross section, such as an oval cross sectional shape. In this case, the thickness is the average of the short and long diameters.

The average diameter of the pores of the fifth monolithic ion exchanger in a dry state is 10 to 200 µm. In the case of the fifth-1 monolithic ion exchanger, a preferable value of the pore diameter of the fifth-1 monolithic ion exchanger in a dry state is 30 to 150 µm. In the case of the fifth-2 monolithic ion exchanger, a preferable value of the pore diameter of the fifth-2 monolithic ion exchanger in a dry state is 10 to 90 µm.

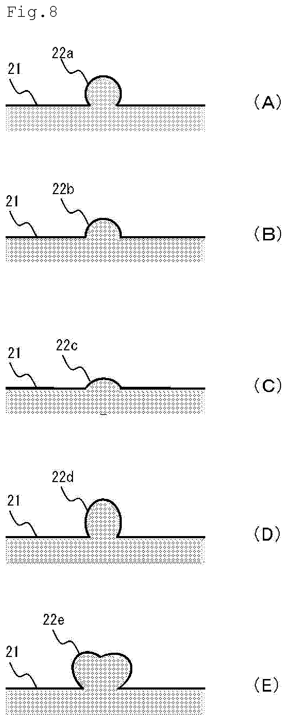

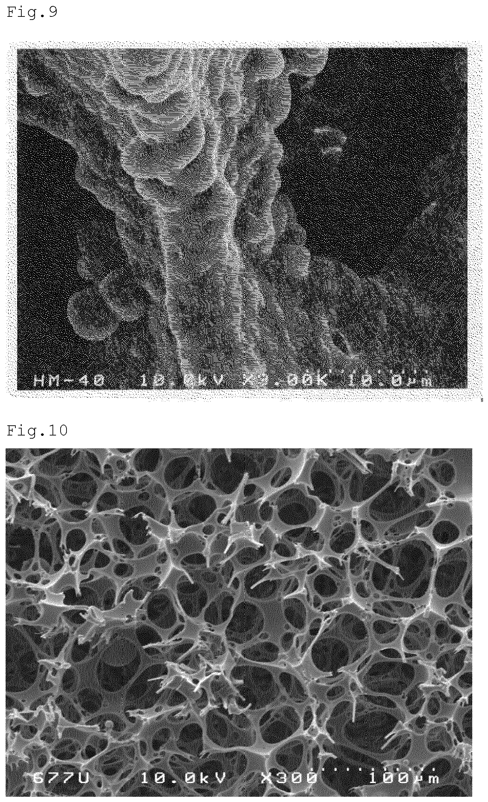

In the fifth monolithic ion exchanger, the diameter of the particle materials and the size of the protruding materials in a dry state are 4 to 40 µm, preferably 4 to 30 µm, and particularly preferably 4 to 20 µm. Note that, in the present invention, both the particle materials and the protruding materials are observed so as to protrude from the framework surface, and those observed in a particulate form are referred to as the particle materials while those in a protruding form that cannot be regarded as the particulate form are referred to as the protruding materials. Figure 8 shows a schematic cross-sectional view of the protruding material. As shown in Figures 8(A) to 8(E), those in a protruding form that projects from a framework surface 21 are protruding materials 22, and examples of the protruding materials 22 include those having a shape close to the particulate form, such as a protruding material 22a shown in (A), hemispheroids such as a protruding material 22b shown in (B), and elevations of the framework surface, such as a protruding material 22c shown in (C). Also, the protruding materials 22 additionally include those having a shape longer in a direction perpendicular to the framework surface 21 than in the planar direction of the framework surface 21, such as a protruding material 22d shown in (D), and those having a shape protruding in a plurality of directions, such as a protruding material 22e shown in (E). In addition, the size of the protruding materials is judged from SEM images when observed with SEM and refers to the length of a portion with the largest width in the SEM image of each individual protruding material. Figure 9 shows a SEM photograph of an exemplary embodiment of the fifth monolithic ion exchanger. A number of protruding materials are formed in the framework surface of an organic porous material.

In the fifth monolithic ion exchanger, the ratio of the particle materials, etc. of 4 to 40 µm in a dry state to the entire particle materials, etc. is 70% or more, and preferably 80% or more. Note that, the ratio of the particle materials, etc. of 4 to 40 µm in a dry state to the entire particle materials, etc. refers to the ratio of the number of the particle materials, etc. of 4 to 40 µm in a dry state to the number of the entire particle materials, etc. 40% or more, and preferably 50% or more of the surface of the framework phase is covered with the entire particle materials, etc. Note that, the covering ratio of the surface of the framework layer with the entire particle materials, etc. refers to an area ratio in a SEM image when the surface is observed with SEM, that is, an area ratio when the surface is planarly viewed. When the size of the particles covering the wall surface or the framework deviates from the range described above, the effect of improving the contact efficiency of the fluid with the framework surface and the inside of the framework in the monolithic ion exchanger is easily reduced. Note that the entire particle materials, etc. refer to all the particle materials and the protruding materials formed in the surface of the framework layer, also including all the particle materials and the protruding materials with a size other than that of the particle materials, etc. of 4 to 40 µm in a dry state.

The diameter or size in a dry state of the particle materials, etc. attached to the framework surface of the fifth monolithic ion exchanger is the diameter or size of particle materials, etc. obtained by the observation of SEM images of the fifth monolithic ion exchanger in a dry state. And, the diameters or sizes of all the particle materials, etc. observed in the SEM images of the fifth monolithic ion exchanger in a dry state are measured, and on the basis of the values, the diameters or sizes in a dry state of the entire particle materials, etc. in the SEM image in one field of view are calculated. This SEM observation of the fifth monolithic ion exchanger in a dry state is performed at least three times, and the diameters or sizes in a dry state of the entire particle materials, etc. in the SEM image in the total field of view are calculated to confirm whether or not the particle materials, etc. with a diameter or a size of 4 to 40 µm are observed. When they are confirmed in the total field of view, it is judged that the particle materials, etc. with a diameter or a size of 4 to 40 µm in a dry state have been formed on the framework surface of the fifth monolithic ion exchanger. In addition, according to the above description, the diameters or sizes in a dry state of the entire particle materials, etc. in the SEM image per field of view are calculated, and the ratio of the particle materials, etc. of 4 to 40 µm in a dry state to the entire particle materials, etc. per field of view is determined. When the ratio of the particle materials, etc. of 4 to 40 µm in a dry state to the entire particle materials, etc. in the total field of view is 70% or more, it is judged that the ratio of the particle materials, etc. of 4 to 40 µm in a dry state to the entire particle materials, etc. formed in the framework surface of the fifth monolithic ion exchanger is 70% or more. In addition, according to the above description, the covering ratio of the surface of the framework layer with the entire particle materials, etc. in the SEM image per field of view is determined. When the covering ratio of the surface of the framework layer with the entire particle materials, etc. in the total field of view is 40% or more, it is judged that the covering ratio of the surface of the framework layer of the fifth monolithic ion exchanger with the entire particle materials, etc. is 40% or more.

In the fifth monolithic ion exchanger, when the covering ratio of the framework phase surface with the particle materials, etc. is less than 40%, the effect of improving the contact efficiency of the reaction liquid with the inside of the framework and the framework surface in the monolithic ion exchanger is easily reduced. Examples of the method for measuring the covering ratio with the particle materials, etc. include an image analysis method with SEM images of the fifth monolithic ion exchanger.

The total pore volume per weight of the fifth monolithic ion exchanger in a dry state is 0.5 to 10 ml/g, and preferably 0.8 to 8 ml/g. When the total pore volume of the monolithic ion exchanger is less than 0.5 ml/g, it is not preferable because the pressure loss upon passing the liquid is large, and furthermore, it is not preferable because the amount of fluid permeate per unit cross sectional area is small, which reduces the throughput. On the other hand, when the total pore volume of the monolithic ion exchanger is greater than 10 ml/g, it is not preferable because the mechanical strength is decreased and the monolithic ion exchanger is largely deformed, especially when the liquid is passed through at a high flow rate. Furthermore, the contact efficiency of the reaction liquid with the monolithic ion exchanger and the platinum group metal ions supported thereon is reduced, thereby also reducing the catalytic effect, which is not preferable.

In the fifth monolithic ion exchanger, the material constituting the framework phase of the continuous pore structure is an organic polymer material having a crosslinked structure. Although the crosslinking density of that polymer material is not particularly limited, it is preferable to include 0.3 to 10 mol%, suitably 0.3 to 5 mol% of crosslinked structural units with respect to the entire constituent units that constitute the polymer material. When the crosslinked structural units are less than 0.3 mol%, it is not preferable because the mechanical strength is insufficient. On the other hand, when they are greater than 10 mol%, it is not preferable because the introduction of ion exchange groups is difficult when the ion exchange groups are introduced, which may decrease the amount to be introduced.

There is no particular limitation on the type of the polymer material used in the production of the fifth monolith, and examples thereof include a crosslinked polymer, including, for example, an aromatic vinyl polymer such as polystyrene, poly(α-methylstyrene), polyvinyl toluene, polyvinylbenzyl chloride, polyvinyl biphenyl, and polyvinyl naphthalene; a polyolefin such as polyethylene and polypropylene; a poly(halogenated polyolefin) such as polyvinyl chloride and polytetrafluoroethylene; a nitrile-based polymer such as polyacrylonitrile; and a (meth)acrylic polymer such as polymethyl methacrylate, polyglycidyl methacrylate, and polyethyl acrylate. The polymers described above may be polymers obtained by copolymerizing a single vinyl monomer and a crosslinking agent, polymers obtained by polymerizing a plurality of vinyl monomers and a crosslinking agent, or a blend of two or more polymers. Among these organic polymer materials, crosslinked polymers of aromatic vinyl polymers are preferable because of the ease of forming a continuous pore structure, the ease of introducing ion exchange groups, the high mechanical strength, and the high stability against acids and alkalis, and in particular, styrenedivinylbenzene copolymers and vinylbenzyl chloridedivinylbenzene copolymers are preferable materials.

In the fifth monolithic ion exchanger, examples of the material constituting the framework phase of the organic porous material, and the particle materials, etc. formed in the surface of the framework phase include those formed of the same material in which the same texture is continuous, and those formed of materials different from each other in which the textures that are not the same are continuous. Examples of those formed of materials different from each other in which the textures that are not the same are continuous include the case of materials differing from each other in the type of the vinyl monomer, and the case of materials differing from each other in the compounding ratio even though having the same type of the vinyl monomer or crosslinking agent.

The ion exchange groups introduced into the fifth monolithic ion exchanger are the same as the ion exchange groups introduced into the first monolithic ion exchanger.

In the fifth monolithic ion exchanger, the introduced ion exchange groups are uniformly distributed not only on the surface of the organic porous material, but also inside the framework of the organic porous material. When the ion exchange groups are uniformly distributed not only on the surface of the fifth monolithic ion exchanger, but also inside the framework, the physical properties and chemical properties of the surface and the inside can be made uniform, thus improving the resistance against swelling and shrinkage.

The fifth monolithic ion exchanger has an ion exchange capacity per weight in a dry state of 1 to 9 mg equivalent/g. When the ion exchange capacity per weight of the fifth monolithic ion exchanger is in the range described above, the ambient environment of a catalytic active point, such as pH inside the catalyst, can be changed, thereby increasing the catalytic activity. The ion exchange capacity per weight of the fifth monolithic ion exchanger in a dry state is 1 to 9 mg equivalent/g, preferably 1 to 8 mg equivalent/g, and particularly preferably 1 to 7 mg equivalent/g.

The fifth monolithic ion exchanger is 1 mm or greater in its thickness and is thus differentiated from a membrane-like porous material. When the thickness is less than 1 mm, it is not preferable because the ion exchange capacity per porous material is extremely low. The thickness of the fifth monolithic ion exchanger is preferably 3 to 1000 mm. In addition, the fifth monolithic ion exchanger has high mechanical strength because the basic structure of the framework is a continuous pores structure.

### <Method for Producing Fifth Monolith and Fifth Monolithic Ion Exchanger>

The fifth monolith is obtained by carrying out the following steps: stirring a mixture of an oil soluble monomer without ion exchange groups, a surfactant, and water, thereby preparing a water in oil type emulsion, and then polymerizing the water in oil type emulsion to obtain a monolithic organic porous intermediate (hereinafter, also referred to as a monolithic intermediate (5)) having a continuous macropore structure with a total pore volume of 5 to 30 ml/g (a step I); preparing a mixture formed of a vinyl monomer, a crosslinking agent having at least two or more vinyl groups in one molecule, an organic solvent that dissolves the vinyl monomer and the crosslinking agent, but does not dissolve a polymer produced by polymerization of the vinyl monomer, and a polymerization initiator (a step II); polymerizing the mixture obtained in the step II while leaving it to stand still and in the presence of the monolithic intermediate (5) obtained in the step I, thereby obtaining a fifth monolith which is a composite monolith with a composite structure (a step III).

The step I according to the method for producing the fifth monolith may be carried out in accordance with the method described in Japanese Patent Laid-Open No. 2002-306976.

In the production of the monolithic intermediate (5) in the step I according to the method for producing the fifth monolith, examples of the oil soluble monomer without ion exchange groups include, for example, a monomer that does not contain ion exchange groups such as carboxylic acid groups, sulfonic acid groups, tertiary amino groups, and quaternary ammonium groups, and that has low solubility in water and is lipophilic. Among these monomers, examples of the suitable one include styrene, α-methylstyrene, vinyl toluene, vinylbenzyl chloride, divinylbenzene, ethylene, propylene, isobutene, butadiene, and ethylene glycol dimethacrylate. These monomers may be used alone as one kind, or may be used in combination of two or more kinds. However, it is preferable to select a crosslinkable monomer such as divinylbenzene or ethylene glycol dimethacrylate as at least one component of the oil soluble monomer and set the content thereof to 0.3 to 10 mol%, or suitably 0.3 to 5 mol%, of the entire oil soluble monomers because ion exchange groups can be introduced in a quantitative amount when the ion exchange groups are introduced in the subsequent step.

The surfactant used in the step I according to the method for producing the fifth monolith is not particularly limited as long as it is capable of forming a water in oil type (W/O) emulsion when mixed with an oil soluble monomer without ion exchange groups and water. Examples of the surfactant that can be used include a non-ionic surfactant such as sorbitan monooleate, sorbitan monolaurate, sorbitan monopalmitate, sorbitan monostearate, sorbitan trioleate, polyoxyethylene nonylphenyl ether, polyoxyethylene stearyl ether, and polyoxyethylene sorbitan monooleate; an anionic surfactant such as potassium oleate, sodium dodecylbenzene sulfonate, sodium dioctyl sulfosuccinate; a cationic surfactant such as distearyl dimethyl ammonium chloride; and an amphoteric surfactant such as lauryl dimethyl betaine. These surfactants may be used alone as one kind or may be used in combination of two or more kinds. Note that a water in oil type emulsion refers to an emulsion in which the oil phase becomes a continuous phase and water droplets are dispersed therein. As for the amount of the surfactant to be added, it is difficult to say in general because it varies significantly depending on the type of oil soluble monomer and the size of the target emulsion particles (macropores), but it can be selected in the range of about 2 to 70% with respect to the total amount of the oil soluble monomer and the surfactant.

In addition, in the step I according to the method for producing the fifth monolith, a polymerization initiator may be used, if required, upon forming the water in oil type emulsion. As the polymerization initiator, a compound that generates radicals by heat or light irradiation is suitably used. The polymerization initiator may be water soluble or oil soluble, and examples thereof include, for example, 2,2'-azobis(isobutyronitrile), 2,2'-azobis(2,4-dimethylvaleronitrile), 2,2'-azobis(2-methylbutyronitrile), 2,2'-azobis(4-methoxy-2,4-dimethylvaleronitrile), dimethyl 2,2'-azobis(isobutyrate), 4,4'-azobis(4-cyanovaleric acid), 1,1'-azobis(cyclohexane-1-carbonitrile), benzoyl peroxide, lauroyl peroxide, potassium persulfate, ammonium persulfate, hydrogen peroxide-ferrous chloride, and sodium persulfate-sodium hydrogen sulfite.

In the step I according to the method for producing the fifth monolith, there is no limitation on the mixing method upon mixing an oil soluble monomer without ion exchange groups, a surfactant, water, and a polymerization initiator to form a water in oil type emulsion. For example, a method in which all components are mixed at once, or a method in which oil soluble components, including an oil soluble monomer, a surfactant, and an oil soluble polymerization initiator, and water soluble components, including water and a water soluble polymerization initiator, are separately dissolved to be uniform, and then these components are mixed together can be used. There is no particular limitation on the mixing apparatus for forming an emulsion, either. For example, an ordinary mixer, homogenizer, or high pressure homogenizer can be used, and an appropriate apparatus may be selected to obtain the target emulsion particle diameter. Also, there is no particular limitation on the mixing conditions, and the stirring speed and stirring time at which the target emulsion particle diameter can be obtained can be arbitrarily set.

The monolithic intermediate (5) obtained in the step I according to the method for producing the fifth monolith has a continuous macropore structure. When this is allowed to coexist in the polymerization system, particle materials, etc. are formed in the surface of the framework phase of a continuous macropore structure or particle materials, etc. are formed in the surface of the framework phase of a co-continuous structure, using the structure of the monolithic intermediate (5) as a mold. In addition, the monolithic intermediate (5) is an organic polymer material having a crosslinked structure. Although the crosslinking density of that polymer material is not particularly limited, it is preferable to include 0.3 to 10 mol%, preferably 0.3 to 5 mol% of crosslinked structural units with respect to the entire constituent units that constitute the polymer material. When the crosslinked structural units are less than 0.3 mol%, it is not preferable because the mechanical strength is insufficient. On the other hand, when they are greater than 10 mol%, it is not preferable because the porous material may lose its flexibility and the introduction of ion exchange groups may be difficult when the ion exchange groups are introduced.

In the step I according to the method for producing the fifth monolith, there is no particular limitation on the type of polymer material of the monolithic intermediate (5), and examples thereof include those that are the same as the polymer material of the fifth monolith mentioned above. This can form a similar polymer in the framework of the monolithic intermediate (5), thereby obtaining the fifth monolith which is a monolith with a composite structure.

The total pore volume per weight of the monolithic intermediate (5) in a dry state, obtained in the step I according to the method for producing the fifth monolith, is 5 to 30 ml/g, and suitably 6 to 28 ml/g. When the total pore volume of the monolithic intermediate is too small, it is not preferable because the total pore volume of the monolith obtained after the polymerization of the vinyl monomer is too small and the pressure loss upon fluid permeation is large. On the other hand, when the total pore volume of the monolithic intermediate is too large, it is not preferable because the structure of the monolith obtained after the polymerization of the vinyl monomer is easily made nonuniform and in some cases, structural collapse is caused. To make the total pore volume of the monolithic intermediate (5) within the numerical range described above, the ratio (weight) of monomer to water should be generally 1:5 to 1:35.

In the step I according to the method for producing the fifth monolith, when this ratio of monomer to water is generally 1:5 to 1:20, a monolithic intermediate (5) with a continuous macropore structure with a total pore volume of 5 to 16 ml/g is obtained and the monolith obtained through the step III is the fifth-1 monolith. Also, when the compounding ratio is generally 1:20 to 1:35, a monolithic intermediate (5) with a continuous macropore structure with a total pore volume of greater than 16 ml/g and 30 ml/g or less is obtained and the monolith obtained through the step III is the fifth-2 monolith.

In addition, for the monolithic intermediate (5) obtained in the step I according to the method for producing the fifth monolith, the average diameter of apertures (mesopores), which are the overlapping portions of macropores with each other, in a dry state is 20 to 200 µm. When the average diameter of the apertures of the monolithic intermediate in a dry state is less than 20 µm, it is not preferable because the aperture diameter of the monolith obtained after the polymerization of the vinyl monomer is small and the pressure loss upon passing the liquid is large. On the other hand, when the average diameter of the apertures of the monolithic intermediate in a dry state is greater than 200 µm, it is not preferable because the aperture diameter of the monolith obtained after the polymerization of the vinyl monomer is too large and the contact between the reaction liquid and the weakly basic monolithic anion exchanger is insufficient, resulting in a reduction in the catalytic activity. It is suitable for the monolithic intermediate (5) to have a uniform structure in which the sizes of the macropores and the diameters of the apertures are uniform, but it is not limited to this, and may be dotted with nonuniform macropores that are larger than the size of the uniform macropores in the uniform structure.

The step II according to the method for producing the third monolith is a step of preparing a mixture formed of a vinyl monomer, a second crosslinking agent having at least two or more vinyl groups in one molecule, an organic solvent that dissolves the vinyl monomer and the second crosslinking agent, but does not dissolve a polymer produced by polymerization of the vinyl monomer, and a polymerization initiator. Note that there is no order for the step I and the step II, and the step II may be performed after the step I or the step I may be performed after the step II.

As for the vinyl monomer used in the step II according to the method for producing the fifth monolith, there is no particular limitation as long as it contains a polymerizable vinyl group in the molecule and is lipophilic vinyl monomer with high solubility in an organic solvent. Specific examples of such a vinyl monomer include an aromatic vinyl monomer such as styrene, α-methylstyrene, vinyl toluene, vinylbenzyl chloride, vinyl biphenyl, and vinyl naphthalene; an α-olefin such as ethylene, propylene, 1-butene, and isobutene; a diene-based monomer such as butadiene, isoprene, and chloroprene; a halogenated olefin such as vinyl chloride, vinyl bromide, vinylidene chloride, and tetrafluoroethylene; a nitrile-based monomer such as acrylonitrile and methacrylonitrile; a vinyl ester such as vinyl acetate and vinyl propionate; and a (meth)acrylic monomer such as methyl acrylate, ethyl acrylate, butyl acrylate, 2-ethylhexyl acrylate, methyl methacrylate, ethyl methacrylate, propyl methacrylate, butyl methacrylate, 2-ethylhexyl methacrylate, cyclohexyl methacrylate, benzyl methacrylate, and glycidyl methacrylate. These monomers may be used alone as one kind, or may be used in combination of two or more kinds. A vinyl monomer that is suitably used is an aromatic vinyl monomer such as styrene or vinylbenzyl chloride.

The amount to be added of the vinyl monomer used in the step II according to the method for producing the fifth monolith is 3 to 50 times, preferably 4 to 40 times by weight, with respect to the monolithic intermediate (5) coexisting at the time of polymerization. When the amount of the vinyl monomer to be added is less than 3 times that of the porous material, it is not preferable because the particle materials, etc. cannot be formed in the framework of the formed monolith and the ion exchange capacity per volume after introduction of ion exchange groups is small when the ion exchange groups are introduced. On the other hand, when the amount of the vinyl monomer to be added is greater than 50 times, it is not preferable because the aperture diameter is small and the pressure loss upon passing the liquid is large.

As the crosslinking agent used in the step II according to the method for producing the fifth monolith, those containing at least two polymerizable vinyl groups in the molecule and having a high solubility in an organic solvent are suitably used. Specific examples of the crosslinking agent include divinylbenzene, divinyl naphthalene, divinyl biphenyl, ethylene glycol dimethacrylate, trimethylolpropane triacrylate, and butanediol diacrylate. These crosslinking agents may be used alone as one kind, or may be used in combination of two or more kinds. A preferable crosslinking agent is an aromatic polyvinyl compound such as divinylbenzene, divinyl naphthalene, and divinyl biphenyl because of its high mechanical strength and stability against hydrolysis. The amount of the crosslinking agent to be used is 0.3 to 20 mol%, and particularly preferably 0.3 to 10 mol%, of the total amount of the vinyl monomer and crosslinking agent. When the amount of the crosslinking agent to be used is less than 0.3 mol%, it is not preferable because the mechanical strength of the monolith is insufficient. On the other hand, when it is greater than 20 mol%, it is not preferable because embrittlement proceeds in the monolith, which in turn loses its flexibility, and the amount of the ion exchange groups to be introduced may be decreased when the ion exchange groups are introduced.

The organic solvent used in the step II according to the method for producing the fifth monolith is an organic solvent that dissolves the vinyl monomer and the crosslinking agent, but does not dissolve a polymer produced by polymerization of the vinyl monomer. In other words, it is a poor solvent for a polymer produced by polymerization of the vinyl monomer. Since the organic solvent greatly varies depending on the type of the vinyl monomer, it is difficult to specifically recite general examples, but for example, when the vinyl monomer is styrene, examples of the organic solvent include an alcohol such as methanol, ethanol, propanol, butanol, hexanol, cyclohexanol, octanol, 2-ethylhexanol, decanol, dodecanol, propylene glycol, and tetramethylene glycol; a chain (poly)ether such as diethyl ether, butyl cellosolve, polyethylene glycol, polypropylene glycol, and polytetramethylene glycol; a chain saturated hydrocarbon such as hexane, heptane, octane, isooctane, decane, and dodecane; an ester such as ethyl acetate, isopropyl acetate, cellosolve acetate, and ethyl propionate. Also, even a good solvent for polystyrene, such as dioxane, THF, or toluene, can be used as the organic solvent when it is used together with the poor solvents described above and the amount thereof to be used is small. It is preferable to use these organic solvents in an amount to be used such that the concentration of the above vinyl monomer is 5 to 80% by weight. When the amount of the organic solvent to be used departs from the range described above and the concentration of the vinyl monomer is less than 5% by weight, it is not preferable because the polymerization rate is reduced. On the other hand, when the concentration of the vinyl monomer is greater than 80% by weight, it is not preferable because the polymerization may run out of control.

As the polymerization initiator used in the step II according to the method for producing the fifth monolith, a compound that generates radicals by heat or light irradiation is suitably used. It is preferable that the polymerization initiator should be oil soluble. Specific examples of the polymerization initiator include 2,2'-azobis(isobutyronitrile), 2,2'-azobis(2,4-dimethylvaleronitrile), 2,2'-azobis(2-methylbutyronitrile), 2,2'-azobis(4-methoxy-2,4-dimethylvaleronitrile), dimethyl 2,2'-azobis(isobutyrate), 4,4'-azobis(4-cyanovaleric acid), 1,1'-azobis(cyclohexane-1-carbonitrile), benzoyl peroxide, lauroyl peroxide, and tetramethylthiuram disulfide. Although the amount of the polymerization initiator to be used varies greatly depending on the type of monomer, polymerization temperature, and the like, it can be used in a range of about 0.01 to 5% with respect to the total amount of the vinyl monomer and the crosslinking agent.

The step III according to the method for producing the fifth monolith is a step of polymerizing the mixture obtained in the step II while leaving it to stand still and in the presence of the monolithic intermediate (5) obtained in the step I, thereby obtaining a fifth monolith. The monolithic intermediate (5) used in the step III plays an extremely important role in creating the fifth monolith. As disclosed in Japanese Translation of PCT International Application Publication No. 1995-501140 and the like, static polymerization of the vinyl monomer and the crosslinking agent in a certain organic solvent in the absence of the monolithic intermediate (5) yields a particle aggregated type monolithic organic porous material. In contrast, when a monolithic intermediate (5) with a continuous macropore structure is present in the above polymerization system, as in the present invention, the structure of the monolith after polymerization is changed dramatically, the fifth monolith with the particular framework structure mentioned above, not a particle aggregated structure, is obtained. There is no particular limitation on the inner volume of the reaction vessel as long as it is large enough to allow the monolithic intermediate (5) to exist in the reaction vessel, and when the monolithic intermediate (5) is placed in the reaction vessel, a gap may be created around the monolith in a plane view or the monolithic intermediate (5) may be placed in the reaction vessel with no gap, either of which is fine. Among the above, a case is efficient in which the fifth monolith after polymerization is placed in the reaction vessel with no gap without receiving pressure from the inner wall of the vessel, which does not cause distortion of the fifth monolith and does not waste reaction raw materials and the like. Note that, even when the inner volume of the reaction vessel is large and there are gaps around the fifth monolith after polymerization, the vinyl monomer and the crosslinking agent are adsorbed and distributed to the monolithic intermediate (5), and therefore, no particle aggregated structure is produced in the part of gaps in the reaction vessel.

In the step III according to the method for producing the fifth monolith, the monolithic intermediate (5) is placed in the reaction vessel in a state of being impregnated with the mixture (solution). As for the compounding ratio between the mixture obtained in the step II and the monolithic intermediate (5), it is suitable that they should be compounded such that the amount of the vinyl monomer to be added is 3 to 50 times, preferably 4 to 40 times by weight with respect to the monolithic intermediate (5), as mentioned above. By doing so, a fifth monolith which is a composite monolith with a particular framework while having a moderate aperture diameter can be obtained. In the reaction vessel, the vinyl monomer and crosslinking agent in the mixture are adsorbed and distributed to the framework of the monolithic intermediate (5) that is left to stand still, and polymerization proceeds in the framework of the monolithic intermediate (5).

As for the polymerization conditions in the step III according to the method for producing the fifth monolith, a variety of conditions can be selected depending on the type of monomer and the type of initiator. For example, when 2,2'-azobis(isobutyronitrile), 2,2'-azobis(2,4-dimethylvaleronitrile), benzoyl peroxide, lauroyl peroxide, or the like is used as the initiator, heat polymerization may be performed at 20 to 100°C for 1 to 48 hours in a sealed container under an inert atmosphere. By the heat polymerization, the vinyl monomer and crosslinking agent that have been adsorbed and distributed to the framework of the monolithic intermediate (5) are polymerized in the framework, forming the particular framework structure. After the completion of polymerization, the contents are taken out and extracted with a solvent such as acetone for the purpose of removing the unreacted vinyl monomer and the organic solvent, thereby obtaining the fifth monolith which is a composite monolith with the particular framework structure.

In producing the fifth monolith mentioned above, when the step II or the step III is performed under conditions that satisfy at least one of the following conditions (1) to (5), a monolith in which particle materials, etc. have been formed in the framework surface, which is a characteristic structure of the fifth monolith, can be produced.
(1) The polymerization temperature in the step III is a temperature that is lower by at least 5°C than the 10-hour half-life temperature of a polymerization initiator.
(2) The mol% of the crosslinking agent used in the step II is two or more times the mol% of the crosslinking agent used in the step I.
(3) The vinyl monomer used in the step II is a vinyl monomer structurally different from the oil soluble monomer used in the step I.
(4) The organic solvent used in the step II is a polyether with a molecular weight of 200 or more.
(5) The concentration of the vinyl monomer used in the step II is 30% by weight or less in the mixture of the step II.

### (Description of Condition (1))

The 10-hour half-life temperature is a characteristic value of a polymerization initiator, and when the polymerization initiator to be used is determined, the 10-hour half-life temperature can be known. Also, if there is a desired 10-hour half-life temperature, a polymerization initiator appropriate therefor can be selected. In the step III, by lowering the polymerization temperature, the polymerization rate is decreased so that particle materials, etc. can be formed in the surface of the framework phase. This is presumably because a decrease in the monomer concentration inside the framework phase of the monolithic intermediate becomes gradual and the monomer distribution rate from the liquid phase part to the monolithic intermediate is decreased and therefore, extra monomers are concentrated in the neighborhood of the surface of the framework layer of the monolithic intermediate and polymerized on site.

In the step III according to the method for producing the fifth monolith, a preferable polymerization temperature is a temperature that is lower by at least 10°C than the 10-hour half-life temperature of the polymerization initiator to be used. Although there is no particular limitation on the lower limit value of the polymerization temperature, a lower temperature decreases the polymerization rate and makes the polymerization time too long to be practically accepted. Therefore, it is preferable to set the polymerization temperature in a range lower by 5 to 20°C than the 10-hour half-life temperature.

### (Description of Condition (2))

When polymerization is performed by setting the mol% of the crosslinking agent used in the step II according to the method for producing the fifth monolith to two or more times the mol% of the crosslinking agent used in the step I, a monolith with a composite structure is obtained. This is presumably because the compatibility between the monolithic intermediate and a polymer produced by impregnation and polymerization is reduced so that phase separation proceeds and therefore, the polymer produced by impregnation and polymerization is eliminated to the neighborhood of the surface of the framework phase of the monolithic intermediate and forms the asperities of particle materials, etc. in the framework phase surface. Note that the mol% of the crosslinking agent is mol% of crosslinking density and refers to the amount (mol%) of the crosslinking agent with respect to the total amount of the vinyl monomer and the crosslinking agent.

There is no particular limitation on the upper limit of the mol% of the crosslinking agent used in the step II according to the method for producing the fifth monolith. When the mol% of the crosslinking agent is drastically large, it is not preferable because problems arise in such a way that cracks are generated in a monolith after polymerization, embrittlement proceeds in the monolith, which in turn loses its flexibility, and the amount of the ion exchange groups to be introduced may be decreased when the ion exchange groups are introduced. A preferable multiple number of the mol% of the crosslinking agent is 2 to 10 times. On the other hand, when the mol% of the crosslinking agent used in the step I was set to two or more times the mol% of the crosslinking agent used in the step II, neither did the formation of particle materials, etc. in the framework phase surface occur, nor the fifth monolith was obtained.

### (Description of Condition (3))

When the vinyl monomer used in the step II according to the method for producing the fifth monolith is a vinyl monomer structurally different from the oil soluble monomer used in the step I, the fifth monolith is obtained. For example, in the case of vinyl monomers, such as styrene and vinylbenzyl chloride, differing in their structures even only slightly, a composite monolith in which particle materials, etc. have been formed in the framework phase surface is produced. In general, two types of homopolymers obtained from two types of monomers differing in their structures even only slightly are not compatible with each other. Accordingly, when the monomer structurally different from the monomer used for monolithic intermediate formation in the step I is used in the step II and polymerization is performed in the step III, the monomer used in the step II is uniformly distributed and impregnated into the monolithic intermediate. Nonetheless, as the polymerization proceeds to produce a polymer, the produced polymer is not compatible with the monolithic intermediate and therefore, phase separation proceeds. The produced polymer is presumably eliminated to the neighborhood of the surface of the framework phase of the monolithic intermediate and forms the asperities of particle materials, etc. in the surface of the framework phase.

### (Description of Condition (4))

When the organic solvent used in the step II according to the method for producing the fifth monolith is polyether with a molecular weight of 200 or more, the fifth monolith is obtained. Polyether has relatively high affinity for the monolithic intermediate, and in particular, low molecular weight cyclic polyether is a good solvent for polystyrene and low molecular weight chained polyether has considerable affinity, albeit being not a good solvent. However, a larger molecular weight of the polyether dramatically reduces the affinity for the monolithic intermediate and eventually exhibits almost no affinity for the monolithic intermediate. When such a solvent having poor affinity is used as the organic solvent, the diffusion of the monomer to the inside of the framework of the monolithic intermediate is inhibited. As a result, the monomer is polymerized only in the neighborhood of the surface of the framework of the monolithic intermediate, and therefore, particle materials, etc. are presumably formed in the framework phase surface and form asperities in the framework surface.

There is no particular limitation on the upper limit of the molecular weight of the polyether used in the step II according to the method for producing the fifth monolith as long as it is 200 or more. However, too high a molecular weight is not preferable because the viscosity of the mixture prepared in the step II is high and the impregnation into the inside of the monolithic intermediate is difficult. A preferable molecular weight of the polyether is 200 to 100000, and particularly preferably 200 to 10000. The terminal structure of the polyether may be an unmodified hydroxy group, may be etherified with an alkyl group such as a methyl group or an ethyl group, or may be esterified with acetic acid, oleic acid, lauric acid, stearic acid, or the like.

### (Description of Condition (5))

When the concentration of the vinyl monomer used in the step II according to the method for producing the fifth monolith is 30% by weight or less in the mixture of the step II, the fifth monolith is obtained. By decreasing the monomer concentration in the step II, the polymerization rate is decreased. For the same reason as in the condition (1), particle materials, etc. can be formed in the framework phase surface and can form asperities in the framework phase surface. Although there is no particular limitation on the lower limit value of the monomer concentration, a lower monomer concentration decreases the polymerization rate and makes the polymerization time too long to be practically accepted. Therefore, it is preferable to set the monomer concentration to 10 to 30% by weight.

A preferable structure of the fifth monolith thus obtained includes a continuous macropore structural material in which bubble-like macropores overlap each other and these overlapping areas become apertures with an average diameter of 10 to 120 µm in a dry state ("fifth-1 monolith"), and a co-continuous structural material formed of a three dimensionally continuous framework with an average thickness of 0.8 to 40 µm in a dry state, and three dimensionally continuous pores in the framework with an average diameter of 8 to 80 µm in a dry state ("fifth-2 monolith").

When the fifth monolith is the fifth-1 monolith, the fifth-1 monolith is a continuous macropore structural material in which bubble-like macropores overlap each other and these overlapping areas become apertures (mesopores) with an average diameter of 10 to 120µm, preferably 20 to 120 µm, and particularly preferably 25 to 120 µm, in a dry state, and the flow channels are in the bubbles formed by the macropores and the apertures (mesopores). It is suitable for the continuous macropore structure to have a uniform structure in which the sizes of the macropores and the diameters of the apertures are uniform, but it is not limited to this, and may be dotted with nonuniform macropores that are larger than the size of the uniform macropores in the uniform structure. When the average diameter of the apertures of the fifth-1 monolith in a dry state is less than 10 µm, it is not preferable because the pressure loss upon passing the liquid is large. When the average diameter of the apertures in a dry state is greater than 120 µm, it is not preferable because the contact of the reaction liquid with the monolithic ion exchanger and the platinum group metal particles supported thereon is insufficient, resulting in a reduction in the catalytic activity.

In the case of the fifth-2 monolith, the fifth-2 monolith has a co-continuous structure having a three dimensionally continuous framework with an average thickness of 0.8 to 40 µm in a dry state, and three dimensionally continuous pores in the framework with an average diameter of 8 to 80 µm in a dry state. When the average diameter of the three dimensionally continuous pores of the fifth-2 monolith in a dry state is less than 8 µm, it is not preferable because the pressure loss upon passing the liquid is large. When it is greater than 80 µm, it is not preferable because the contact of the reaction liquid with the monolith or weakly basic monolithic anion exchanger and the platinum group metal particles supported thereon is insufficient, resulting in a reduction in the catalytic activity. Also, when the average thickness of the framework of the fifth-2 monolith in a dry state is less than 0.8 µm, it is not preferable because there is not only a disadvantage such as a decrease in the ion exchange capacity per volume of the monolithic ion exchanger when the ion exchange groups are introduced, but also a decrease in mechanical strength leading to a large deformation of the monolith or monolithic ion exchanger, especially when the liquid is passed through at a high flow rate. On the other hand, when the average thickness of the framework in a dry state is greater than 80 µm, it is not preferable because the pressure loss upon passing the liquid is increased.

The fifth monolithic ion exchanger is obtained by performing a step IV to introduce ion exchange groups into the fifth monolith obtained in the step III.

The method for introducing ion exchange groups into the fifth monolith is the same as the method for introducing ion exchange groups into the first monolith.

In the platinum group metal ion-supported catalyst of the present invention, platinum group metal ions or platinum group metal complex ions are supported on a nonparticulate organic porous monolithic ion exchanger (monolithic ion exchanger). That is, in the platinum group metal ion-supported catalyst of the present invention, a platinum group metal in an ionic state is supported on a monolithic ion exchanger. Examples of the platinum group metal ion-supported catalyst of the present invention include a catalyst in which platinum group metal ions or platinum group metal complex ions are bonded via ionic bonding or coordination bonding to weakly basic anion exchange groups, for example, tertiary amino groups, such as dimethylamino groups or diethylamino groups, in the monolithic ion exchanger so that the platinum group metal ions are supported on the weakly basic monolithic anion exchanger groups. Examples of the platinum group metal ion-supported catalyst of the present invention include a catalyst in which platinum group metal ions are bonded via ionic bonding to strongly acidic cation exchange groups, for example, sulfonic acid groups, or weakly acidic cation exchange groups, for example, carboxyl groups, in the monolithic ion exchanger so that the platinum group metal ions are supported on the strongly acidic cation exchange groups or weakly acidic cation exchange groups. Examples thereof also include a catalyst in which platinum group metal complex ions are bonded via ionic bonding to strongly basic anion exchange groups, for example, quaternary ammonium groups, in the monolithic ion exchanger so that the platinum group metal ions are supported on the strongly basic monolithic anion exchange groups.

In the platinum group metal ion-supported catalyst of the present invention, the platinum group metal is ruthenium, rhodium, palladium, osmium, iridium, or platinum. These platinum group metals may be used alone as one kind or may be used in combination of two or more kinds of metals. Then, the platinum group metal ions are ions of the platinum group metals.

The platinum group metal ions or platinum group metal complex ions being supported on the platinum group metal catalyst support of the present invention can be confirmed by performing transmission electron microscope (TEM) observation.

The amount of the platinum group metal ions or platinum group metal complex ions supported in the platinum group metal ion-supported catalyst of the present invention ((mass based on the platinum group metal atom / mass of the platinum group metal ion-supported catalyst in a dry state) × 100) is 0.01 to 10.0% by mass and preferably 0.10 to 5.0% by mass based on the atom. When the amount of the platinum group metal ions or platinum group metal complex ions supported is in the range described above, the catalyst functions as such a catalyst and becomes inexpensive from the viewpoint of the raw material. Note that, in the present invention, the platinum group metal atoms in the platinum group metal ion-supported catalyst are quantified by using an ICP emission spectroscopic apparatus.

There is no particular limitation on the method for producing the platinum group metal ion-supported catalyst of the present invention, and the platinum group metal ion-supported catalyst is obtained by supporting platinum group metal ions or platinum group metal complex ions onto the monolithic ion exchanger by a known method. Examples of the method include a method in which the monolithic ion exchanger in a dry state is immersed in an organic solution of a platinum group metal compound and platinum group metal ions are adsorbed onto the monolithic ion exchanger by ion exchange, and a method in which the monolithic ion exchanger is immersed in an aqueous solution of a platinum group metal complex compound such as a tetraamminepalladium complex and platinum group metal ions are adsorbed and supported on the monolithic ion exchanger by ion exchange.

The introduction of palladium ions to the monolithic ion exchanger may be performed in a batch manner or in a circulation manner without particular limitations.

The platinum group metal compound used in the method for producing the platinum group metal ion-supported catalyst may be any organic salt and inorganic salt, and examples thereof include a halide, sulfate, nitrate, phosphate, organic acid salt, and an inorganic complex salt. Specific examples of the platinum group metal compound include palladium chloride, palladium nitrate, palladium sulfate, palladium acetate, tetraamminepalladium chloride, tetraamminepalladium nitrate, platinum chloride, tetraammineplatinum chloride, tetraammineplatinum nitrate, chlorotriammineplatinum chloride, hexaammineplatinum chloride, hexaammineplatinum sulfate, chloropentaammineplatinum chloride, cis-tetrachlorodiammineplatinum chloride, trans-tetrachlorodiammineplatinum chloride, rhodium chloride, rhodium acetate, hexaamminerhodium chloride, hexaamminerhodium bromide, hexaamminerhodium sulfate, pentaammineaquarhodium chloride, pentaammineaquarhodium nitrate, cis-dichlorotetraamminerhodium chloride, trans-dichlorotetraamminerhodium chloride, ruthenium chloride, hexaammineruthenium chloride, hexaammineruthenium bromide, hexaammineruthenium iodide, chloropentaammineruthenium chloride, cis-dichlorotetraammineruthenium chloride, trans-dichlorotetraamminerhodium chloride, iridium(III) chloride, iridium(IV) chloride, hexaammineiridium chloride, hexaammineiridium nitrate, chloropentaammineiridium chloride, chloropentaammineiridium bromide, hexaammineosmium chloride, hexaammineosmium bromide, and hexaammineosmium iodide.

The platinum group metal compound is usually dissolved in a solvent and used upon supporting of platinum group metal ions or platinum group metal complex ion. As for the solvent, water; an alcohol such as methanol, ethanol, propanol, butanol, or benzyl alcohol; ketone such as acetone or methyl ethyl ketone; nitrile such as acetonitrile; amide such as dimethylformamide, dimethylacetamide, or N-methylpyrrolidone or a mixture thereof is used. In order to enhance the solubility of the platinum group metal compound in the solvent, an acid such as hydrochloric acid, sulfuric acid, or nitric acid, or a base such as sodium hydroxide or tetramethylammonium hydroxide may be added.

The method for forming a carbon-carbon bond of the present invention is a method for forming a carbon-carbon bond to form a carbon-carbon bond by performing (1) reaction of an aromatic halide with an organoboron compound, (2) reaction of an aromatic halide with a compound having a terminal alkynyl group, or (3) reaction of an aromatic halide with a compound having an alkenyl group,
wherein the carbon-carbon bond-forming reaction is performed by introducing a raw material liquid (i) containing the aromatic halide and the organoboron compound, a raw material liquid (ii) containing the aromatic halide and the compound having a terminal alkynyl group, or a raw material liquid (iii) containing the aromatic halide and the compound having an alkenyl group, through an introduction path of a filling container filled with a platinum group metal ion-supported catalyst, into the filling container, passing the raw material liquid through the platinum group metal ion-supported catalyst, and discharging the reaction liquid from a discharge path of the filling container, and
the platinum group metal ion-supported catalyst is the platinum group metal ion-supported catalyst of the present invention described above.

The method for forming a carbon-carbon bond of the present invention is a method for forming a carbon-carbon bond to perform carbon-carbon bond-forming reaction by passing a raw material liquid of the reaction, that is, a raw material liquid (i), a raw material liquid (ii) or a raw material liquid (iii), through the platinum group metal ion-supported catalyst of the present invention, and thereby contacting the raw material liquid with the platinum group metal ion-supported catalyst of the present invention. Note that, in the method for forming a carbon-carbon bond of the present invention, when the carbon-carbon bond-forming reaction is (1) reaction of an aromatic halide with an organoboron compound, the raw material liquid is a raw material liquid (i) containing the aromatic halide and the organoboron compound. In the method for forming a carbon-carbon bond of the present invention, when the carbon-carbon bond-forming reaction is (2) reaction of an aromatic halide with a compound having a terminal alkynyl group, the raw material liquid is a raw material liquid (ii) containing the aromatic halide and the compound having a terminal alkynyl group. In the method for forming a carbon-carbon bond of the present invention, when the carbon-carbon bond-forming reaction is (3) reaction of an aromatic halide with a compound having an alkenyl group, the raw material liquid is a raw material liquid (iii) containing the aromatic halide and the compound having an alkenyl group. Note that, hereinbelow, an aromatic halide and an organoboron compound, an aromatic halide and a compound having a terminal alkynyl group, or an aromatic halide and an alkenyl group may be referred to as a raw material.

In the method for forming a carbon-carbon bond of the present invention, the platinum group metal ion-supported catalyst of the present invention is filled into a filling container. And, the raw material liquid of the reaction passes through the continuous pores of the porous structure of the platinum group metal ion-supported catalyst of the present invention, by introducing the raw material liquid, through an introduction path of the filling container filled with the platinum group metal ion-supported catalyst of the present invention, into the filling container, passing the raw material liquid through the platinum group metal ion-supported catalyst, and discharging the reaction liquid from a discharge path of the filling container. In the meantime, the raw material liquid of the reaction comes into contact with the platinum group metal in the continuous pores of the porous structure of the platinum group metal ion-supported catalyst of the present invention, thereby causing carbon-carbon bond-forming reaction with the platinum group metal. That is, the method for forming a carbon-carbon bond of the present invention has a reaction scheme in a fixed-bed continuous circulation manner.

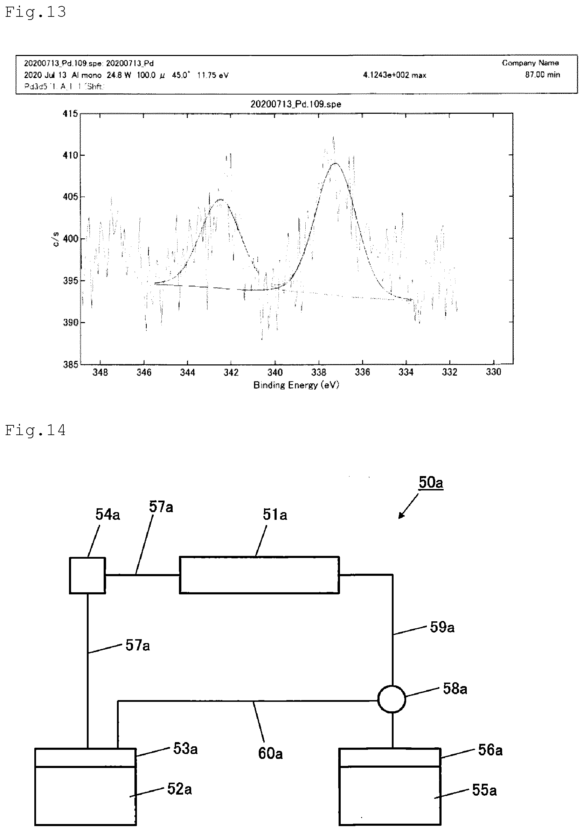

A flow diagram of an exemplary first embodiment of a reaction apparatus for carrying out the method for forming a carbon-carbon bond of the present invention is shown in Figure 14. In Figure 14, a carbon-carbon bond-forming reaction apparatus 50a comprises: a cylindrical filling container 51a filled with the platinum group metal ion-supported catalyst of the present invention; a raw material container 53a in which a raw material liquid 52a of the reaction is to be placed; a raw material liquid supply pump 54a for supplying the raw material liquid 52a to the filling container 51a; a reaction liquid receiver 56a for placing therein a reaction liquid 55a discharged from the filling container 51a; a raw material liquid introduction pipe 57a which connects the raw material container 53a with the filling container 51a and serves as an introduction path for the raw material liquid; a reaction liquid discharge pipe 59a which connects the filling container 51a with the reaction liquid receiver 56a and is provided with a switching valve 58a in the middle thereof; and a circulation pipe 60a which is branched from the reaction liquid discharge pipe 59a via the switching valve 58a and linked to the raw material liquid introduction pipe 57a. If necessary, a heating member or a heating apparatus for heating the inside of the filling container 51a is attached to the filling container 51a.

In the carbon-carbon bond-forming reaction apparatus 50a, the raw material liquid 52a is continuously supplied from the raw material container 53a toward the filling container 51a by the raw material liquid supply pump 54a. The raw material liquid supplied into the filling container 51a passes through the platinum group metal ion-supported catalyst according to the present invention, specifically, the continuous pores of the organic porous material, filled into the filling container 51a. By this, the raw material liquid continuously comes into contact with the platinum group metal ion-supported catalyst according to the present invention so that the raw materials in the raw material liquid react to perform carbon-carbon bond formation. Then, the reaction liquid 55a after carbon-carbon bond formation is sent, through the reaction liquid discharge pipe 59a, to the reaction liquid receiver 56a. Alternatively, the reaction liquid 55a after carbon-carbon bond formation is returned, through the circulation pipe 60a branched from the reaction liquid discharge pipe 59a, to the raw material container 53a. Note that the supply of the reaction liquid 55a to the reaction liquid receiver 56a and the return thereof to the raw material container 53a are switched by the switching valve 58a. In addition, by switching the supply of the reaction liquid 55a to the reaction liquid receiver 56a and the return thereof to the raw material container 53a, the reaction may be performed by passing the liquid through the layer of the platinum group metal ion-supported catalyst according to the present invention only once, or may be performed by passing the liquid through the catalyst layer two or more times.

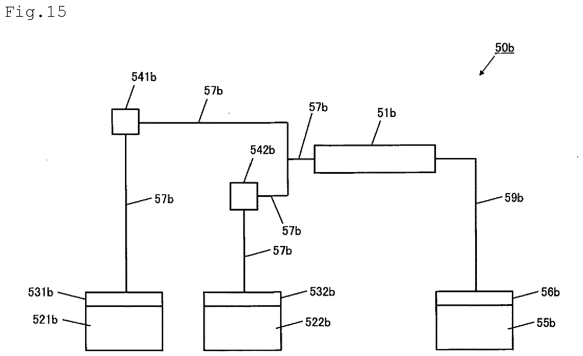

A flow diagram of an exemplary second embodiment of a reaction apparatus for carrying out the method for forming a carbon-carbon bond of the present invention is shown in Figure 15. In Figure 15, a carbon-carbon bond-forming reaction apparatus 50b comprises: a cylindrical filling container 51b filled with platinum group metal ion-supported catalyst of the present invention; a raw material container 531b in which a raw material liquid 521b of the reaction is to be placed; a raw material container 532b in which an aqueous inorganic base solution 522b is to be placed; a raw material liquid supply pump 541b for supplying the raw material liquid 521b to the filling container 51b; a raw material liquid supply pump 542b for supplying an aqueous inorganic base solution 522b to the filling container 51b; a reaction liquid receiver 56b for placing therein a reaction liquid 55b discharged from the filling container 51b; and a raw material liquid introduction pipe 57b which extends from each of the raw material container 531b and the raw material container 532b, meets in midstream, connects to the filling container 51a, and serve as an introduction path for the raw material liquid and aqueous inorganic base solution. If necessary, a heating member or a heating apparatus for heating the inside of the filling container 51b is attached to the filling container 51b.

In the carbon-carbon bond-forming reaction apparatus 50b, the raw material liquid 521b is continuously supplied from the raw material container 531b toward the filling container 51b by the raw material liquid supply pump 541b, and the aqueous inorganic base solution 522b is continuously supplied from the raw material container 532b toward the filling container 51b by the raw material liquid supply pump 542b. Then, the raw material liquid 521b is mixed with the aqueous inorganic base solution 521b at the meeting point, and a mixture to be obtained is supplied to the filling container 51b. The mixture of the raw material liquid and the aqueous inorganic base solution supplied into the filling container 51b passes through the platinum group metal ion-supported catalyst of the present invention, specifically, the continuous pores of the organic porous material, filled into the filling container 51b. By this, the raw material liquid and the aqueous inorganic base solution continuously come into contact with the platinum group metal ion-supported catalyst of the present invention so that the raw materials in the raw material liquid react to perform carbon-carbon bond formation. Then, the reaction liquid 55b after carbon-carbon bond formation is sent, through the reaction liquid discharge pipe 59b, to the reaction liquid receiver 56b.

In the method for forming a carbon-carbon bond of the present invention, the size of the filling container, the thickness of a catalyst-filled layer, the flow rates of a solvent and reaction raw materials, the type of the solvent, the direction of circulation of a solution or hydrogen (upward, downward, or lateral), etc. are appropriately selected according to the type or reaction conditions of the reaction. In the method for forming a carbon-carbon bond of the present invention, the raw material liquid may be passed through the platinum group metal ion-supported catalyst of the present invention only once, or the raw material liquid is passed through the platinum group metal ion-supported catalyst of the present invention and then the obtained reaction liquid may be passed again through the platinum group metal ion-supported catalyst of the present invention. When the raw material liquid is passed through the platinum group metal ion-supported catalyst of the present invention and then the obtained reaction liquid is passed again through the platinum group metal ion-supported catalyst of the present invention, the reaction liquid may be directly passed again through the platinum group metal ion-supported catalyst of the present invention, or the reaction liquid is mixed with the raw material liquid and the mixed liquid may be passed through the platinum group metal ion-supported catalyst of the present invention.

A first embodiment of the method for forming a carbon-carbon bond of the present invention (hereinafter, also referred to as a method (1) for forming a carbon-carbon bond) involves reaction to form a carbon-carbon single bond by passing the raw material liquid (i) containing the aromatic halide and the organoboron compound through the platinum group metal ion-supported catalyst according to the present invention, and reacting the aromatic halide with the organoboron compound.

The organoboron compound used in the method (1) for forming a carbon-carbon bond is an organoboron compound represented by the general formulas (I) to (III).

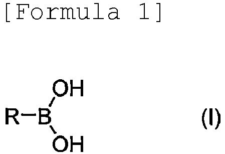

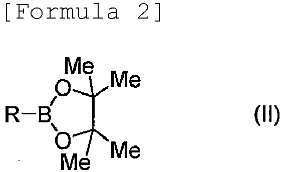

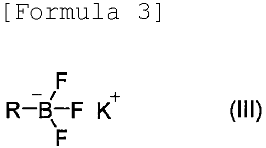

In the formulas, each R is an organic group and is not particularly limited as long as it is an organic group. Examples thereof include a linear alkyl group, a branched alkyl group, a cyclic alkyl group, an aromatic carbocyclic group, and an aromatic heterocyclic group. An amino group, methoxy group, ethoxy group, carboxyl group, acetyl group, nitro group, cyano group, or the like may be introduced into such an organic group R without inhibiting the advantageous effects of the present invention.

The organoboron compound used in the method (1) for forming a carbon-carbon bond is preferably an aromatic boron compound represented by any of the following general formulas (IV) to (VI). 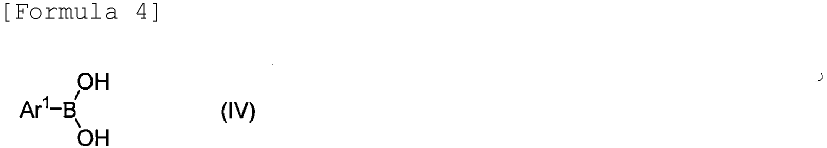 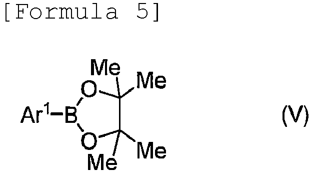 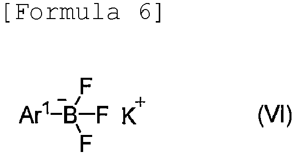 wherein each Ar¹ is an aromatic carbocyclic group or aromatic heterocyclic group having 6 to 18 carbon atoms.

In the formulas (IV) to (VI), examples of the aromatic carbocyclic group or aromatic heterocyclic group according to Ar¹ include a phenyl group, a naphthyl group, a biphenyl group, an anthranyl group, a pyridyl group, a pyrimidyl group, an indolyl group, a benzimidazolyl group, a quinolyl group, a benzofuranyl group, an indanyl group, an indenyl group, and a dibenzofuranyl group. There is no particular limitation on the position at which boron is bonded to the aromatic carbocyclic group or aromatic heterocyclic group, and it can be bonded to an arbitrary position. One or more substituents may be introduced into the aromatic carbocyclic group or aromatic heterocyclic group. Examples of the substituent include a hydrocarbon group such as a methyl group, an ethyl group, a propyl group, a butyl group, a hexyl group, and a benzyl group; an alkoxy group such as a methoxy group, an ethoxy group, a propoxy group, and a butoxy group; and a 9-fluorenylmethoxycarbonyl group, a butoxycarbonyl group, a benzyloxycarbonyl group, a nitro group, and a cyano group.

The aromatic halide used in the method (1) for forming a carbon-carbon bond is an aromatic halide represented by the following general formula (VII).

Ar²-X (VII)

wherein Ar² is an aromatic carbocyclic group or aromatic heterocyclic group having 6 to 18 carbon atoms, and X is a halogen atom.

In the formula (VII), examples of the aromatic carbocyclic group or aromatic heterocyclic group according to Ar² include a phenyl group, a naphthyl group, a biphenyl group, an anthranyl group, a pyridyl group, a pyrimidyl group, an indolyl group, a benzimidazolyl group, a quinolyl group, a benzofuranyl group, an indanyl group, an indenyl group, and a dibenzofuranyl group. There is no particular limitation on the position at which the halogen atom is bonded to the aromatic carbocyclic group or aromatic heterocyclic group, and it can be bonded to an arbitrary position. One or more substituents may be introduced into the aromatic carbocyclic group or aromatic heterocyclic group. Examples of the substituent include a hydrocarbon group such as a methyl group, an ethyl group, a propyl group, a butyl group, a hexyl group, and a benzyl group; an alkoxy group such as a methoxy group, an ethoxy group, a propoxy group, and a butoxy group; and a 9-fluorenylmethoxycarbonyl group, a butoxycarbonyl group, a benzyloxycarbonyl group, a nitro group, a cyano group, a carboxyl group, and an amino group optionally substituted with an organic group. Note that X is a halogen atom and is specifically a fluorine atom, chlorine atom, bromine atom, or iodine atom.

The formation of a carbon-carbon bond by the method (1) for forming a carbon-carbon bond refers to the formation of a carbon-carbon bond between an organic group obtained by the elimination of a boron-containing functional group from the organoboron compound and an aromatic residue obtained by the elimination of halogen from the aromatic halide. For example, when the organoboron compound is an aromatic boron compound represented by any of the formulas (IV) to (VI) and the aromatic halide is an aromatic halide represented by the formula (VII), the resulting coupling product is a compound represented by the formula (VIII).

Ar²-Ar² (VIII)

wherein Ar¹ and Ar² are as defined in the formulas (IV) to (VII).

The ratio between the aromatic boron compound and the aromatic halide to be used in the method (1) for forming a carbon-carbon bond is in a range of aromatic boron compound:aromatic halide = 0.5 to 2:1 in terms of molar ratio, which allows for their application to the reaction without problems, though it is desirable to use them in equimolar amounts.

A second embodiment of the method for forming a carbon-carbon bond of the present invention (hereinafter, also referred to as a method (2) for forming a carbon-carbon bond) involves reaction to form a carbon-carbon single bond by passing the raw material liquid (ii) containing the aromatic halide and the compound having a terminal alkynyl group through the platinum group metal ion-supported catalyst according to the present invention, and reacting the aromatic halide with the compound having a terminal alkynyl group.

The aromatic halide used in the method (2) for forming a carbon-carbon bond is an aromatic halide represented by the formula (VII).

The compound having a terminal alkynyl group used in the method (2) for forming a carbon-carbon bond is a compound represented by the formula (IX).

HC≡C-R¹ (IX)

wherein R¹ is a hydrogen atom, an aromatic carbocyclic group or aromatic heterocyclic group having 6 to 18 carbon atoms and optionally having a substituent, an aliphatic hydrocarbon group having 1 to 18 carbon atoms and optionally having a substituent, an alkenyl group having 2 to 18 carbon atoms and optionally having a substituent, an alkynyl group having 2 to 10 carbon atoms and optionally having a substituent, or a silyl group having 1 to 18 carbon atoms and optionally having a substituent.

In the formula (IX), examples of the aromatic carbocyclic group or aromatic heterocyclic group having 6 to 18 carbon atoms and optionally having a substituent according to R¹ are the same as in Ar¹ and Ar² according to the formulas (IV) to (VII). In the formula (IX), examples of the aliphatic hydrocarbon group having 1 to 18 carbon atoms and optionally having a substituent according to R¹ include a methyl group, an ethyl group, a propyl group, a butyl group, a hexyl group, an octyl group, a dodecyl group, and an octadecyl group. In the formula (IX), examples of the alkenyl group having 2 to 18 carbon atoms and optionally having a substituent according to R¹ include a vinyl group, an allyl group, a methallyl group, a propenyl group, a butenyl group, a hexenyl group, an octenyl group, a decenyl group, and an octadecenyl group. In the formula (IX), examples of the alkynyl group having 2 to 10 carbon atoms and optionally having a substituent according to R¹ include an ethynyl group, a propynyl group, a hexynyl group, and an octenyl group. Examples of the substituent for the aliphatic hydrocarbon group, the alkenyl group, or the alkynyl group include a hydroxy group, a hydrocarbon group, and a heteroatom-containing hydrocarbon group. Examples of the silyl group having 1 to 18 carbon atoms and optionally having a substituent include a trimethylsilyl group, a triethylsilyl group, a triisopropylsilyl group, a tertbutyldimethylsilyl group, and a tert-butyldiphenylsilyl group.

In the method (2) for forming a carbon-carbon bond, the aromatic halide represented by the formula (VII) reacts with the compound represented by the formula (IX) by the platinum group metal ion-supported catalyst according to the present invention to give a product of the formula (X).

Ar²-C≡C-R¹ (X)

wherein Ar² and R¹ are as defined in the formulas (VII) and (IX).

The ratio between the aromatic halide and the compound having a terminal alkynyl group to be used in the method (2) for forming a carbon-carbon bond is in a range of aromatic halide:compound having a terminal alkynyl group = 0.5 to 3:1 in terms of molar ratio, which allows for their application to the reaction without problems, though it is desirable to use them in equimolar amounts.

A third embodiment of the method for forming a carbon-carbon bond of the present invention (hereinafter, also referred to as a method (3) for forming a carbon-carbon bond) involves reaction to form a carbon-carbon single bond by passing the raw material liquid (iii) containing the aromatic halide and the compound having an alkenyl group through the platinum group metal ion-supported catalyst according to the present invention, and reacting the aromatic halide with the compound having an alkenyl group.

The aromatic halide used in the method (3) for forming a carbon-carbon bond is an aromatic halide represented by the formula (VII).

The compound having an alkenyl group used in the method (3) for forming a carbon-carbon bond is a compound represented by the formula (XI).

R²HC=CR³R⁴ (XI)

wherein R², R³, and R⁴ are each independently a hydrogen atom, an aromatic carbocyclic group or aromatic heterocyclic group having 6 to 18 carbon atoms and optionally having a substituent, an aliphatic hydrocarbon group having 1 to 18 carbon atoms and optionally having a substituent, a carboxylic acid derivative, an acid amide derivative, or a cyano group.

In the formula (XI), examples of the aromatic carbocyclic group or aromatic heterocyclic group having 6 to 18 carbon atoms and optionally having a substituent, and the aliphatic hydrocarbon group having 1 to 18 carbon atoms and optionally having a substituent according to R², R³ and R⁴ are the same as in R¹ according to the formula (IX). In the formula (XI), examples of the carboxylic acid derivative according to R², R³ and R⁴ include an alkoxycarbonyl group such as a methoxycarbonyl, an ethoxycarbonyl, and a butoxycarbonyl. In the formula (XI), examples of the acid amide derivative according to R², R³ and R⁴ include a carbamoyl group such as a N-methylcarbamoyl group and a N,N-dimethylcarbamoyl group.

In the method (3) for forming a carbon-carbon bond, the aromatic halide represented by the formula (VII) reacts with the compound represented by the formula (XI) by the platinum group metal ion-supported catalyst according to the present invention to give a product of the formula (XII).

R²Ar²C=CR³R⁴ (XII)

wherein Ar², R², R³ and R⁴ are as defined in the formulas (VII) and (XI).

The ratio between the aromatic halide and the compound having a terminal alkynyl group to be used in the method (3) for forming a carbon-carbon bond is not particularly limited and is in a range of aromatic halide:compound having an alkenyl group = 0.5 to 2:1 in terms of molar ratio, which allows for their application to the reaction without problems.

In the carbon-carbon bond-forming reactions (1) to (3) of the present invention, the amount of the platinum group metal ion-supported catalyst according to the present invention to be used is 0.01 to 20 mol% based on the platinum group metal atom with respect to the aromatic halide.

In the method for forming a carbon-carbon bond of the present invention, the carbon-carbon bond-forming reaction may be performed without a solvent or may be performed using a solvent.

In the method for forming a carbon-carbon bond of the present invention, examples of the method for performing the carbon-carbon bond-forming reaction without a solvent include a method for performing the carbon-carbon bond-forming reaction by introducing the liquid raw material liquid 53a into the reaction raw material liquid 52a of the reaction apparatus 50a shown in Figure 14, introducing the liquid raw material liquid 53a through an introduction path of the filling container 51a filled with the platinum group metal ion-supported catalyst of the present invention into the filling container 51a, passing the liquid raw material liquid 53a through the platinum group metal ion-supported catalyst of the present invention, and discharging the reaction liquid 59a from a discharge path of the filling container 51a.

In the method for forming a carbon-carbon bond of the present invention, when the carbon-carbon bond-forming reaction is performed using a solvent, the solvent to be used is not particularly limited as long as the solvent does not inhibit the carbon-carbon bond-forming reaction. Examples of the solvent include a hydrocarbon solvent such as hexane and heptane; an aromatic solvent such as toluene and xylene; an ether-based solvent such as diisopropyl ether, tetrahydrofuran, dioxane, and 4-methyltetrahydropyran; an ester-based solvent such as ethyl acetate, propyl acetate, and ethyl propionate; an amide-based solvent such as N,N-dimethylformamide, N,N-dimethylacetamide, and N-methylpyrrolidone; an alcohol-based solvent such as methanol, ethanol, propanol, 2-propanol, butanol, ethylene glycol, propylene glycol, and glycerin; and water. These solvents may be used alone as one kind and may be used in combination of two or more kinds of solvents. In respect of inexpensiveness and a high yield, toluene, xylene, tetrahydrofuran, 4-methyltetrahydropyran, N-methylpyrrolidone, 2-propanol, propylene glycol, water, a mixed solvent of toluene and water, a mixed solvent of xylene and water, a mixed solvent of tetrahydrofuran and water, a mixed solvent of 4-methyltetrahydropyran and water, a mixed solvent of N-methylpyrrolidone and water, a mixed solvent of 2-propanol and water, and a mixed solvent of propylene glycol and water are preferable, and toluene, 4-methyltetrahydropyran, N-methylpyrrolidone, 2-propanol, water, a mixed solvent of toluene and water, a mixed solvent of 4-methyltetrahydropyran and water, a mixed solvent of N-methylpyrrolidone and water, and a mixed solvent of 2-propanol and water are more preferable.

In the method for forming a carbon-carbon bond of the present invention, examples of the method for performing the carbon-carbon bond-forming reaction using a solvent include a method for performing the carbon-carbon bond-forming reaction by introducing the raw material liquid 53a in which the raw material is dissolved in an organic solvent into the reaction raw material liquid 52a of the reaction apparatus 50a shown in Figure 14, introducing the raw material liquid 52a in which the raw material is dissolved in an organic solvent through an introduction path of the filling container 51a filled with the platinum group metal ion-supported catalyst of the present invention into the filling container 51a, passing the raw material liquid 52a in which the raw material is dissolved in an organic solvent through the platinum group metal ion-supported catalyst of the present invention, and discharging the reaction liquid 59a from a discharge path of the filling container 51a.

In the carbon-carbon bond-forming reaction of the present invention, it is preferable to perform the carbon-carbon bond-forming reaction in the presence of an inorganic base. Examples of the inorganic base to be used include sodium carbonate, sodium bicarbonate, potassium carbonate, cesium carbonate, potassium acetate, sodium phosphate, potassium phosphate, potassium phenoxide, barium hydroxide, sodium methoxide, sodium ethoxide, potassium butoxide, trimethylamine, and triethylamine. The amount of such an inorganic base to be used is set in a range of 50 to 300 mol% with respect to the aromatic halide.

Examples of the exemplary first embodiment of the implementation process of the carbon-carbon bond-forming reaction of the present invention include a method for forming a carbon-carbon bond, wherein
the raw material liquid (i), the raw material liquid (ii), or the raw material liquid (iii) is an inorganic base-dissolved raw material liquid in which the raw material (an aromatic halide and an organoboron compound, an aromatic halide and a compound having a terminal alkynyl group, or an aromatic halide and an alkenyl group) and an inorganic base are dissolved in water or a hydrophilic solvent, and
the carbon-carbon bond-forming reaction is performed by introducing the inorganic base-dissolved raw material liquid through an introduction path of a filling container filled with the platinum group metal ion-supported catalyst of the present invention into the filling container, passing the inorganic base-dissolved raw material liquid through the platinum group metal ion-supported catalyst, and discharging the reaction liquid from a discharge path of the filling container.

Examples of a method for performing the exemplary first embodiment of the implementation process of the carbon-carbon bond-forming reaction of the present invention include a method for performing the carbon-carbon bond-forming reaction by introducing the raw material liquid (i), the raw material liquid (ii), or the raw material liquid (iii) in which an inorganic base is dissolved, that is, the inorganic base-dissolved raw material liquid 52a in which the raw material (an aromatic halide and an organoboron compound, an aromatic halide and a compound having a terminal alkynyl group, or an aromatic halide and an alkenyl group) and an inorganic base are dissolved in water or a hydrophilic solvent, into the reaction container 53a of the reaction apparatus 50a shown in Figure 14, introducing the inorganic base-dissolved raw material liquid 52a through an introduction path of the filling container 51a filled with the platinum group metal ion-supported catalyst of the present invention into the filling container 51a, passing the inorganic base-dissolved raw material liquid 52a through the platinum group metal ion-supported catalyst of the present invention, and discharging the reaction liquid 59a from a discharge path of the filling container 51a.

Examples of the method for performing the exemplary first embodiment of the implementation process of the carbon-carbon bond-forming reaction of the present invention include a method for performing the carbon-carbon bond-forming reaction by introducing, as the raw material liquid 521b, the raw material liquid (i), the raw material liquid (ii), or the raw material liquid (iii) in which the raw material (an aromatic halide and an organoboron compound, an aromatic halide and a compound having a terminal alkynyl group, or an aromatic halide and an alkenyl group) is dissolved in a hydrophilic organic solvent into the reaction container 531b of the reaction apparatus 50b shown in Figure 14, additionally introducing the aqueous inorganic base solution 522b into the raw material container 532b, supplying the raw material liquid 521b in which the raw material is dissolved in a hydrophilic organic solvent and the aqueous inorganic base solution 522b toward the filling container 51a filled with the platinum group metal ion-supported catalyst of the present invention; then introducing a mixture to be obtained by mixing the raw material liquid 521b and the aqueous inorganic base solution 522b at the meeting point, that is, the inorganic base-dissolved raw material liquid in which the raw material (an aromatic halide and an organoboron compound, an aromatic halide and a compound having a terminal alkynyl group, or an aromatic halide and an alkenyl group) and an inorganic base are dissolved in water or a hydrophilic solvent, through an introduction path of the filling container 51b into the filling container 51b, passing the inorganic base-dissolved raw material liquid through the platinum group metal ion-supported catalyst of the present invention, and discharging the reaction liquid 59b through a discharge path of the filling container 51b.

The exemplary first embodiment of the implementation process of the carbon-carbon bond-forming reaction of the present invention is a homogeneous reaction process in which water or a hydrophilic organic solvent is used as the solvent, wherein an inorganic base raw material liquid in which the raw material and an inorganic base are dissolved in water or a hydrophilic organic solvent is passed through the platinum group metal ion-supported catalyst of the present invention.

In the exemplary first embodiment of the implementation process of the carbon-carbon bond-forming reaction of the present invention, both the raw material and an inorganic base are dissolved in water or a hydrophilic organic solvent in advance to prepare the inorganic base-dissolved raw material liquid, which may be passed through the platinum group metal ion-supported catalyst of the present invention, or alternatively, a raw material liquid in which the raw material is dissolved in water or a hydrophilic organic solvent and an aqueous inorganic base solution are separately prepared and each introduced into separate containers, the raw material liquid and the aqueous inorganic base solution are supplied from each of the containers to an introduction path for supplying the reaction raw material to the platinum group metal ion-supported catalyst of the present invention, and the raw material liquid and the aqueous inorganic base solution are mixed in the introduction path to prepare a homogeneous inorganic base-dissolved raw material liquid, which may be passed through the platinum group metal ion-supported catalyst of the present invention.

Examples of an exemplary second embodiment of the implementation process of the carbon-carbon bond-forming reaction of the present invention include a method for forming a carbon-carbon bond, wherein the raw material liquid (i), the raw material liquid (ii), or the raw material liquid (iii) is a hydrophobic solvent raw material liquid in which the raw material is dissolved in a hydrophobic solvent, and
the carbon-carbon bond-forming reaction is performed by introducing a mixture of the hydrophobic solvent raw material liquid and an aqueous inorganic base solution through an introduction path of a filling container filled with the platinum group metal ion-supported catalyst of the present invention into the filling container, passing the hydrophobic solvent-dissolved raw material liquid and the aqueous inorganic base solution through the platinum group metal ion-supported catalyst, and discharging the reaction liquid from a discharge path of the filling container.

Examples of a method for performing the exemplary second embodiment of the implementation process of the carbon-carbon bond-forming reaction of the present invention include a method for performing the carbon-carbon bond-forming reaction by introducing the raw material liquid (i), the raw material liquid (ii), or the raw material liquid (iii) in which the raw material (an aromatic halide and an organoboron compound, an aromatic halide and a compound having a terminal alkynyl group, or an aromatic halide and an alkenyl group) is dissolved in a hydrophobic organic solvent, that is, the hydrophobic solvent raw material liquid and an aqueous inorganic base solution into the reaction container 53a of the reaction apparatus 50a shown in Figure 15, mixing the hydrophobic solvent raw material liquid and an aqueous inorganic base solution in the reaction container 53a to form a suspension, introducing the obtained suspension through an introduction path of the filling container 51a filled with the platinum group metal ion-supported catalyst of the present invention into filling container 51a, passing the suspension of the hydrophobic solvent raw material liquid and the aqueous inorganic base solution through the platinum group metal ion-supported catalyst of the present invention, and discharging the reaction liquid 59a from a discharge path of the filling container 51a.

Examples of the method for performing the exemplary second embodiment of the implementation process of the carbon-carbon bond-forming reaction of the present invention include a method for performing the carbon-carbon bond-forming reaction by introducing, as the raw material liquid 521b, the raw material liquid (i), the raw material liquid (ii), or the raw material liquid (iii) in which the raw material (an aromatic halide and an organoboron compound, an aromatic halide and a compound having a terminal alkynyl group, or an aromatic halide and an alkenyl group) is dissolved in a hydrophobic organic solvent, that is, the hydrophobic solvent raw material liquid 521b, into the reaction container 531b of the reaction apparatus 50b shown in Figure 15, additionally introducing the aqueous inorganic base solution 522b into the raw material container 532b, supplying the hydrophobic solvent raw material liquid 521b and the aqueous inorganic base solution 522b toward the filling container 51a filled with the platinum group metal ion-supported catalyst of the present invention; then, introducing a mixture to be obtained by mixing the hydrophobic solvent raw material liquid 521b and the aqueous inorganic base solution 522b at the meeting point through an introduction path of the filling container 51b into the filling container 51b, passing the hydrophobic solvent raw material liquid and the aqueous inorganic base solution through the platinum group metal ion-supported catalyst of the present invention, and discharging the reaction liquid 59b through a discharge path of the filling container 51b.

The exemplary second embodiment of the implementation process of carbon-carbon bond-forming reaction of the present invention is a heterogeneous reaction process in which a hydrophobic organic solvent is used as the solvent, wherein a mixture of a raw material liquid in which the raw material is dissolved in a hydrophobic organic solvent and an aqueous inorganic base solution is passed through the platinum group metal ion-supported catalyst of the present invention.

In the exemplary second embodiment of the implementation process of the carbon-carbon bond-forming reaction of the present invention, a hydrophobic solvent raw material liquid in which the raw material is dissolved in a hydrophobic organic solvent and an aqueous inorganic base solution are mixed in a container in advance to prepare a mixture, which may be passed through the platinum group metal ion-supported catalyst of the present invention, or alternatively, a hydrophobic solvent raw material liquid in which the raw material is dissolved in a hydrophobic organic solvent and an aqueous inorganic base solution are separately prepared and each introduced into separate containers, the hydrophobic solvent raw material liquid and the aqueous inorganic base solution from each of the containers are supplied to an introduction path for supplying the reaction raw material to the platinum group metal ion-supported catalyst of the present invention, and the hydrophobic solvent raw material liquid and the aqueous inorganic base solution are mixed in the introduction path to obtain a heterogeneous mixture, which may be passed through the platinum group metal ion-supported catalyst of the present invention.

The atmosphere in which the method for forming a carbon-carbon bond of the present invention is performed is preferably an inert gas (preferably nitrogen, argon, etc.) atmosphere, though it may be performed in air. The reaction temperature is not particularly limited and is arbitrarily set in a range of -20°C to 150°C, preferably 0°C to 120°C, and particularly preferably 20°C to 100°C. In the carbon-carbon bond-forming reaction of the present invention, the supply of the reaction liquid is carried out at an arbitrary flow rate in a range of SV = 0.1 h⁻¹ to 10000 h⁻¹, preferably SV = 0.5 h⁻¹ to 2000 h⁻¹, and particularly preferably SV = 1 h⁻¹ to 1000 h⁻¹, from the viewpoint of a high yield.

The raw material concentration of the reaction liquid in the method for forming a carbon-carbon bond of the present invention is not particularly limited, but in the method for forming a carbon-carbon bond of the present invention, the carbon-carbon bond-forming reaction can be performed at a high yield, even if the raw material concentration is high, for example, 0.40 to 1.00 mol/L and preferably 0.40 to 0.80 mol/L. Note that, in the method for forming a carbon-carbon bond of the present invention, the carbon-carbon bond-forming reaction can be performed satisfactorily, even if the raw material concentration is in the range smaller than the range described above.

In the method for forming a carbon-carbon bond of the present invention, the filling container is a container into which the platinum group metal ion-supported catalyst according to the present invention is filled, and has an introduction path for the raw material liquid, which serves as a path for supplying the raw material liquid, at one end of the platinum group metal ion-supported catalyst according to the present invention, and a discharge path for the reaction liquid, which serves as a path for supplying the reaction liquid to be discharged to the outside of the filling container, at the other end of the platinum group metal ion-supported catalyst according to the present invention. There is no particular limitation on the shape of the filling container, and examples thereof include a circle, a rectangle, and a hexagon as a cross-sectional shape when it is cut at a surface perpendicular to the direction of liquid passing. In the method for forming a carbon-carbon bond of the present invention, it is preferable that the gap between the filling container and the platinum group metal ion-supported catalyst should be as small as possible because a raw material solution can be selectively passed through the continuous pores in the platinum group metal ion-supported catalyst according to the present invention.

In the method for forming a carbon-carbon bond of the present invention, carbon-carbon bond-forming reaction can be selectively caused in the continuous pores of the platinum group metal ion-supported catalyst according to the present invention while the contact time between the catalyst and the raw material liquid and the contact manner between the catalyst and the raw material liquid can be made uniform, by filling the platinum group metal ion-supported catalyst according to the present invention into the filling container, and passing a raw material solution through the platinum group metal ion-supported catalyst according to the present invention. Therefore, the selectivity for the target product can be high.

On the other hand, when a raw material liquid and the platinum group metal ion-supported catalyst according to the present invention are mixed and contacted with each other in a reaction vessel by a reaction scheme in a batch manner, the contact time between the catalyst and the raw material liquid is long and the heating time of the raw material liquid, especially when heating is required for the reaction, is also long. Since side reaction cannot be controlled, the selectivity for the target product is low.

The platinum group metal ion-supported catalyst of the present invention enables the carbon-carbon bond-forming reaction to be performed with a high yield both when an aromatic iodine compound having high reactivity is used and when an aromatic bromine compound having reactivity lower than that of such an aromatic iodine compound is used, as the raw material. The platinum group metal ion-supported catalyst of the present invention enables the carbon-carbon bond-forming reaction to be performed with a high yield, when the reaction is performed in the presence of an inorganic base, both in a homogeneous system in which the inorganic base-dissolved raw material liquid in which the raw material and an inorganic base are dissolved in water or a hydrophilic organic solvent is used and in a heterogeneous system in which a hydrophobic organic solvent is used as a solvent for dissolving the raw material therein and a hydrophobic solvent raw material liquid in which the raw material is dissolved in the hydrophobic organic solvent and an aqueous inorganic base solution are used.

Next, Examples will be given to illustrate the present invention in detail, but these are merely examples and do not limit the present invention.

### [Examples]

### (Reference Example 1) Production of weakly basic monolithic anion exchanger

### (Production of monolithic intermediate (step I))

9.28 g of styrene, 0.19 g of divinylbenzene, 0.50 g of sorbitan monooleate (hereinafter, abbreviated as SMO), and 0.25 g of 2,2'-azobis(isobutyronitrile) were mixed and uniformly dissolved. Next, the styrene/divinylbenzene/SMO/2,2'-azobis(isobutyronitrile) mixture was added to 180 g of pure water and stirred under reduced pressure using Vacuum Mixing & Degassing Mixer (available from EME CORPORATION), which is a planetary stirring apparatus, to obtain a water in oil type emulsion. This emulsion was immediately transferred to a reaction vessel and allowed to be polymerized under static conditions at 60°C for 24 hours after sealing. After the completion of polymerization, the contents were taken out, extracted with methanol, and then dried under reduced pressure to produce a monolithic intermediate having a continuous macropore structure. The internal structure of the monolithic intermediate (dried material) thus obtained was observed with SEM. As the SEM image is shown in Figure 10, it was found that the wall part dividing two adjacent macropores was very thin and rod-like, but had a continuous bubble structure, and the apertures (mesopores), which were the overlapping portions of macropores with each other, had an average diameter of 40 µm and a total pore volume of 18.2 ml/g, as measured by the mercury injection method.

### (Production of monolith)

Then, 216.6 g of styrene, 4.4 g of divinylbenzene, 220 g of 1-decanol, and 0.8 g of 2,2'-azobis(2,4-dimethylvaleronitrile) were mixed and uniformly dissolved (step II). Next, the monolithic intermediate was placed in a reaction vessel and immersed in the styrene/divinylbenzene/1-decanol/2,2'-azobisis(2,4-dimethylvaleronitrile) mixture. After defoaming in a reduced pressure chamber, the reaction vessel was sealed and the mixture was polymerized under static conditions at 50°C for 24 hours. After the completion of polymerization, the content was taken out, soxhlet extracted with acetone, and then dried under reduced pressure (step III).

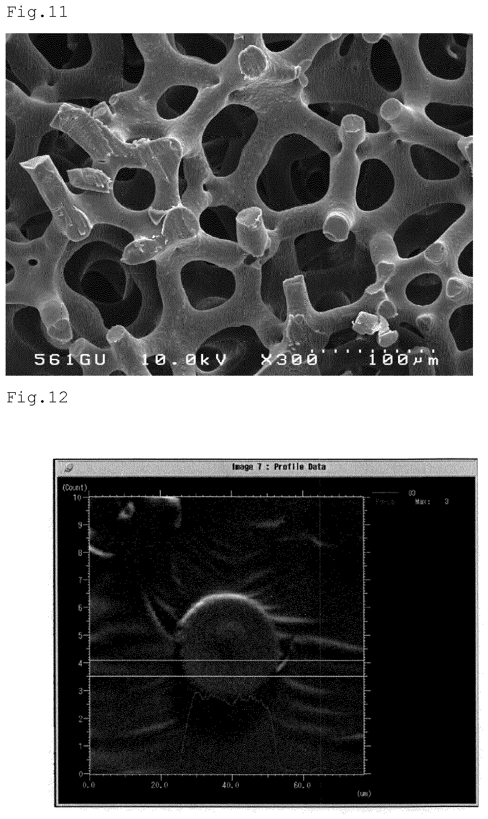

The internal structure of the monolith (dried material) thus obtained, containing 1.2 mol% of crosslinked components formed of the styrene/divinylbenzene copolymer, was observed with SEM, and the results are shown in Figure 11. As is evident from Figure 11, the monolith had a co-continuous structure in which the framework and pores were three dimensionally continuous and both phases were intertwined. In addition, the average thickness of the framework measured from SEM images was 20 µm. Also, the average diameter of the three dimensionally continuous pores in the monolith was 70 µm and the total pore volume was 4.4 ml/g, as measured by the mercury injection method. Note that the average diameter of the pores was determined from the maximum value of the pore distribution curve obtained by the mercury injection method.

### (Production of weakly basic monolithic anion exchanger)

The monolith produced above was placed in a column-like reactor, and a solution containing 1600 g of thionyl chloride, 400 g of tin tetrachloride, and 2500 ml of dimethoxymethane was circulated and passed therethrough and reacted at 30°C for 5 hours to introduce chloromethyl groups. After the completion of reaction, the chloromethylated monolith was washed with a mixed solvent of THF/water = 2/1 and further washed with THF to obtain a chloromethylated monolith.

Then, the chloromethylated monolith was dried under reduced pressure. The weight of the chloromethylated monolith thus dried was 8.4 g. This chloromethylated monolith was placed in a separable flask containing a stirring bar, and a solution containing 56 ml of an aqueous solution of 50% dimethylamine and 180 ml of THF was introduced into the separable flask, which was then stirred for 10 hours under reflux. After the completion of reaction, the product was washed with methanol and then washed to obtain a weakly basic monolithic anion exchanger.

The total anion exchange capacity in a dry state of the obtained weakly basic monolithic anion exchanger was 4.7 mg equivalent/g, and the weak anion exchange capacity was 4.3 mg equivalent/g. The average thickness of the framework in a dry state measured from SEM images was 25 µm.

### (Example 1)

The weakly basic monolithic anion exchanger produced in Reference Example 1 was dried under reduced pressure. The weight of the weakly basic monolithic anion exchanger thus dried was 8.7 g. This monolith in a dry state was treated with hydrochloric acid in methanol and then immersed in dilute hydrochloric acid containing 146 mg of palladium chloride dissolved therein for 24 hours to attach tetrachloropalladate ions to the monolith. After the completion of immersion, the monolith was washed with pure water several times to thereby prepare a Pd ion-supported weakly basic monolithic anion exchanger. As a result of determining the amount of palladium supported in the obtained Pd ion-supported weakly basic monolithic anion exchanger by ICP emission spectroscopy, the amount of palladium supported was 1.0% by weight. The analysis results of the EPMA (Electron Probe Micro Analyzer) analysis and ESCA (Electron Spectroscopy for Chemical Analysis) analysis of the obtained Pd ion-supported weakly basic monolithic anion exchanger are shown respectively in Figure 12 and Figure 13.

Hereinafter, the platinum group metal ion-supported catalyst obtained in Example 1 is referred to as a "Pd ion-supported weakly basic monolithic anion exchanger".

### (Example 2)

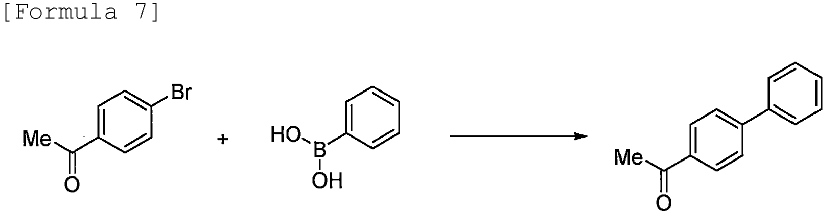

To a solution of 4-bromoacetophenone (0.796 g, 4.0 mmol) and phenylboronic acid (0.536 g, 4.4 mmol) in toluene (2.0 mL), a 1.1 M aqueous sodium hydroxide solution (4.0 mL, 4.4 mmol) was added, and the mixture was stirred. This suspension was passed, at a flow rate of 0.3 mL/min, through an ETFE column filled with a Pd ion-supported weakly basic monolithic anion exchanger (φ2.0 × 150 mm) and heated to 80°C, and collected in a flask containing an aqueous saturated ammonium chloride solution. As a result of analyzing the organic layer of the obtained liquid by GC, 4-acetylbiphenyl was obtained at a conversion ratio of 83%.

### (Example 3)

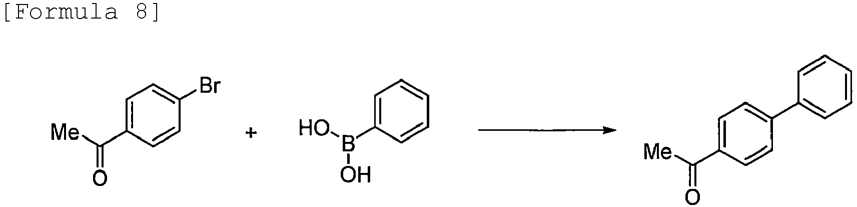

A solution of 4-bromoacetophenone (0.796 g, 4.0 mmol) in toluene (2.0 mL) and a solution of phenylboronic acid (0.536 g, 4.4 mmol) and sodium hydroxide (4.4 mmol) in water (4.0 mL) were supplied at 0.1 mL/min and at 0.2 mL/min, respectively, and both the solutions were combined at a joint. The liquid combined was passed through an ETFE column filled with a Pd ion-supported weakly basic monolithic anion exchanger (φ2.0 × 150 mm) and heated to 80°C, and collected in a flask containing an aqueous saturated ammonium chloride solution. As a result of analyzing the organic layer of the obtained liquid by GC, 4-acetylbiphenyl was obtained at a conversion ratio of 78%.

### (Example 4)

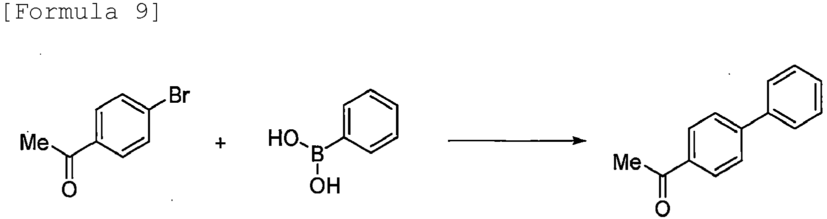

A solution of 4-bromoacetophenone (0.995 g, 5.0 mmol) and phenylboronic acid (0.671 g, 5.5 mmol) in NMP (10 mL) and 0.55 M sodium hydroxide (10 mL, 5.5 mmol) were supplied at 0.5 mL/min and at 0.2 mL/min, respectively, and both the solutions were combined at a joint. The liquid combined was passed through an ETFE column filled with a Pd ion-supported weakly basic monolithic anion exchanger (φ2.0 × 150 mm) and heated to 80°C, and collected in a flask containing an aqueous saturated ammonium chloride solution. As a result of analyzing the organic layer of the obtained liquid by GC, 4-acetylbiphenyl was obtained at a conversion ratio of 94%.

### (Example 5)

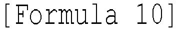

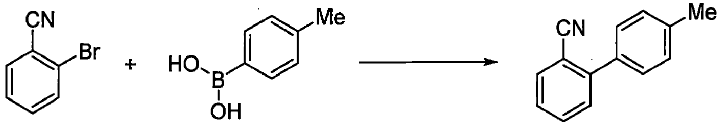

To a solution of 2-bromobenzonitrile (0.728 g, 4.0 mmol) and 4-methylphenylboronic acid (0.598 g, 4.4 mmol) in toluene (1.0 mL), a 1.0 M aqueous sodium hydroxide solution (4.0 mL, 4.0 mmol) was added, and the mixture was stirred. This suspension was passed, at a flow rate of 0.5 mL/min, through an ETFE column filled with a Pd ion-supported weakly basic monolithic anion exchanger (φ2.0 × 150 mm) and heated to 80°C, and collected in a flask containing an aqueous saturated ammonium chloride solution. As a result of analyzing the organic layer of the obtained liquid by GC, 2-cyano-4'-methylbiphenyl was obtained at a conversion ratio of 97%.

### (Example 6)

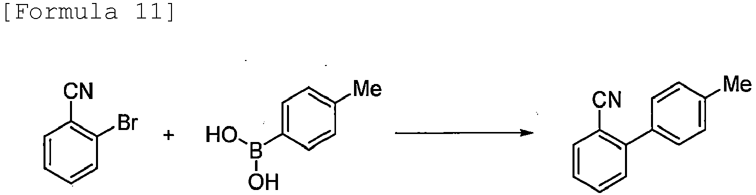

A solution of 2-bromobenzonitrile (0.728 g, 4.0 mmol) in toluene (2.0 mL) and a solution of 4-methylphenylboronic acid (0.598 g, 4.4 mmol) - sodium hydroxide (4.4 mmol) in water (4.0 mL) were supplied at 0.1 mL/min and at 0.4 mL/min, respectively, and both the solutions were combined at a joint. The liquid combined was passed through an ETFE column filled with a Pd ion-supported weakly basic monolithic anion exchanger (φ2.0 × 150 mm) and heated to 80°C, and collected in a flask containing an aqueous saturated ammonium chloride solution. As a result of analyzing the organic layer of the obtained liquid by GC, 2-cyano-4'-methylbiphenyl was obtained at a conversion ratio of 95%.

### (Reference Example 2) Production of strongly acidic monolithic cation exchanger

### (Production of monolithic intermediate (step I))

9.28 g of styrene, 0.19 g of divinylbenzene, 0.50 g of sorbitan monooleate (hereinafter, abbreviated as SMO), and 0.25 g of 2,2'-azobis(isobutyronitrile) were mixed and uniformly dissolved. Next, the styrene/divinylbenzene/SMO/2,2'-azobis(isobutyronitrile) mixture was added to 180 g of pure water and stirred under reduced pressure using Vacuum Mixing & Degassing Mixer (available from EME CORPORATION), which is a planetary stirring apparatus, to obtain a water in oil type emulsion. This emulsion was immediately transferred to a reaction vessel and allowed to be polymerized under static conditions at 60°C for 24 hours after sealing. After the completion of polymerization, the contents were taken out, extracted with methanol, and then dried under reduced pressure to produce a monolithic intermediate having a continuous macropore structure. When the internal structure of the monolithic intermediate (dried material) thus obtained was observed with SEM, it was found that the wall part dividing two adjacent macropores was very thin and rod-like, but had a continuous bubble structure, and the apertures (mesopores), which were the overlapping portions of macropores with each other, had an average diameter of 40 µm and a total pore volume of 18.2 ml/g, as measured by the mercury injection method.

### (Production of monolith)

Then, 216.6 g of styrene, 4.4 g of divinylbenzene, 220 g of 1-decanol, and 0.8 g of 2,2'-azobis(2,4-dimethylvaleronitrile) were mixed and uniformly dissolved (step II). Next, the monolithic intermediate was placed in a reaction vessel and immersed in the styrene/divinylbenzene/1-decanol/2,2'-azobisis(2,4-dimethylvaleronitrile) mixture. After defoaming in a reduced pressure chamber, the reaction vessel was sealed and the mixture was allowed to be polymerized under static conditions at 50°C for 24 hours. After the completion of polymerization, the content was taken out, soxhlet extracted with acetone, and then dried under reduced pressure (step III).

When the internal structure of the monolith (dried material) thus obtained, containing 1.2 mol% of crosslinked components formed of the styrene/divinylbenzene copolymer, was observed with SEM, the monolith had a co-continuous structure in which the framework and pores were three dimensionally continuous and both phases were intertwined. In addition, the average thickness of the framework measured from the SEM image was 20 µm. Also, the average diameter of the three dimensionally continuous pores in the monolith was 70 µm and the total pore volume was 4.4 ml/g, as measured by the mercury injection method. Note that the average diameter of the pores was determined from the maximum value of the pore distribution curve obtained by the mercury injection method.

### (Production of strongly acidic monolithic cation exchanger)

The monolith produced above was placed in a column-like reactor, and a solution containing 500 g of chlorosulfonic acid and 1000 ml of dichloroethane was circulated and passed therethrough and reacted at 30°C for 3 hours. After the completion of reaction, the chloromethylated monolith was washed with dichloroethane and further washed with methanol to obtain a strongly acidic monolithic cation exchanger.

The total anion exchange capacity in a dry state of the obtained strongly acidic monolithic cation exchanger was 4.7 mg equivalent/g, and the strong cation exchange capacity was 4.3 mg equivalent/g. The average thickness of the framework in a dry state measured from SEM images was 25 µm.

### (Example 7)

The strongly acidic monolithic cation exchanger produced in Reference Example 2 was dried under reduced pressure, and the monolith thus dried in a dry state having a weight of 7.5 g was immersed in methanol in which 800 mg of palladium acetate was dissolved, under nitrogen atmosphere at room temperature so that palladium ions were supported on the strongly acidic monolithic cation exchanger. Then, the strongly acidic monolithic cation exchanger was taken out and washed with pure water several times to prepare a Pd ion-supported strongly acidic monolithic cation exchanger. As a result of determining the amount of palladium supported in the obtained Pd ion-supported strongly acidic monolithic cation exchanger by ICP emission spectroscopy, the amount of palladium supported was 4.6% by weight.

Hereinafter, the platinum group metal ion-supported catalyst obtained in Example 7 is referred to as a "Pd ion-supported strongly acidic monolithic cation exchanger".

### (Example 8)

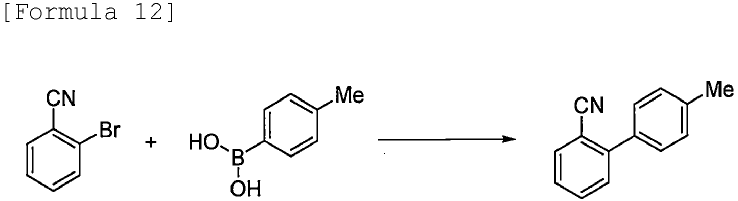

To a solution of 2-bromobenzonitrile (0.728 g, 4.0 mmol) and 4-methylphenylboronic acid (0.598 g, 4.4 mmol) in toluene (1.0 mL), a 1.0 M aqueous sodium hydroxide solution (4.0 mL, 4.0 mmol) was added, and the mixture was stirred. This suspension was passed through a SUS column filled with a Pd ion-supported strongly acidic monolithic cation exchanger (φ4.6 × 30mm) and heated to 80°C, and collected in a flask containing an aqueous saturated ammonium chloride solution. As a result of analyzing the organic layer of the obtained liquid by GC, 2-cyano-4'-methylbiphenyl was obtained at a conversion ratio of 24%.

### (Comparative Example 1)

The Pd ion-supported weakly basic monolithic anion exchanger produced in Example 1 was immersed in an aqueous hydrazine solution for 24 hours so as to reduce Pd ions. After the completion of immersion, the exchanger was washed with pure water several times to thereby prepare a Pd nanoparticle-supported weakly basic monolithic anion exchanger.

### (Comparative Example 2)

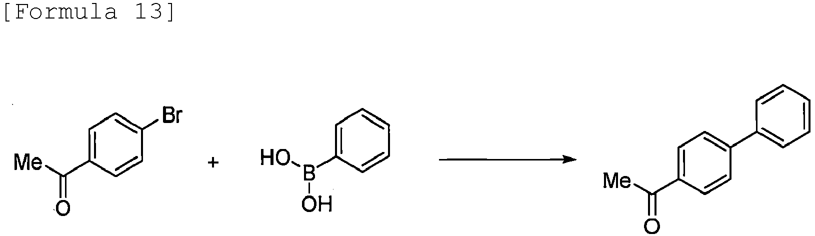

A solution of 4-bromoacetophenone (0.796 g, 4.0 mmol) in toluene (2.0 mL) and a solution of phenylboronic acid (0.536 g, 4.4 mmol) and sodium hydroxide (4.4 mmol) in water (4.0 mL) were supplied at 0.1 mL/min and at 0.2 mL/min, respectively, and both the solutions were combined at a joint. The liquid combined was passed through an ETFE column filled with the Pd nanoparticle-supported weakly basic monolithic anion exchanger (φ2.0 × 150 mm) produced in Comparative Example 1 and heated to 80°C, and collected in a flask containing an aqueous saturated ammonium chloride solution. As a result of analyzing the organic layer of the obtained liquid by GC, 4-acetylbiphenyl was obtained at a conversion ratio of 53%.

### (Comparative Example 3)

10 g in dry weight of a weakly basic anion exchange resin (Amberlite(TM) A21) was treated with hydrochloric acid in methanol and then immersed in dilute hydrochloric acid containing 170 mg of palladium chloride dissolved therein for 24 hours to attach tetrachloropalladate ions to the resin. After the completion of immersion, the resultant was washed with pure water several times to thereby prepare a Pd ion-supported weakly basic anion exchange resin.

### (Comparative Example 4)

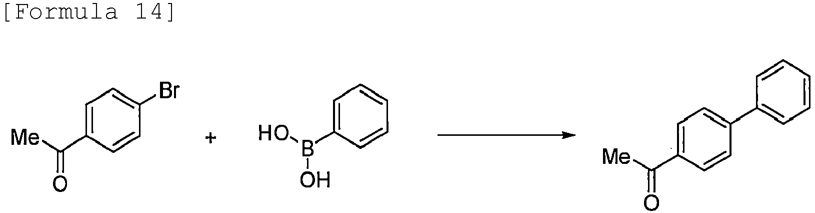

A solution of 4-bromoacetophenone (0.796 g, 4.0 mmol) in toluene (2.0 mL) and a solution of phenylboronic acid (0.536 g, 4.4 mmol) and sodium hydroxide (4.4 mmol) in water (4.0 mL) were supplied at 0.1 mL/min and at 0.2 mL/min, respectively, and both the solutions were combined at a joint. The liquid combined was passed through a SUS column filled with the Pd ion-supported weakly basic anion exchange resin (0.5 mL) produced in Comparative Example 3 and heated to 80°C, and collected in a flask containing an aqueous saturated ammonium chloride solution. As a result of analyzing the organic layer of the obtained liquid by GC, 4-acetylbiphenyl was obtained at a conversion ratio of 28%.

### [Reference Signs List]

1 Framework phase
2 Pore phase
10 Monolith
11 Image region
12 Framework part appearing in cross section
13 Macropore
21 Framework surface
22 Protruding material
30 Monolith (monolithic ion exchanger)
31 Macropore
32 Aperture
33 Inner layer part
34 Surface layer part
35 Gas phase part (Bubble part)
36 Wall part (framework part)
37 Pore
50a, 50b Carbon-carbon bond-forming reaction apparatus
51a, 51b Filling container
52a, 521b Raw material liquid
53a, 531b, 532b Raw material container
54a, 541b, 542b Raw material liquid supply pump
55a, 55b Reaction liquid
56a, 56b Reaction liquid receiver
57a, 57b Raw material liquid introduction pipe
58a Switching valve
59a Reaction liquid discharge pipe
60a Circulation pipe
522b Aqueous inorganic base solution

## Claims

1. A platinum group metal ion-supported catalyst in which platinum group metal ions or platinum group metal complex ions are supported on a non-particulate organic porous ion exchanger, wherein the non-particulate organic porous ion exchanger is formed of a continuous framework phase and a continuous pore phase; has a thickness of a continuous framework of 1 to 100 µm, an average diameter of continuous pores of 1 to 1000 µm, and a total pore volume of 0.5 to 50 ml/g; has an ion exchange capacity per weight in a dry state of 1 to 9 mg equivalent/g; and has ion exchange groups wherein the ion exchange groups are uniformly distributed in the organic porous ion exchanger.

2. The platinum group metal ion-supported catalyst according to claim 1, wherein the non-particulate organic porous ion exchanger has a continuous bubble structure with macropores linked to each other and common apertures (mesopores) with an average diameter of 1 to 1000 µm within the walls of the macropores; has a total pore volume of 1 to 50 ml/g; has an ion exchange capacity per weight in a dry state of 1 to 9 mg equivalent/g; and has ion exchange groups wherein the ion exchange groups are uniformly distributed in the organic porous ion exchanger.

3. The platinum group metal ion-supported catalyst according to claim 1, wherein the non-particulate organic porous ion exchanger forms a framework portion with aggregated and thus three dimensionally continuous organic polymer particles with an average particle diameter of 1 to 50 µm; has three dimensionally continuous pores in the framework with an average diameter of 20 to 100 µm; has a total pore volume of 1 to 10 ml/g; has an ion exchange capacity per weight in a dry state of 1 to 9 mg equivalent/g; and has ion exchange groups wherein the ion exchange groups are uniformly distributed in the organic porous ion exchanger.

4. The platinum group metal ion-supported catalyst according to claim 1, wherein the non-particulate organic porous ion exchanger is a continuous macropore structural material in which bubble-like macropores overlap each other and these overlapping areas become apertures with an average diameter of 30 to 300 µm; has a total pore volume of 0.5 to 10 ml/g and an ion exchange capacity per weight in a dry state of 1 to 9 mg equivalent/g; has ion exchange groups wherein the ion exchange groups are uniformly distributed in the organic porous ion exchanger; and, in a SEM image of the cut section of the continuous macropore structural material (dried material), has an area of the framework part appearing in the cross section of 25 to 50% in the image region.

5. The platinum group metal ion-supported catalyst according to claim 1, wherein the non-particulate organic porous ion exchanger is a co-continuous structural material formed of a three dimensionally continuous framework comprising an aromatic vinyl polymer containing 0.1 to 5.0 mol% of crosslinked structural units among the entire constituent units into which ion exchange groups have been introduced, with an average thickness of 1 to 60 µm, and three dimensionally continuous pores in the framework with an average diameter of 10 to 200 µm; has a total pore volume of 0.5 to 10 ml/g; has an ion exchange capacity per weight in a dry state of 1 to 9 mg equivalent/g; and has ion exchange groups wherein the ion exchange groups are uniformly distributed in the organic porous ion exchanger.

6. The platinum group metal ion-supported catalyst according to claim 1, wherein the non-particulate organic porous ion exchanger is formed of a continuous framework phase and a continuous pore phase; in the framework, has a number of particle materials with a diameter of 4 to 40 µm adhering to the surface or a number of protruding materials with a size of 4 to 40 µm formed on the framework surface of the organic porous material; has an average diameter of continuous pores of 10 to 200 µm and a total pore volume of 0.5 to 10 ml/g; has an ion exchange capacity per weight in a dry state of 1 to 9 mg equivalent/g; and has ion exchange groups wherein the ion exchange groups are uniformly distributed in the organic porous ion exchanger.

7. The platinum group metal ion-supported catalyst according to any one of claims 1 to 6, wherein an amount of the platinum group metal ions or the platinum group metal complex ions supported is 0.01 to 10.0% by mass based on the platinum group metal atom.

8. A method for forming a carbon-carbon bond to form a carbon-carbon bond by performing (1) reaction of an aromatic halide with an organoboron compound, (2) reaction of an aromatic halide with a compound having a terminal alkynyl group, or (3) reaction of an aromatic halide with a compound having an alkenyl group,
wherein the carbon-carbon bond-forming reaction is performed by introducing a raw material liquid (i) containing the aromatic halide and the organoboron compound, a raw material liquid (ii) containing the aromatic halide and the compound having a terminal alkynyl group, or a raw material liquid (iii) containing the aromatic halide and the compound having an alkenyl group, through an introduction path of a filling container filled with a platinum group metal ion-supported catalyst, into the filling container, passing the raw material liquid through the platinum group metal ion-supported catalyst, and discharging the reaction liquid from a discharge path of the filling container, and
the platinum group metal ion-supported catalyst is the platinum group metal ion-supported catalyst according to any one of claims 1 to 7.

9. The method for forming a carbon-carbon bond according to claim 8, wherein the carbon-carbon bond-forming reaction is performed in the presence of an inorganic base.

10. The method for forming a carbon-carbon bond according to claim 8 or 9, wherein the raw material liquid (i) containing the aromatic halide and the organoboron compound, the raw material liquid (ii) containing the aromatic halide and the compound having a terminal alkynyl group, or the raw material liquid (iii) containing the aromatic halide and the compound having an alkenyl group is an inorganic base-dissolved raw material liquid in which the raw material and an inorganic base are dissolved in water or a hydrophilic solvent, and
the carbon-carbon bond-forming reaction is performed by introducing the inorganic base-dissolved raw material liquid through an introduction path of a filling container filled with the platinum group metal ion-supported catalyst into the filling container, passing the inorganic base-dissolved raw material liquid through the platinum group metal ion-supported catalyst, and discharging the reaction liquid from a discharge path of the filling container.

11. The method for forming a carbon-carbon bond according to claim 8 or 9, wherein the raw material liquid (i) containing the aromatic halide and the organoboron compound, the raw material liquid (ii) containing the aromatic halide and the compound having a terminal alkynyl group, or the raw material liquid (iii) containing the aromatic halide and the compound having an alkenyl group is a hydrophobic solvent raw material liquid in which the raw material is dissolved in a hydrophobic organic solvent, and
the carbon-carbon bond-forming reaction is performed by introducing a mixture of the hydrophobic solvent raw material liquid and an aqueous inorganic base solution through an introduction path of a filling container filled with the platinum group metal ion-supported catalyst into the filling container, passing the hydrophobic solvent-dissolved raw material liquid and the aqueous inorganic base solution through the platinum group metal ion-supported catalyst, and discharging the reaction liquid from a discharge path of the filling container.
